# EUROPEAN PATENT APPLICATION

(11) **EP 4 206 213 A1**
(43) Date of publication of application: **05.07.2023**
(21) Application number: 22206076.6
(22) Date of filing: 17.07.2017
(51) Int. Cl.: C07H 21/02, A61K 48/00, A61P 21/00, A61P 21/02, A61P 25/28, A61P 43/00

(54) **COMPOUNDS AND METHODS FOR MODULATION OF SMN2**

(30) Priority: 15.07.2016 US 201662363195 P
(62) Divisional of application: 17828625.8
(71) Applicant: Ionis Pharmaceuticals, Inc., Carlsbad, CA 92010 (US)
(72) Inventor: RIGO, Frank, Carlsbad, 92010 (US); PRAKASH, Thazha, P., Carlsbad, 92010 (US); SETH, Punit, P., Carlsbad, 92010 (US)
(74) Representative: Carpmaels & Ransford LLP

(57) **Abstract**

Disclosed herein are compounds, compositions and compounds for use in modulating splicing of SMN2, said compounds consisting in oligomers comprising a modified oligonucleotide consisting of 14-25 linked nucleosides, wherein the modified oligonucleotide is complementary to an SMN2 pre-mRNA; and wherein at least one nucleoside of the modified oligonucleotide comprises a 2'-O(N-alkyl acetamide) modified sugar moiety. Also provided are uses of disclosed compounds and compositions in the manufacture of a medicament for treatment of spinal muscular atrophy.

## Description

### Sequence Listing

The present application is being filed along with a Sequence Listing in electronic format. The Sequence Listing corresponds to a file entitled BIOL0304WOSEQ_ST25.txt created July 17, 2017, which is 88 Kb in size. The information in the electronic format of the Sequence Listing and the file entitled BIOL0304WOSEQ_ST25.txt is incorporated herein by reference in its entirety.

### Field of the Invention

Provided herein are methods, compounds, and compositions for modulation of SMN2.

### BACKGROUND

Proximal spinal muscular atrophy (SMA) is a genetic, neurodegenerative disorder characterized by the loss of spinal motor neurons. SMA is an autosomal recessive disease of early onset and is currently the leading cause of death among infants. The severity of SMA varies among patients and has thus been classified into three types. Type I SMA is the most severe form with onset at birth or within 6 months and typically results in death within 2 years. Children with type I SMA are unable to sit or walk. Type II SMA is the intermediate form and patients are able to sit, but cannot stand or walk. Patients with type III SMA, a chronic form of the disease, typically develop SMA after 18 months of age (Lefebvre et al., Hum. Mol. Genet., 1998, 7, 1531-1536).

The molecular basis of SMA is caused by the loss of both copies of survival motor neuron gene 1 (SMN1), which may also be known as SMN Telomeric, a protein that is part of a multi-protein complex thought to be involved in snRNP biogenesis and recycling. A nearly identical gene, SMN2, which may also be known as SMN Centromeric, exists in a duplicated region on chromosome 5q13 and modulates disease severity. Expression of the normal SMN1 gene results solely in expression of survival motor neuron (SMN) protein. Although SMN1 and SMN2 have the potential to code for the same protein, SMN2 contains a translationally silent mutation at position +6 of exon 7, which results in inefficient inclusion of exon 7 in SMN2 transcripts. Thus, the predominant form of SMN2 is a truncated version, lacking exon 7, which is unstable and inactive (Cartegni and Krainer, Nat. Genet., 2002, 30, 377-384). Expression of the SMN2 gene results in approximately 10-20% of the SMN protein and 80-90% of the unstable/non-functional SMNdelta7 protein. SMN protein plays a well-established role in assembly of the spliceosome and may also mediate mRNA trafficking in the axon and nerve terminus of neurons.

Antisense technology is an effective means for modulating the expression of one or more specific gene products, including alternative splice products, and is uniquely useful in a number of therapeutic, diagnostic, and research applications. The principle behind antisense technology is that an antisense compound, which hybridizes to a target nucleic acid, modulates gene expression activities such as transcription, splicing or translation through one of a number of antisense mechanisms. The sequence specificity of antisense compounds makes them extremely attractive as tools for target validation and gene functionalization, as well as therapeutics to selectively modulate the expression of genes involved in disease.

### SUMMARY

The present disclosure provides compounds, methods, and compositions for modulation of SMN2 pre-mRNA. The present disclosure also provides compounds, methods, and compositions useful, for example, to treat, prevent, or ameliorate one or more symptoms of Spinal Muscular Atrophy.

In certain embodiments, the present disclosure provides oligomeric compounds comprising or consisting of modified oligonucleotides having one or more 2'-O-(N-alkyl acetamide) or 2'-O-(N-methyl acetamide) modified sugar moieties and complementary to SMN pre-mRNA. Modified oligonucleotides having one or more 2'-O-(N-alkyl acetamide) or 2'-O-(N-methyl acetamide) modified sugar moieties have enhanced cellular uptake and/or pharmacologic activity in muscle tissue. Since SMN2 is expressed in muscle tissue, modified oligonucleotides having one or more 2'-O-(N-alkyl acetamide) modifications will have improved activity in muscle tissue.

Provided herein are compounds and methods useful for modulating processing of a SMN2 pre-mRNA. The compounds comprise modified oligonucleotides that comprise 2'-O-(N-alkyl acetamide) or 2'-O-(N-methyl acetamide) modified sugar moieties. In certain embodiments, the modified oligonucleotides comprise 2'-O-(N-methyl acetamide) or 2'-O-(N-methyl acetamide) modified sugar moieties. In certain embodiments, compounds of the invention modulate splicing of a pre-mRNA. Modified oligonucleotides having one or more 2'-O-(N-alkyl acetamide) or 2'-O-(N-methyl acetamide) modified sugar moieties also have enhanced pharmacologic activity for modulation of SMN2 pre-mRNA. For example, in certain embodiments, modified oligonucleotides having one or more 2'-O-(N-alkyl acetamide) or 2'-O-(N-methyl acetamide) modified sugar moieties targeted to SMN2 pre-mRNA increase the inclusion of exon 7 to a greater extent than modified oligonucleotides lacking one or more 2'-O-(N-alkyl acetamide) or 2'-O-(N-methyl acetamide) modified sugar moieties.

Further provided herein are methods of enhancing cellular uptake, methods of enhancing pharmacologic activity and methods of modulating tissue distribution of oligomeric compounds comprising or consisting of a conjugate group and a modified oligonucleotide comprising 2'-O-(N-alkyl acetamide) or 2'-O-(N-methyl acetamide) modified sugar moieties. Also provided are compounds comprising a modified oligonucleotide comprising 2'-O-(N-alkyl acetamide) or 2'-O-(N-methyl acetamide) modified sugar moieties for use in therapy. Compounds for the preparation of medicaments for modulation of processing of a selected precursor transcript in cells or tissues are also provided.

The present disclosure provides the following non-limiting numbered embodiemnts:
Embodiment 1: An oligomeric compound comprising a modified oligonucleotide consisting of 14-25 linked nucleosides, wherein the modified oligonucleotide is complementary to an SMN2 pre-mRNA; and wherein at least one nucleoside of the modified oligonucleotide has a structure of Formula I: 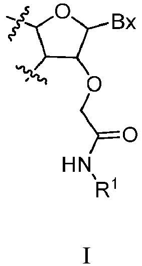
   wherein Bx is a nucleobase;
   and R¹ for each nucleoside of Formula I is independently selected from among: methyl, ethyl, propyl, and isopropyl.
Embodiment 2: The oligomeric compound of embodiment 1, wherein Bx is selected from among adenine, guanine, cytosine, thymine, uracil, and 5-methyl cytosine.
Embodiment 3: The oligomeric compound of embodiment 1 or 2, wherein R¹ is methyl.
Embodiment 4: The oligomeric compound of any of embodiments 1-3, wherein each of at least 2 nucleosides of the modified oligonucleotide has a structure independently selected from Formula I.
Embodiment 5: The oligomeric compound of any of embodiments 1-3, wherein each of 7 nucleosides of the modified oligonucleotide has a structure independently selected from Formula I.
Embodiment 6: The oligomeric compound of any of embodiments 1-3, wherein each of 8 nucleosides of the modified oligonucleotide has a structure independently selected from Formula I.
Embodiment 7: The oligomeric compound of any of embodiments 1-3, wherein each of 9 nucleosides of the modified oligonucleotide has a structure independently selected from Formula I.
Embodiment 8: The oligomeric compound of any of embodiments 1-3, wherein each of 10 nucleosides of the modified oligonucleotide has a structure independently selected from Formula I.
Embodiment 9: The oligomeric compound of any of embodiments 1-3, wherein each of 11 nucleosides of the modified oligonucleotide has a structure independently selected from Formula I.
Embodiment 10: The oligomeric compound of any of embodiments 1-3, wherein each of 12 nucleosides of the modified oligonucleotide has a structure independently selected from Formula I.
Embodiment 11: The oligomeric compound of any of embodiments 1-3, wherein each of 13 nucleosides of the modified oligonucleotide has a structure independently selected from Formula I.
Embodiment 12: The oligomeric compound of any of embodiments 1-3, wherein each of 14 nucleosides of the modified oligonucleotide has a structure independently selected from Formula I.
Embodiment 13: The oligomeric compound of any of embodiments 1-3, wherein each of 15 nucleosides of the modified oligonucleotide has a structure independently selected from Formula I.
Embodiment 14: The oligomeric compound of any of embodiments 1-3, wherein each of 16 nucleosides of the modified oligonucleotide has a structure independently selected from Formula I.
Embodiment 15: The oligomeric compound of any of embodiments 1-3, wherein each of 17 nucleosides of the modified oligonucleotide has a structure independently selected from Formula I.
Embodiment 16: The oligomeric compound of any of embodiments 1-3, wherein each of 18 nucleosides of the modified oligonucleotide has a structure independently selected from Formula I.
Embodiment 17: The oligomeric compound of any of embodiments 1-3, wherein each of 19 nucleosides of the modified oligonucleotide has a structure independently selected from Formula I.
Embodiment 18: The oligomeric compound of any of embodiments 1-3, wherein each of 20 nucleosides of the modified oligonucleotide has a structure independently selected from Formula I.
Embodiment 19: The oligomeric compound of any of embodiments 1-18, wherein R¹ of at least one nucleoside having a structure of Formula I is methyl.
Embodiment 20: The oligomeric compound of any of embodiments 1-19, wherein R¹ is the same for all of the nucleosides having a structure of Formula I.
Embodiment 21: An oligomeric compound comprising a modified oligonucleotide consisting of 14-25 linked nucleosides, wherein the modified oligonucleotide is complementary to an SMN2 pre-mRNA; and wherein at least one nucleoside of the modified oligonucleotide comprises a 2'-O-(N-alkyl acetamide) modified sugar moiety.
Embodiment 22: The oligomeric compound of embodiment 21, wherein the sugar moiety of each nucleoside comprising a 2'-O-(N-alkyl acetamide) modified sugar moiety is selected from 2'-O-(N-methyl acetamide) and 2'-O-(N-ethyl acetamide).
Embodiment 23: The oligomeric compound of embodiment 21 or 22, wherein each of 7 nucleosides of the modified oligonucleotide comprises an independently selected 2'-O-(N-alkyl acetamide) modified sugar moiety.
Embodiment 24: The oligomeric compound of embodiment 21 or 22, wherein each of 8 nucleosides of the modified oligonucleotide comprises an independently selected 2'-O-(N-alkyl acetamide) modified sugar moiety.
Embodiment 25: The oligomeric compound of embodiment 21 or 22, wherein each of 9 nucleosides of the modified oligonucleotide comprises an independently selected 2'-O-(N-alkyl acetamide) modified sugar moiety.
Embodiment 26: The oligomeric compound of embodiment 21 or 22, wherein each of 10 nucleosides of the modified oligonucleotide comprises an independently selected 2'-O-(N-alkyl acetamide) modified sugar moiety.
Embodiment 27: The oligomeric compound of embodiment 21 or 22, wherein each of 11 nucleosides of the modified oligonucleotide comprises an independently selected 2'-O-(N-alkyl acetamide) modified sugar moiety.
Embodiment 28: The oligomeric compound of embodiment 21 or 22, wherein each of 12 nucleosides of the modified oligonucleotide comprises an independently selected 2'-O-(N-alkyl acetamide) modified sugar moiety.
Embodiment 29: The oligomeric compound of embodiment 21 or 22, wherein each of 13 nucleosides of the modified oligonucleotide comprises an independently selected 2'-O-(N-alkyl acetamide) modified sugar moiety.
Embodiment 30: The oligomeric compound of embodiment 21 or 22, wherein each of 14 nucleosides of the modified oligonucleotide comprises an independently selected 2'-O-(N-alkyl acetamide) modified sugar moiety.
Embodiment 31: The oligomeric compound of embodiment 21 or 22, wherein each of 15 nucleosides of the modified oligonucleotide comprises an independently selected 2'-O-(N-alkyl acetamide) modified sugar moiety.
Embodiment 32: The oligomeric compound of embodiment 21 or 22, wherein each of 16 nucleosides of the modified oligonucleotide comprises an independently selected 2'-O-(N-alkyl acetamide) modified sugar moiety.
Embodiment 33: The oligomeric compound of embodiment 21 or 22, wherein each of 17 nucleosides of the modified oligonucleotide comprises an independently selected 2'-O-(N-alkyl acetamide) modified sugar moiety.
Embodiment 34: The oligomeric compound of embodiment 21 or 22, wherein each of 18 nucleosides of the modified oligonucleotide comprises an independently selected 2'-O-(N-alkyl acetamide) modified sugar moiety.
Embodiment 35: The oligomeric compound of embodiment 21 or 22, wherein each of 19 nucleosides of the modified oligonucleotide comprises an independently selected 2'-O-(N-alkyl acetamide) modified sugar moiety.
Embodiment 36: The oligomeric compound of embodiment 21 or 22, wherein each of 20 nucleosides of the modified oligonucleotide comprises an independently selected 2'-O-(N-alkyl acetamide) modified sugar moiety.
Embodiment 37: The oligomeric compound of any of embodiments 21-36, wherein the sugar moiety of at least one of the nucleosides comprising a 2'-O-(N-alkyl acetamide) modified sugar moiety is a 2'-O-(N-methyl acetamide) modified sugar moiety.
Embodiment 38: The oligomeric compound of any of embodiments 21-37, wherein the N-alkyl group of each of the 2'-O-(N-alkyl acetamide) modified sugar moieties is the same N-alkyl group.
Embodiment 39: The oligomeric compound of any of embodiments 21-38, wherein each of the 2'-O-(N-alkyl acetamide) modified sugar moieties is a 2'-O-(N-methyl acetamide) sugar moiety.
Embodiment 40: The oligomeric compound of any of embodiments 21-39, wherein each sugar moiety of each nucleoside of the modified oligonucleotide is a 2'-O-(N-methyl acetamide) modified sugar moiety.
Embodiment 41: The oligomeric compound of any of embodiments 1-40, wherein the modified oligonucleotide consists of 16-23 linked nucleosides.
Embodiment 42: The oligomeric compound of any of embodiments 1-40, wherein the modified oligonucleotide consists of 18-20 linked nucleosides.
Embodiment 43: The oligomeric compound of any of embodiments 1-41, wherein the modified oligonucleotide consists of 16 nucleosides.
Embodiment 44: The oligomeric compound of any of embodiments 1-41, wherein the modified oligonucleotide consists of 17 nucleosides.
Embodiment 45: The oligomeric compound of any of embodiments 1-41, wherein the modified oligonucleotide consists of 18 nucleosides.
Embodiment 46: The oligomeric compound of any of embodiments 1-41, wherein the modified oligonucleotide consists of 19 nucleosides.
Embodiment 47: The oligomeric compound of any of embodiments 1-41, wherein the modified oligonucleotide consists of 20 nucleosides.
Embodiment 48: The oligomeric compound of any of embodiments 1-47, wherein the modified oligonucleotide comprises at least one modified internucleoside linkage.
Embodiment 49: The oligomeric compound of any of embodiments 1-48, wherein the modified oligonucleotide comprises at least one phosphorothioate internucleoside linkage.
Embodiment 50: The oligomeric compound of embodiment 49, wherein each internucleoside linkage of the modified oligonucleotide is selected from among a phosphorothioate internucleoside linkage and a phosphate internucleoside linkage.
Embodiment 51: The oligomeric compound of embodiment 50, wherein the phosphate internucleoside linkage is a phosphodiester internucleoside linkage.
Embodiment 52: The oligomeric compound of any of embodiments 1-50, wherein each internucleoside linkage of the modified oligonucleotide is a phosphorothioate internucleoside linkage.
Embodiment 53: The oligomeric compound of any of embodiments 1-52, wherein the modified oligonucleotide comprises at least one modified nucleobase.
Embodiment 54: The oligomeric compound of any of embodiments 1-53, wherein the modified oligonucleotide comprises at least one 5-methyl cytosine.
Embodiment 55: The oligomeric compound of any of embodiments 1-54, wherein each nucleobase of the modified oligonucleotide is selected from among thymine, 5-methyl cytosine, cytosine, adenine, uracil, and guanine.
Embodiment 56: The oligomeric compound of any of embodiments 1-55, wherein each cytosine of the modified oligonucleotide is a 5-methyl cytosine.
Embodiment 57: The oligomeric compound of any of embodiments 1-56, wherein each nucleobase of the modified oligonucleotide is selected from among thymine, 5-methyl cytosine, adenine, and guanine.
Embodiment 58: The oligomeric compound of any of embodiments 1-57, wherein the modified oligonucleotide is at least 70% complementary to the SMN2 pre-mRNA.
Embodiment 59: The oligomeric compound of any of embodiments 1-57, wherein the modified oligonucleotide is at least 75% complementary to the SMN2 pre-mRNA.
Embodiment 60: The oligomeric compound of any of embodiments 1-57, wherein the modified oligonucleotide is at least 80% complementary to the SMN2 pre-mRNA.
Embodiment 61: The oligomeric compound of any of embodiments 1-57, wherein the modified oligonucleotide is at least 85% complementary to the SMN2 pre-mRNA.
Embodiment 62: The oligomeric compound of any of embodiments 1-57, wherein the modified oligonucleotide is at least 90% complementary to the SMN2 pre-mRNA.
Embodiment 63: The oligomeric compound of any of embodiments 1-57, wherein the modified oligonucleotide is at least 95% complementary to the SMN2 pre-mRNA.
Embodiment 64: The oligomeric compound of any of embodiments 1-57, wherein the modified oligonucleotide is at least 100% complementary to the SMN2 pre-mRNA.
Embodiment 65: The oligomeric compound of any of embodiments 1-64, wherein the modified oligonucleotide is complementary to intron 7 of the SMN2 pre-mRNA.
Embodiment 66: The oligomeric compound of any of embodiments 1-64, wherein the modified oligonucleotide is complementary to ISS-N1 of the SMN2 pre-mRNA.
Embodiment 67: The oligomeric compound of any of embodiments 1-64, wherein the nucleobase sequence of the modified oligonucleotide comprises a sequence selected from among SEQ ID Numbers 1, 2, or 3.
Embodiment 68: The oligomeric compound of any of embodiments 1-64, wherein the nucleobase sequence of the modified oligonucleotide comprises a sequence selected from among any of SEQ ID Numbers 12-239.
Embodiment 69: The oligomeric compound of any of embodiments 1-64, wherein the nucleobase sequence of the modified oligonucleotide consists of a sequence selected from among any of SEQ ID Numbers 1, 2, 3, or 12-239.
Embodiment 70: The oligomeric compound of embodiment 1-64, wherein the modified oligonucleotide is complementary to exon 7 of the SMN2 pre-mRNA.
Embodiment 71: The oligomeric compound of embodiment 1-64, wherein the modified oligonucleotide is complementary to intron 6 of the SMN2 pre-mRNA.
Embodiment 72: The oligomeric compound of any of embodiments 1-64, wherein the modified oligonucleotide is complementary to an exonic splicing enhancer in exon 7 of the SMN2 pre-mRNA.
Embodiment 73: The oligomeric compound of any of embodiments 1-64, wherein the modified oligonucleotide is complementary to an aberrant exonic splicing silencer in exon 7 of the SMN2 pre-mRNA.
Embodiment 74: The oligomeric compound of any of embodiments 1-73, wherein the oligomeric compound comprises a conjugate group.
Embodiment 75: The oligomeric compound of embodiment 74, wherein the conjugate group comprises a lipid or lipophilic group.
Embodiment 76: The oligomeric compound of embodiment 75, wherein the lipid or lipophilic group is selected from among: cholesterol, a C₁₀-C₂₆ saturated fatty acid, a C₁₀- C₂₆ unsaturated fatty acid, C₁₀-C₂₆ alkyl, a triglyceride, tocopherol, or cholic acid.
Embodiment 77: The oligomeric compound of embodiment 76, wherein the lipid or lipophilic group is a saturated hydrocarbon chain or an unsaturated hydrocarbon chain.
Embodiment 78: The oligomeric compound of any of embodiments 75-77, wherein the lipid or lipophilic group is a C₁₆ lipid.
Embodiment 79: The oligomeric compound of any of embodiments 75-77, wherein the lipid or lipophilic group is a C₁₈ lipid.
Embodiment 80: The oligomeric compound of any of embodiments 75-77, wherein the lipid or lipophilic group is C₁₆ alkyl.
Embodiment 81: The oligomeric compound of any of embodiments 75-77, wherein the lipid or lipophilic group is C₁₈ alkyl.
Embodiment 82: The oligomeric compound of embodiment 76, wherein the lipid or lipophilic group is cholesterol.
Embodiment 83: The oligomeric compound of embodiment 76, wherein the lipid or lipophilic group is tocopherol.
Embodiment 84: The oligomeric compound of embodiment 76, wherein the lipid or lipophilic group is saturated C₁₆.
Embodiment 85: The oligomeric compound of any of embodiments 74-84, wherein the conjugate group is attached to the modified oligonucleotide at the 5'-end of the modified oligonucleotide.
Embodiment 86: The oligomeric compound of any of embodiments 74-85, wherein the conjugate group is attached to the modified oligonucleotide at the 3'-end of the modified oligonucleotide.
Embodiment 87: The oligomeric compound of any of embodiments 74-86, wherein the conjugate group comprises a cleavable linker.
Embodiment 88: The oligomeric compound of embodiment 87 wherein the cleavable linker comprises one or more linker nucleosides.
Embodiment 89: The oligomeric compound of any of embodiments 1-73 consisting of the modified oligonucleotide.
Embodiment 90: The oligomeric compound of any of embodiments 74-88 consisting of the modified oligonucleotide and the conjugate group.
Embodiment 91: The oligomeric compound of any of embodiments 1-90, wherein the oligomeric compound modulates splicing of the SMN2 pre-mRNA.
Embodiment 92: The oligomeric compound of any of embodiments 1-91, wherein the oligomeric compound is single stranded.
Embodiment 93: The oligomeric compound of any of embodiments 1-91, wherein the oligomeric compound is paired with a complementary oligomeric compound to form a double stranded compound.
Embodiment 94: The oligomeric compound of embodiment 93, wherein the complementary oligomeric compound comprises a conjugate group.
Embodiment 95: A pharmaceutical composition comprising the oligomeric compound of any of embodiments 1-94 and at least one pharmaceutically acceptable carrier or diluent.
Embodiment 96: A method of modulating splicing of an SMN2 pre-mRNA comprising contacting a cell, tissue, or organ with the oligomeric compound or composition of any of embodiments 1-95.
Embodiment 97: A method of promoting inclusion of exon 7 in SMN2 mRNA in a cell, tissue or organ, comprising contacting said cell, tissue or organ with the oligomeric compound of any of embodiments 1 to 91 or the composition of embodiment 95.
Embodiment 98: The method of embodiment 96 or 97, wherein the cell, tissue, or organ is in an animal.
Embodiment 99: The method of embodiments 96 or 97, wherein the cell, tissue, or organ is in a human.
Embodiment 100: A method of treating Spinal Muscular Atrophy in a patient, comprising administering the oligomeric compound of any of embodiments 1 to 94 or the composition of embodiment 95 to a patient in need thereof.
Embodiment 101: A method of treating Spinal Muscular Atrophy Type I in a patient, comprising administering the oligomeric compound of any of embodiments 1 to 94 or the composition of embodiment 95 to a patient in need thereof.
Embodiment 102: A method of treating Spinal Muscular Atrophy Type II in a patient, comprising administering the oligomeric compound of any of embodiments 1 to 94 or the composition of embodiment 95 to a patient in need thereof.
Embodiment 103: A method of treating Spinal Muscular Atrophy Type III in a patient, comprising administering the oligomeric compound of any of embodiments 1 to 94 or the composition of embodiment 95 to a patient in need thereof.
Embodiment 104: A method of treating Spinal Muscular Atrophy Type IV in a patient, comprising administering the oligomeric compound of any of embodiments 1 to 94 or the composition of embodiment 95 to a patient in need thereof.
Embodiment 105: The method of any of embodiments 100-104, wherein the oligomeric compound is administered to the central nervous system.
Embodiment 106: The method of embodiment 105, wherein the oligomeric compound is administered intrathecally.
Embodiment 107: The method of any of embodiments 100-104, wherein the oligomeric compound is administered systemically.
Embodiment 108: The method of embodiment 107, wherein the oligomeric compound is administered subcutaneously.
Embodiment 109: The method of embodiment 107, wherein the oligomeric compound is administered intramuscularly.
Embodiment 110: The method of any of embodiments 100-109, wherein the oligomeric compound is administered to the central nervous system and systemically.
Embodiment 111: The method of any of embodiments 100-110, wherein the oligomeric compound is co-administered with a second compound.
Embodiment 112: The method of embodiment 111, wherein the second compound is selected from among a small molecule and an oligomeric compound.
Embodiment 113: The method of embodiment 112, wherein the second compound is an oligomeric compound according to embodiments 1-94.
Embodiment 114: The method of any of embodiments 111-113, wherein the second compound is administered to the CSF.
Embodiment 115: The method of embodiment 114, wherein the second compound is administered intrathecally.
Embodiment 116: The method of any of embodiments 111-115, wherein the second compound is a second oligomeric compound comprising a second modified oligonucleotide having at least one modified nucleoside comprising a modified sugar moiety.
Embodiment 117: The method of embodiment 116, wherein the modified sugar moiety of the modified nucleoside of the second modified oligonucleotide of the second oligomeric compounds is selected from 2'-OMe and 2'-MOE.
Embodiment 118: The method of any of embodiments 116-117, wherein each nucleoside of the second modified oligonucleotide comprises a modified sugar moiety.
Embodiment 119: The method of embodiment 118, wherein the modified sugar moiety of the second oligonucleotide is a 2'-MOE sugar moiety.
Embodiment 120: The method of embodiment 111 or 112, wherein the second compound is an oligomeric compound comprising a morpholino oligonucleotide.
Embodiment 121: The method of any of embodiments 111-120, wherein the second oligomeric compound has the nucleobase sequence of SEQ ID NO: 1.
Embodiment 122: The method of embodiment 121, wherein the second oligomeric compound has the nucleobase sequence and chemical modification motif of Isis 396443.
Embodiment 123: The method of any of embodiments 111-122, wherein the second oligomeric compound consists of the modified oligonucleotide.
Embodiment 124: The method of any of embodiments 111-123, wherein the second compound is administered prior to administration of the oligomeric compound of any of embodiments 1 to 94 or the composition of embodiment 95.
Embodiment 125: The method of any of embodiments 111-123, wherein the second compound is administered after administration of the oligomeric compound of any of embodiments 1 to 94 or the composition of embodiment 95.
Embodiment 126: The method of any of embodiments 111-123, wherein the second compound is administered less frequently than administration of the oligomeric compound of any of embodiments 1 to 94 or the composition of embodiment 95.
Embodiment 127: An oligomeric compound of any of embodiments 1 to 94 or the composition of embodiment 95 for use in therapy.
Embodiment 128: Use of an oligomeric compound of any of embodiments 1 to 94 or the composition of embodiment 95 for the preparation of a medicament for the treatment of Spinal Muscular Atrophy Type I.
Embodiment 129: Use of an oligomeric compound of any of embodiments 1 to 94 or the composition of embodiment 95 for the preparation of a medicament for the treatment of Spinal Muscular Atrophy Type II
Embodiment 130: Use of an oligomeric compound of any of embodiments 1 to 94 or the composition of embodiment 95 for the preparation of a medicament for the treatment of Spinal Muscular Atrophy Type III.
Embodiment 131: Use of an oligomeric compound of any of embodiments 1 to 94 or the composition of embodiment 95 for the preparation of a medicament for the treatment of Spinal Muscular Atrophy Type IV.
Embodiment 132: Use of an oligomeric compound of any of embodiments 1 to 95 for the preparation of a medicament for the treatment of Spinal Muscular Atrophy.
Embodiment 133: A method comprising (i) systemically administering a compound comprising the oligomeric compound of any of embodiments 1-73 and (ii) administering to the CNS a compound comprising the oligomeric compound of any of embodiments 1-73.
Embodiment 134: A method comprising (i) systemically administering a compound comprising the oligomeric compound or composition of any of embodiments 1-73 and (ii) administering to the CNS a compound comprising a modified oligonucleotide having at least one modified nucleoside comprising a modified sugar moiety selected from 2'-OMe and 2'-MOE.
Embodiment 135: A method comprising (i) systemically administering a compound comprising the oligomeric compound of any of embodiments 74-94 and (ii) administering to the CNS a compound comprising the oligomeric compound of any of embodiments 1-73. A method comprising (i) systemically administering a compound comprising the oligomeric compound of any of embodiments 74-94 and (ii) administering to the CNS a compound comprising a modified oligonucleotide having at least one modified nucleoside comprising a modified sugar moiety selected from 2'-OMe and 2'-MOE.
Embodiment 136: A method comprising (i) systemically administering a modified oligonucleotide having at least one modified nucleoside comprising a modified sugar moiety selected from 2'-OMe and 2'-MOE and a conjugate, and (ii) administering to the CNS a compound comprising the oligomeric compound of any of embodiments 1-73.
Embodiment 137: A method comprising (i) systemically administering a modified oligonucleotide having at least one modified nucleoside comprising a modified sugar moiety selected from 2'-OMe and 2'-MOE and a conjugate group, and (ii) administering to the CNS a compound comprising a modified oligonucleotide having at least one modified nucleoside comprising a modified sugar moiety selected from 2'-OMe and 2'-MOE.
Embodiment 138: The method of embodiment 137, wherein the conjugate group comprises a lipid or lipophilic group selected from cholesterol, a C10-C26 saturated fatty acid, a C10- C26 unsaturated fatty acid, C10-C26 alkyl, a triglyceride, tocopherol, and cholic acid.
Embodiment 139: The compound or method of any of embodiments 1-138, wherein the SMN2 pre-mRNA is a nucleobase sequence selected from any of SEQ ID NO: 10, 11, or 240.

### DETAILED DESCRIPTION

It is to be understood that both the foregoing general description and the following detailed description are exemplary and explanatory only and are not restrictive of the embodiments, as claimed. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting.

The section headings used herein are for organizational purposes only and are not to be construed as limiting the subject matter described. All documents, or portions of documents, cited in this application, including, but not limited to, patents, patent applications, articles, books, treatises, and GenBank and NCBI reference sequence records are hereby expressly incorporated by reference for the portions of the document discussed herein, as well as in their entirety.

Unless otherwise indicated, the following terms have the following meanings:
As used herein, "SMN2 pre-mRNA" means an RNA sequence, including all exons, introns, and untranslated regions, transcribed from DNA encoding human SMN2. In certain embodiments, DNA encoding SMN2 includes the human SMN2 genomic sequence provided in GENBANK Accession No. NT_006713.14 truncated from nucleotides 19939708_to 19967777, incorporated by reference herein as SEQ ID NO: 240. In certain embodiments, SMN2 pre-mRNA comprises SEQ ID NO: 10. Nucleotides 1-60 of SEQ ID NO: 10 represent a portion of intron 6, nucleotides 61-114 of SEQ ID NO: 10 represent exon 7, and nucleotides 115-174 of SEQ ID NO: 10 represent a portion of intron 7.

As used herein, "ISS-N1" means an intronic splice silencing domain in intron 7. In certain embodiments, ISS-N1 comprises the nucleobase sequence of SEQ ID NO: 11.

As used herein, "2'-deoxyribonucleoside" means a nucleoside comprising 2'-H(H) furanosyl sugar moiety, as found in naturally occurring deoxyribonucleic acids (DNA). In certain embodiments, a 2'-deoxyribonucleoside may comprise a modified nucleobase or may comprise an RNA nucleobase (uracil).

As used herein, "2'-substituted nucleoside" or "2-modified nucleoside" means a nucleoside comprising a 2'-substituted or 2'-modified sugar moiety. As used herein, "2'-substituted" or "2-modified" in reference to a sugar moiety means a sugar moiety comprising at least one 2'-substituent group other than H or OH.

As used herein, "antisense activity" means any detectable and/or measurable change attributable to the hybridization of an antisense compound to its target nucleic acid. In certain embodiments, antisense activity is a decrease in the amount or expression of a target nucleic acid or protein encoded by such target nucleic acid compared to target nucleic acid levels or target protein levels in the absence of the antisense compound.

As used herein, "antisense compound" means a compound comprising an antisense oligonucleotide and optionally one or more additional features, such as a conjugate group or terminal group.

As used herein, "antisense oligonucleotide" means an oligonucleotide having a nucleobase sequence that is at least partially complementary to a target nucleic acid.

As used herein, "ameliorate" in reference to a treatment means improvement in at least one symptom relative to the same symptom in the absence of the treatment. In certain embodiments, amelioration is the reduction in the severity or frequency of a symptom or the delayed onset or slowing of progression in the severity or frequency of a symptom.

As used herein, "bicyclic nucleoside" or "BNA" means a nucleoside comprising a bicyclic sugar moiety. As used herein, "bicyclic sugar" or "bicyclic sugar moiety" means a modified sugar moiety comprising two rings, wherein the second ring is formed via a bridge connecting two of the atoms in the first ring thereby forming a bicyclic structure. In certain embodiments, the first ring of the bicyclic sugar moiety is a furanosyl moiety. In certain such embodiments, the furanosyl moiety is a ribosyl moiety. In certain embodiments, the bicyclic sugar moiety does not comprise a furanosyl moiety.

As used herein, "branching group" means a group of atoms having at least 3 positions that are capable of forming covalent linkages to at least 3 groups. In certain embodiments, a branching group provides a plurality of reactive sites for connecting tethered ligands to an oligonucleotide via a conjugate linker and/or a cleavable moiety.

As used herein, "cell-targeting moiety" means a conjugate group or portion of a conjugate group that results in improved uptake to a particular cell type and/or distribution to a particular tissue relative to an oligomeric compound lacking the cell-targeting moiety.

As used herein, "cleavable moiety" means a bond or group of atoms that is cleaved under physiological conditions, for example, inside a cell, an animal, or a human.

As used herein, "complementary" in reference to an oligonucleotide means that at least 70% of the nucleobases of such oligonucleotide or one or more regions thereof and the nucleobases of another nucleic acid or one or more regions thereof are capable of hydrogen bonding with one another when the nucleobase sequence of the oligonucleotide and the other nucleic acid are aligned in opposing directions. Complementary nucleobases means nucleobases that are capable of forming hydrogen bonds with one another. Complementary nucleobase pairs include adenine (A) and thymine (T), adenine (A) and uracil (U), cytosine (C) and guanine (G), 5-methyl cytosine (^{m}C) and guanine (G). Complementary oligonucleotides and/or nucleic acids need not have nucleobase complementarity at each nucleoside. Rather, some mismatches are tolerated. As used herein, "fully complementary" or "100% complementary" in reference to oligonucleotides means that such oligonucleotides are complementary to another oligonucleotide or nucleic acid at each nucleoside of the oligonucleotide.

As used herein, "conjugate group" means a group of atoms that is directly or indirectly attached to an oligonucleotide. Conjugate groups include a conjugate moiety and a conjugate linker that attaches the conjugate moiety to the oligonucleotide.

As used herein, "conjugate linker" means a group of atoms comprising at least one bond that connects a conjugate moiety to an oligonucleotide.

As used herein, "conjugate moiety" means a group of atoms that is attached to an oligonucleotide via a conjugate linker.

As used herein, "contiguous" in the context of an oligonucleotide refers to nucleosides, nucleobases, sugar moieties, or internucleoside linkages that are immediately adjacent to each other. For example, "contiguous nucleobases" means nucleobases that are immediately adjacent to each other in a sequence.

As used herein, "double-stranded antisense compound" means an antisense compound comprising two oligomeric compounds that are complementary to each other and form a duplex, and wherein one of the two said oligomeric compounds comprises an antisense oligonucleotide.

As used herein, "fully modified" in reference to a modified oligonucleotide means a modified oligonucleotide in which each sugar moiety is modified. "Uniformly modified" in reference to a modified oligonucleotide means a fully modified oligonucleotide in which each sugar moiety is the same. For example, the nucleosides of a uniformly modified oligonucleotide can each have a 2'-MOE modification but different nucleobase modifications, and the internucleoside linkages may be different.

As used herein, "gapmer" means a modified oligonucleotide comprising an internal region having a plurality of nucleosides comprising unmodified sugar moieties positioned between external regions having one or more nucleosides comprising modified sugar moieties, wherein the nucleosides of the external regions that are adjacent to the internal region each comprise a modified sugar moiety. The internal region may be referred to as the "gap" and the external regions may be referred to as the "wings."

As used herein, "hybridization" means the pairing or annealing of complementary oligonucleotides and/or nucleic acids. While not limited to a particular mechanism, the most common mechanism of hybridization involves hydrogen bonding, which may be Watson-Crick, Hoogsteen or reversed Hoogsteen hydrogen bonding, between complementary nucleobases.

As used herein, "inhibiting the expression or activity" refers to a reduction or blockade of the expression or activity relative to the expression of activity in an untreated or control sample and does not necessarily indicate a total elimination of expression or activity.

As used herein, the terms "internucleoside linkage" means a group or bond that forms a covalent linkage between adjacent nucleosides in an oligonucleotide. As used herein "modified internucleoside linkage" means any internucleoside linkage other than a naturally occurring, phosphate internucleoside linkage. Non-phosphate linkages are referred to herein as modified internucleoside linkages. "Phosphorothioate linkage" means a modified phosphate linkage in which one of the non-bridging oxygen atoms is replaced with a sulfur atom. A phosphorothioate internucleoside linkage is a modified internucleoside linkage. Modified internucleoside linkages include linkages that comprise abasic nucleosides. As used herein, "abasic nucleoside" means a sugar moiety in an oligonucleotide or oligomeric compound that is not directly connected to a nucleobase. In certain embodiments, an abasic nucleoside is adjacent to one or two nucleosides in an oligonucleotide.

As used herein "Isis 396443" means an oligonucleotide having the following structure:
Tes ^{m}Ces Aes ^{m}Ces Tes Tes Tes ^{m}Ces Aes Tes Aes Aes Tes Ges ^{m}Ces Tes Ges Ge
wherein "^{m}C" indicates 5-methyl cytosine; "e" indicates a 2'-MOE modification; "C" indicates cytidine, "T" indicates thymidine, "A" indicates adenosine, "G" indicates guanosine, and "s" indicates phosphorothioate linkage. Isis 396443 is also referred to in the art as Nusinersen and as lonis-SMNRx.

As used herein, "linker-nucleoside" means a nucleoside that links, either directly or indirectly, an oligonucleotide to a conjugate moiety. Linker-nucleosides are located within the conjugate linker of an oligomeric compound. Linker-nucleosides are not considered part of the oligonucleotide portion of an oligomeric compound even if they are contiguous with the oligonucleotide.

As used herein, "non-bicyclic modified sugar" or "non-bicyclic modified sugar moiety" means a modified sugar moiety that comprises a modification, such as a substitutent, that does not form a bridge between two atoms of the sugar to form a second ring.

As used herein, "linked nucleosides" are nucleosides that are connected in a continuous sequence (i.e. no additional nucleosides are present between those that are linked).

As used herein, "mismatch" or "non-complementary" means a nucleobase of a first oligonucleotide that is not complementary with the corresponding nucleobase of a second oligonucleotide or target nucleic acid when the first and second oligomeric compound are aligned.

As used herein, "MOE" means methoxyethyl. "2'-MOE" means a -OCH₂CH₂OCH₃ group at the 2' position of a furanosyl ring.

As used herein, "motif' means the pattern of unmodified and/or modified sugar moieties, nucleobases, and/or internucleoside linkages, in an oligonucleotide.

As used herein, "naturally occurring" means found in nature.

As used herein, "nucleobase" means a naturally occurring nucleobase or a modified nucleobase. As used herein a "naturally occurring nucleobase" is adenine (A), thymine (T), cytosine (C), uracil (U), and guanine (G). As used herein, a modified nucleobase is a group of atoms capable of pairing with at least one naturally occurring nucleobase. A universal base is a nucleobase that can pair with any one of the five unmodified nucleobases. As used herein, "nucleobase sequence" means the order of contiguous nucleobases in a nucleic acid or oligonucleotide independent of any sugar or internucleoside linkage modification.

As used herein, "nucleoside" means a compound comprising a nucleobase and a sugar moiety. The nucleobase and sugar moiety are each, independently, unmodified or modified. As used herein, "modified nucleoside" means a nucleoside comprising a modified nucleobase and/or a modified sugar moiety.

As used herein, "2'-O-(N-alkyl acetamide)" means a -O-CH₂-C(O)-NH-alkyl group at the 2' position of a furanosyl ring.

As used herein, "2'-O-(N-methyl acetamide)" or "2'-NMA" means a -O-CH₂-C(O)-NH-CH₃ group at the 2' position of a furanosyl ring.

As used herein, "oligomeric compound" means a compound consisting of an oligonucleotide and optionally one or more additional features, such as a conjugate group or terminal group.

As used herein, "oligonucleotide" means a strand of linked nucleosides connected via internucleoside linkages, wherein each nucleoside and internucleoside linkage may be modified or unmodified. Unless otherwise indicated, oligonucleotides consist of 8-50 linked nucleosides. As used herein, "modified oligonucleotide" means an oligonucleotide, wherein at least one nucleoside or internucleoside linkage is modified. As used herein, "unmodified oligonucleotide" means an oligonucleotide that does not comprise any nucleoside modifications or internucleoside modifications.

As used herein, "pharmaceutically acceptable carrier or diluent" means any substance suitable for use in administering to an animal. Certain such carriers enable pharmaceutical compositions to be formulated as, for example, tablets, pills, dragees, capsules, liquids, gels, syrups, slurries, suspension and lozenges for the oral ingestion by a subject. In certain embodiments, a pharmaceutically acceptable carrier or diluent is sterile water; sterile saline; or sterile buffer solution.

As used herein "pharmaceutically acceptable salts" means physiologically and pharmaceutically acceptable salts of compounds, such as oligomeric compounds, *i.e.,* salts that retain the desired biological activity of the parent compound and do not impart undesired toxicological effects thereto.

As used herein "pharmaceutical composition" means a mixture of substances suitable for administering to a subject. For example, a pharmaceutical composition may comprise an antisense compound and a sterile aqueous solution. In certain embodiments, a pharmaceutical composition shows activity in free uptake assay in certain cell lines.

As used herein, "phosphorus moiety" means a group of atoms comprising a phosphorus atom. In certain embodiments, a phosphorus moiety comprises a mono-, di-, or tri-phosphate, or phosphorothioate.

As used herein, "phosphodiester internucleoside linkage" means a phosphate group that is covalently bonded to two adjacent nucleosides of a modified oligonucleotide.

As used herein, "precursor transcript" means a coding or non-coding RNA that undergoes processing to form a processed or mature form of the transcript. Precursor transcripts include but are not limited to pre-mRNAs, long non-coding RNAs, pri-miRNAs, and intronic RNAs.

As used herein, "processing" in reference to a precursor transcript means the conversion of a precursor transcript to form the corresponding processed transcript. Processing of a precursor transcript includes but is not limited to nuclease cleavage events at processing sites of the precursor transcript.

As used herein "prodrug" means a therapeutic agent in a form outside the body that is converted to a differentform within the body or cells thereof. Typically conversion of a prodrug within the body is facilitated by the action of an enzymes (e.g., endogenous or viral enzyme) or chemicals present in cells or tissues and/or by physiologic conditions.

As used herein, "RNAi compound" means an antisense compound that acts, at least in part, through RISC or Ago2 to modulate a target nucleic acid and/or protein encoded by a target nucleic acid. RNAi compounds include, but are not limited to double-stranded siRNA, single-stranded RNA (ssRNA), and microRNA, including microRNA mimics. In certain embodiments, an RNAi compound modulates the amount, activity, and/or splicing of a target nucleic acid. The term RNAi compound excludes antisense oligonucleotides that act through RNase H.

As used herein, the term "single-stranded" in reference to an antisense compound means such a compound consisting of one oligomeric compound that is not paired with a second oligomeric compound to form a duplex. "Self-complementary" in reference to an oligonucleotide means an oligonucleotide that at least partially hybridizes to itself. A compound consisting of one oligomeric compound, wherein the oligonucleotide of the oligomeric compound is self-complementary, is a single-stranded compound. A single-stranded antisense or oligomeric compound may be capable of binding to a complementary oligomeric compound to form a duplex.

As used herein, "splicing" means the process by which a pre-mRNA is processed to form the corresponding mRNA. Splicing includes but is not limited to the removal of introns from pre-mRNA and the joining together of exons.

As used herein, "sugar moiety" means an unmodified sugar moiety or a modified sugar moiety. As used herein, "unmodified sugar moiety" means a 2'-OH(H) furanosyl moiety, as found in RNA (an "unmodified RNA sugar moiety"), or a 2'-H(H) moiety, as found in DNA (an "unmodified DNA sugar moiety"). Unmodified sugar moieties have one hydrogen at each of the 1', 3', and 4' positions, an oxygen at the 3' position, and two hydrogens at the 5' position. As used herein, "modified sugar moiety" or "modified sugar" means a modified furanosyl sugar moiety or a sugar surrogate. As used herein, modified furanosyl sugar moiety means a furanosyl sugar comprising a non-hydrogen substituent in place of at least one hydrogen of an unmodified sugar moiety. In certain embodiments, a modified furanosyl sugar moiety is a 2'-substituted sugar moiety. Such modified furanosyl sugar moieties include bicyclic sugars and non-bicyclic sugars. As used herein, "sugar surrogate" means a modified sugar moiety having other than a furanosyl moiety that can link a nucleobase to another group, such as an internucleoside linkage, conjugate group, or terminal group in an oligonucleotide. Modified nucleosides comprising sugar surrogates can be incorporated into one or more positions within an oligonucleotide and such oligonucleotides are capable of hybridizing to complementary oligomeric compounds or nucleic acids.

As used herein, "target precursor transcript," mean a precursor transcript to which an oligonucleotide is designed to hybridize. In certain embodiments, a target precursor transcript is a target pre-mRNA. As used herein, "target processed transcript" means the RNA that results from processing of the corresponding target precursor transcript. In certain embodiments, a target processed transcript is a target mRNA. As used herein, "target pre-mRNA" means a pre-mRNA to which an oligonucleotide is designed to hybridize. As used herein, "target mRNA" means a mRNA that results from the splicing of the corresponding target pre-mRNA.

As used herein, "terminal group" means a chemical group or group of atoms that is covalently linked to a terminus of an oligonucleotide.

### Spinal Muscular Atrophy

SMA is a genetic disorder characterized by degeneration of spinal motor neurons. SMA is caused by the homozygous loss of both functional copies of the SMN1 gene. However, the SMN2 gene has the potential to code for the same protein as SMN1 and thus overcome the genetic defect of SMA patients. SMN2 contains a translationally silent mutation (C→T) at position +6 of exon 7, which results in inefficient inclusion of exon 7 in SMN2 transcripts. Therefore, the predominant form of SMN2, one which lacks exon 7, is unstable and inactive. Thus, therapeutic compounds capable of modulating SMN2 splicing such that the percentage of SMN2 transcripts containing exon 7 is increased would be useful for the treatment of SMA. In certain embodiments, modified oligonucleotides having one or more 2'-O-(N-alkyl acetamide) modified sugar moieties have enhanced pharmacologic activity for modulation of SMN2 pre-mRNA, including increasing the percentage of SMN2 transcripts containing exon 7. In certain embodiments, modified oligonucleotides having one or more 2'-O-(N-methyl acetamide) modified sugar moieties have enhanced pharmacologic activity for modulation of SMN2 pre-mRNA, including increasing the percentage of SMN2 transcripts containing exon 7.

Although SMA is generally characterized by degeneration of spinal motor neurons, SMN2 is ubiquitously expressed in cells throughout the body, including muscle cells. In certain embodiments, improved SMN activity in muscle cells provides a therapeutic benefit. In certain embodiments, modified oligonucleotides having one or more 2'-O-(N-alkyl acetamide) modified sugar moieties have enhanced cellular distribution to tissues throughout the body, including muscle tissue. In certain embodiments, modified oligonucleotides having one or more 2'-O-(N-methyl acetamide) modified sugar moieties have enhanced cellular distribution to tissues throughout the body, including muscle tissue.

In certain embodiments, modified oligonucleotides having one or more 2'-O-(N-alkyl acetamide) modified sugar moieties comprise a conjugate which can further enhance cellular distribution and/or pharmacologic activity. In certain embodiments, the conjugate enhances distribution to muscle tissue. In certain embodiments, the conjugate is a lipid.

In certain embodiments, modified oligonucleotides having one or more 2'-O-(N-methyl acetamide) modified sugar moieties comprise a conjugate which can further enhance cellular distribution and/or pharmacologic activity. In certain embodiments, the conjugate enhances distribution to muscle tissue. In certain embodiments, the conjugate is a lipid.

Certain nucleobase sequences targeted to SMN2 pre-mRNA are exemplified in the non-limiting table below. Any of the nucleobase sequences in the table below may be modified with six or more 2'-O-(N-alkyl acetamide) modified sugar moieties. Any of the nucleobase sequences in the table below may be modified with six or more 2'-O-(N-methyl acetamide) modified sugar moieties.

Any of the nucleobase sequences in the table below may be modified with six or more 2'-O-(N-alkyl acetamide) modified sugar moieties and may comprise a conjugate moiety. Any of the nucleobase sequences in the table below may be modified with six or more 2'-O-(N-methyl acetamide) modified sugar moieties and may comprise a conjugate moiety.

### Nucleobase Sequences Targeting SMN2 Pre-mRNA

| **Sequence** | **Length** | **SEQ ID NO** |
|---|---|---|
| AUUCACUUUCAUAAUGCUGG | 20 | 12 |
| CUUUCAUAAUGCUGG | 15 | 13 |
| UUUCAUAAUGCUGGC | 15 | 14 |
| UUCAUAAUGCUGGCA | 15 | 15 |
| UCAUAAUGCUGGCAG | 15 | 16 |
| ACUUUCAUAAUGCUG | 15 | 17 |
| CACUUUCAUAAUGCU | 15 | 18 |
| UCACUUUCAUAAUGC | 15 | 19 |
| UUUCAUAAUGCUGG | 14 | 20 |
| CUUUCAUAAUGCUG | 14 | 21 |
| UUCAUAAUGCUGG | 13 | 22 |
| CUUUCAUAAUGCU | 13 | 23 |
| UUUCAUAAUGCUG | 13 | 24 |
| UCAUAAUGCUGG | 12 | 25 |
| UUCAUAAUGCUG | 12 | 26 |
| UUUCAUAAUGCU | 12 | 27 |
| TTTCATAATGCTGG | 14 | 28 |
| CTTTCATAATGCTG | 14 | 29 |
| TGGTGTCATTTAGTGCTGCT | 20 | 30 |
| CATATAATAGCCAGTATGATAGCC | 24 | 31 |
| TCACTTTCATCTGTTGAAACTTGG | 24 | 32 |
| CCUCUGUGGACACCAG | 16 | 33 |
| CUUUCAUAAUGCUGG | 15 | 13 |
| UUAAUUUAAGGAA | 13 | 34 |
| UUAAUUUAAGGAAUGUG | 17 | 35 |
| CACUUUCAUAAUGCUGG | 17 | 36 |
| UUUGAUUUUGUCUAAAACC | 19 | 37 |
| GAUUUUGUCUAAAACCCUG | 19 | 38 |
| UUUGAUUUUGACUAAAACC | 19 | 39 |
| GAUUUUGUCAAAAACCCUG | 19 | 40 |
| GCUAUACCAGCGUCGUCAU | 19 | 41 |
| UAGCUUUAUAUGGAUGUUA | 19 | 42 |
| UAAAACCCUGUAAGGAAAA | 19 | 43 |
| UCUAAAACCCUGUAAGGAA | 19 | 44 |
| CUUUCAUAAUGCUGGCAGA | 19 | 45 |
| UCACUUUCAUAAUGCUGGC | 19 | 46 |
| CUUUCUAACAUCUGAACUU | 19 | 47 |
| CAACUUUCUAACAUCUGAA | 19 | 48 |
| UAGCUUUAUUUGGAUGUUA | 19 | 49 |
| GUUUCACAACACAUUUUAC | 19 | 50 |
| UUUUGUCUAAUACCCUGUA | 19 | 51 |
| AGGAAUGUGACCACCUUCC | 19 | 52 |
| CUUUCAUAAAGCUGGCAGA | 19 | 53 |
| UCACUUUCAAAAUGCUGGC | 19 | 54 |
| CUUUCUAACUUCUGAACUU | 19 | 55 |
| AUUCACUUUCAUAAUGCUGG | 20 | 12 |
| TTTTTGATTTTGTCT | 15 | 56 |
| ATTTAAGGAATGTGA | 15 | 57 |
| ATTCACTTTCATAATGCTG | 19 | 58 |
| ATTCACTTTCATAATGCTGG | 20 | 59 |
| TCCTTTAAAGTATTGTGACC | 20 | 2 |
| TTATATACTTTTAAACATATAGAAGATAG | 29 | 60 |
| AGATTCTCTTGATGATGCTGAATG | 24 | 61 |
| GTTTCAGACAAAATCAAAAAGAAGGA | 26 | 62 |
| TCTATAACGCTTCACATTCCAGATCT | 26 | 63 |
| ATGCCAGCATTTCTCCTTAATTTAAGG | 27 | 64 |
| GTCAACCCCACCGTGTTCTT | 20 | 65 |
| TTGGAACTTTGTCTGCAAACA | 21 | 66 |
| CTTGGGCCGCGTCT | 14 | 67 |
| CCTCTTACCTCAGTTACAATTTATA | 25 | 68 |
| AAGTCTGCAGGTCTGCCTACTAGTG | 25 | 69 |
| AAGTCTGCAGGTCAGCCTACTAGTG | 25 | 70 |
| AAGTCTGCTGGTCTGCCTAC | 20 | 71 |
| GTTTTCCACAAACCAGCAGACTT | 23 | 72 |
| TTCAACTTTCTAACATCTGAACTTT | 25 | 73 |
| CTAGTAGGGATGTAGATTAACCTTT | 25 | 74 |
| CTAACATCTGCAAACCAGCAGACTT | 25 | 75 |
| CTTCCACACAACCAACCAGTTAAGT | 25 | 76 |
| CTAGTAGGTCAAACCATGCAGACTT | 25 | 77 |
| GCGTGGTGGCTCAGGCTAGGCACAG | 25 | 78 |
| TAGCTATATAGACATAGATAGCTAT | 25 | 79 |
| ATAGACATAGATTTGGCTCAGGCTA | 25 | 80 |
| ATGTGAGCACCTTCCTTCTTTTTGA | 25 | 81 |
| ATTTAAGGAATGTGA | 15 | 57 |
| TTTTTGATTTTGTCTAAAAC | 20 | 82 |
| AAGGAATGTGATTTTTTGATTTTGT | 25 | 83 |
| AAGGAATGTGATTGATTTTGTCTAA | 25 | 84 |
| CTTTCTAACATCTGAACTTTTTAAA | 25 | 85 |
| CCTTTCAACTTTCTAACATCTGAAC | 25 | 86 |
| ATTAACCTTTCAACTTTCTAACATC | 25 | 87 |
| CTATATATAGATAGTTATTCAACAAA | 25 | 88 |
| TAGATAGCTTTACATTTTACTTATT | 25 | 89 |
| TATGGATGTTAAAAAGCATTTTGTT | 25 | 90 |
| CTATATATAGATAGCTTTATATGGA | 25 | 91 |
| CATTTTACTTATTTTATTCAACAAA | 25 | 92 |
| GCTTTATATGGACATTTTACTTATT | 25 | 93 |
| GATGTTAAAAAGCGTTTCACAAGAC | 25 | 94 |
| TATATGGATGTTATTATTCAACAAA | 25 | 95 |
| GCATTTTGTTTCACAAGTTATTCAA | 25 | 96 |
| CTATATATAGATAGCGACATTTTAC | 25 | 97 |
| AGATAGCTTTATATGGATTTATTCAA | 26 | 98 |
| CTATATATAGTTATTCAACA | 20 | 99 |
| TTTATATGGATGAAGACATTTTAC | 24 | 100 |
| ATTCACTTTCATAATGCTGG | 20 | 2 |
| CACTTTCATAATGCTGG | 17 | 101 |
| TTTCATAATGCTGG | 14 | 28 |
| CTAGTATTTCCTGCAAATGAG | 21 | 102 |
| CCAGCATTTCCTGCAAATGAG | 21 | 103 |
| ATGCCAGCATTTCCTGCAAATGAGA | 25 | 104 |
| GCTCTATGCCAGCATTTCCTGCAAA | 25 | 105 |
| GCTGAGTGATTACTTA | 16 | 106 |
| ATGCTGAGTGATTACT | 16 | 107 |
| AGATGCTGAGTGATTA | 16 | 108 |
| AAAGATGCTGAGTGAT | 16 | 109 |
| GAAAAGATGCTGAGTG | 16 | 110 |
| AGGAAAAGATGCTGAG | 16 | 111 |
| TCAGGAAAAGATGCTG | 16 | 112 |
| TGTCAGGAAAAGATGC | 16 | 113 |
| ATTGTCAGGAAAAGAT | 16 | 114 |
| AAATTGTCAGGAAAAG | 16 | 115 |
| AAAAATTGTCAGGAAA | 16 | 116 |
| AAAAAAATTGTCAGGA | 16 | 117 |
| ACAAAAAAATTGTCAG | 16 | 118 |
| CTACAAAAAAATTGTC | 16 | 119 |
| AACTACAAAAAAATTG | 16 | 120 |
| ATAACTACAAAAAAAT | 16 | 121 |
| CATAACTACAAAAAAA | 16 | 122 |
| CACATAACTACAAAAA | 16 | 123 |
| GTCACATAACTACAAA | 16 | 124 |
| AAGTCACATAACTACA | 16 | 125 |
| CACATAACTACA | 12 | 126 |
| CAAAGTCACATAACTA | 16 | 127 |
| GTCACATAACTA | 12 | 128 |
| AACAAAGTCACATAAC | 16 | 129 |
| AAGTCACATAAC | 12 | 130 |
| AAAACAAAGTCACATA | 16 | 131 |
| CAAAGTCACATA | 12 | 132 |
| ACAAAACAAAGTCACA | 16 | 133 |
| AACAAAGTCACA | 12 | 134 |
| TTACAAAACAAAGTCA | 16 | 135 |
| ATTTACAAAACAAAGT | 16 | 136 |
| AAATTTACAAAACAAA | 16 | 137 |
| ATAAATTTACAAAACA | 16 | 138 |
| TATAAATTTACAAAAC | 16 | 139 |
| GACATTTTACTTATTT | 16 | 140 |
| AAGACATTTTACTTAT | 16 | 141 |
| ACAAGACATTTTACTT | 16 | 142 |
| TCACAAGACATTTTAC | 16 | 143 |
| TTTCACAAGACATTTT | 16 | 144 |
| GTTTCACAAGACATTT | 16 | 145 |
| TGTTTCACAAGACATT | 16 | 146 |
| TTGTTTCACAAGACAT | 16 | 147 |
| TTTTGTTTCACAAGAC | 16 | 148 |
| CATTTTGTTTCACAAG | 16 | 149 |
| AGCATTTTGTTTCACA | 16 | 150 |
| AAAGCATTTTGTTTCA | 16 | 151 |
| TAAAAAGCATTTTGTT | 16 | 152 |
| GTTAAAAAGCATTTTG | 16 | 153 |
| ATGTTAAAAAGCATTT | 16 | 154 |
| TGGATGTTAAAAAGCA | 16 | 155 |
| TATGGATGTTAAAAAG | 16 | 156 |
| TTATATGGATGTTAAA | 16 | 157 |
| GCTTTATATGGATGTT | 16 | 158 |
| TAGCTTTATATGGATG | 16 | 159 |
| GATAGCTTTATATGGA | 16 | 160 |
| TAGATAGCTTTATATG | 16 | 161 |
| TATAGATAGCTTTATA | 16 | 162 |
| TATATAGATAGCTTTA | 16 | 163 |
| TATATATAGATAGCTT | 16 | 164 |
| AAAAACATTTGTTTTC | 16 | 165 |
| TCAAAAACATTTGTTT | 16 | 166 |
| GTTCAAAAACATTTGT | 16 | 167 |
| ATGTTCAAAAACATTT | 16 | 168 |
| AAATGTTCAAAAACAT | 16 | 169 |
| TTAAATGTTCAAAAAC | 16 | 170 |
| TTTTAAATGTTCAAAA | 16 | 171 |
| GTTTTTAAATGTTCAA | 16 | 172 |
| AAGTTTTTAAATGTTC | 16 | 173 |
| TGAAGTTTTTAAATGT | 16 | 174 |
| CTGAAGTTTTTAAATG | 16 | 175 |
| TCTGAAGTTTTTAAAT | 16 | 176 |
| CATCTGAAGTTTTTAA | 16 | 177 |
| AACATCTGAAGTTTTT | 16 | 178 |
| CTAACATCTGAAGTTT | 16 | 179 |
| TTCTAACATCTGAAGT | 16 | 180 |
| CTTTCTAACATCTGAA | 16 | 181 |
| AACTTTCTAACATCTG | 16 | 182 |
| TCAACTTTCTAACATC | 16 | 183 |
| TTTCAACTTTCTAACA | 16 | 184 |
| CTTTCAACTTTCTAAC | 16 | 185 |
| CCTTTCAACTTTCTAA | 16 | 186 |
| TTAACCTTTCAACTTT | 16 | 187 |
| CATTAACCTTTCAACT | 16 | 188 |
| AUUCACUUUCAUAAUGCUGG | 20 | 12 |
| GUAAGAUUCACUUUCAUAAUGCUGG | 25 | 189 |
| GAUAGCUAUAUAUAGAUAGCUUU | 23 | 190 |
| AUAGAUAGCUAUAUAUAGAUAGCUUU | 26 | 191 |
| GAUAGCUAUAUAUAGAUAGC | 20 | 192 |
| AUAGAUAUAGAUAGCUAUAU | 20 | 193 |
| AUUAACCUUUUAUCUAAUAGUUU | 23 | 194 |
| AUUAACCUUUUAUCUAAUAGUUUUGG | 26 | 195 |
| AAUAGUUUUGGCAUCAAAAU | 20 | 196 |
| AAUAGUUUUGGCAUCAAAAUUCU | 23 | 197 |
| AAUAGUUUUGGCAUCAAAAUUCUUUA | 26 | 198 |
| UCUAAUAGUUUUGGCAUCAA | 20 | 199 |
| UCUAAUAGUUUUGGCAUCAAAAU | 23 | 200 |
| GAUCUGUCUGAUCGUUUCUU | 20 | 201 |
| AUCUUCUAUAACGCUUCACAUUCCA | 25 | 202 |
| UCUAUAACGCUUCACAUUCCAGAUC | 25 | 203 |
| CUUCUAUAACGCUUCACAUUCCAGA | 25 | 204 |
| UUUGUUUCACAAGACAUUUU | 20 | 205 |
| ACCUUCCUUCUUUUUGAUUUUGUCU | 25 | 206 |
| CUGGCAGACUUACUCCUUAAUUUAAGGAAU | 30 | 207 |
| UCCUUCUUUUUGAUUUUGUCU | 21 | 208 |
| CACCUUCCUUCUUUUUGAUU | 20 | 209 |
| ACAACUUUGGGAGGCGGAGG | 20 | 210 |
| AAUCCCACAACUUUGGGAGG | 20 | 211 |
| GCUCAUGCCUACAACCCCAC | 20 | 212 |
| GCAGUGGCUCAUGCCUACAA | 20 | 213 |
| CAAUUAUUAGGCUGCAGUUA | 20 | 214 |
| TATCCCAAAGAAAACAATTA | 20 | 215 |
| UUUUAAUGUACUUUAAAAGU | 20 | 216 |
| AUAGUCUUUUAAUGUACUUU | 20 | 217 |
| UAUGAUCAGAAAUUAAGUUG | 20 | 218 |
| UAUUCAACAAAAUAUGAUCA | 20 | 219 |
| UUUUGGCAUCAAAAUUCUUUAAUAU | 25 | 220 |
| AAUAGUUUUGGCAUCAAAAUUCUUU | 25 | 221 |
| UCUAAUAGUUUUGGCAUCAAAAUUCUUU | 28 | 222 |
| CCUUUUAUCUAAUAGUUUUGGCAUCAAAAU | 30 | 223 |
| AUUAACCUUUUAUCUAAUAGUUUUGGCAUC | 30 | 224 |
| AUUAACCUUUUAUCUAAUAGUUUUG | 25 | 225 |
| AUUAACCUUUUAUCUAAUAG | 20 | 226 |
| UAGAUUAACCUUUUAUCUAAUAG | 23 | 227 |
| AUGUAGAUUAACCUUUUAUCUAAUAG | 26 | 228 |
| GAAUUCUAGUAGGGAUGUAG | 20 | 229 |
| AAAAUGGCAUCAUAUCCUAA | 20 | 230 |
| GAUAUAAAAUGGCAUCAUAUCCUAA | 25 | 231 |
| UAUAAAAUGGCAUCAUAUCC | 20 | 232 |
| GAUAUAAAAUGGCAUCAUAU | 20 | 233 |
| AAUAGUUUUGGCUUCAAAAUUCU | 23 | 234 |
| AAUAGUUUUGGCAUCAAAAUUUU | 23 | 235 |
| UGGAGCUUGACACCACCCUG | 20 | 236 |
| ACUUGAGACCUGGAGCUUGA | 20 | 237 |
| UAGGGGGAUCACUUGAGACC | 20 | 238 |
| GAGGCGGAGGUAGGGGGAUC | 20 | 239 |

### Certain NMA Containing Oligomeric Compounds Targeting SMN2 Pre-mRNA

| Chemistry Notation | SEQ ID NO: (modified / unmodified) | |
|---|---|---|
| GₙₛTₙₓ^{m}CₙₓTₙₓGₙₓ^{m}Cₙₓ^{m}CₙₓAₙₓGₙₓ^{m}CₙₓAₙₓTₙₓTₙₓAₙₓTₙₓGₙₓAₙₓAₙₓAₙₛGₙ | 306 | 241 |
| Tₙₛ^{m}CₙₓTₙₓGₙₓ^{m}Cₙₓ^{m}CₙₓAₙₓGₙₓ^{m}CₙₓAₙₓTₙₓTₙₓAₙₓTₙₓGₙₓAₙₓAₙₓAₙₓGₙₛTₙ | 307 | 242 |
| Tₙₛ^{m}CₙₓTₙₓGₙₓ^{m}Cₙₓ^{m}CₙₓAₙₓGₙₓ^{m}CₙₓAₙₓTₙₓTₙₓAₙₓTₙₓGₙₓAₙₓAₙₓAₙₛGₙ | 299 | 243 |
| ^{m}CₙₛTₙₓGₙₓ^{m}Cₙₓ^{m}CₙₓAₙₓGₙₓ^{m}CₙₓAₙₓTₙₓTₙₓAₙₓTₙₓGₙₓAₙₓAₙₓAₙₓGₙₓTₙₛGₙ | 308 | 244 |
| ^{m}CₙₛTₙₓGₙₓ^{m}Cₙₓ^{m}CₙₓAₙₓGₙₓ^{m}CₙₓAₙₓTₙₓTₙₓAₙₓTₙₓGₙₓAₙₓAₙₓAₙₓGₙₓTₙ | 300 | 245 |
| ^{m}CₙₛTₙₓGₙₓ^{m}Cₙₓ^{m}CₙₓAₙₓGₙₓ^{m}CₙₓAₙₓTₙₓTₙₓAₙₓTₙₓGₙₓAₙₓAₙₓAₙₛGₙ | 290 | 246 |
| TₙₛGₙₓ^{m}Cₙₓ^{m}CₙₓAₙₓGₙₓ^{m}CₙₓAₙₓTₙₓTₙₓAₙₓTₙₓGₙₓAₙₓAₙₓAₙₓGₙₓTₙₓGₙₛAₙ | 309 | 247 |
| TₙₛGₙₓ^{m}Cₙₓ^{m}CₙₓAₙₓGₙₓ^{m}CₙₓAₙₓTₙₓTₙₓAₙₓTₙₓGₙₓAₙₓAₙₓAₙₓGₙₓTₙₛGₙ | 301 | 248 |
| TₙₛGₙₓ^{m}Cₙₓ^{m}CₙₓAₙₓGₙₓ^{m}CₙₓAₙₓTₙₓTₙₓAₙₓTₙₓGₙₓAₙₓAₙₓAₙₓGₙₛTₙ | 291 | 249 |
| TₙₛGₙₓ^{m}Cₙₓ^{m}CₙₓAₙₓGₙₓ^{m}CₙₓAₙₓTₙₓTₙₓAₙₓTₙₓGₙₓAₙₓAₙₓAₙₛGₙ | 279 | 250 |
| Gₙₛ^{m}Cₙₓ^{m}CₙₓAₙₓGₙₓ^{m}CₙₓAₙₓTₙₓTₙₓAₙₓTₙₓGₙₓAₙₓAₙₓAₙₓGₙₓTₙₓGₙₓAₙₛAₙ | 310 | 251 |
| Gₙₛ^{m}Cₙₓ^{m}CₙₓAₙₓGₙₓ^{m}CₙₓAₙₓTₙₓTₙₓAₙₓTₙₓGₙₓAₙₓAₙₓAₙₓGₙₓTₙₓGₙₛAₙ | 302 | 252 |
| Gₙₛ^{m}Cₙₓ^{m}CₙₓAₙₓGₙₓ^{m}CₙₓAₙₓTₙₓTₙₓAₙₓTₙₓGₙₓAₙₓAₙₓAₙₓGₙₛTₙₓGₙ | 292 | 253 |
| Gₙₛ^{m}Cₙₓ^{m}CₙₓAₙₓGₙₓ^{m}CₙₓAₙₓTₙₓTₙₓAₙₓTₙₓGₙₓAₙₓAₙₓAₙₓGₙₛTₙ | 280 | 254 |
| Gₙₛ^{m}Cₙₓ^{m}CₙₓAₙₓGₙₓ^{m}CₙₓAnₓTₙₓTₙₓAₙₓTₙₓGₙₓAₙₓAₙₓAₙₛGₙ | 275 | 255 |
| ^{m}Cₙₛ^{m}CₙₓAₙₓGₙₓ^{m}CₙₓAₙₓTₙₓTₙₓAₙₓTₙₓGₙₓAₙₓAₙₓAₙₓGₙₓTₙₓGₙₓAₙₓAₙₛTₙ | 311 | 256 |
| ^{m}Cₙₛ^{m}CₙₓAₙₓGₙₓ^{m}CₙₓAₙₓTₙₓTₙₓAₙₓTₙₓGₙₓAₙₓAₙₓAₙₓGₙₓTₙₓGₙₛAₙ | 293 | 257 |
| ^{m}Cₙₛ^{m}CₙₓAₙₓGₙₓ^{m}CₙₓAₙₓTₙₓTₙₓAₙₓTₙₓGₙₓAₙₓAₙₓAₙₓGₙₓTₙₛGₙ | 281 | 258 |
| ^{m}Cₙₛ^{m}CₙₓAₙₓGₙₓ^{m}CₙₓAₙₓTₙₓTₙₓAₙₓTₙₓGₙₓAₙₓAₙₓAₙₓGₙₛTₙ | 276 | 259 |
| ^{m}Cₙₛ^{m}CₙₓAₙₓGₙₓ^{m}CₙₓAₙₓTₙₓTₙₓAₙₓTₙₓGₙₓAₙₓAₙₓAₙₛGₙ | 266 | 260 |
| ^{m}CₙₛAₙₓGₙₓ^{m}CₙₓAₙₓTₙₓTₙₓAₙₓTₙₓGₙₓAₙₓAₙₓAₙₓGₙₓTₙₓGₙₓAₙₓAₙₓTₙₛ^{m}Cₙ | 312 | 261 |
| ^{m}CₙₛAₙₓGₙₓ^{m}CₙₓAₙₓTₙₓTₙₓAₙₓTₙₓGₙₓAₙₓAₙₓAₙₓGₙₓTₙₓGₙₓAₙₓAₙₛTₙ | 303 | 262 |
| ^{m}CₙₛAₙₓGₙₓ^{m}CₙₓAₙₓTₙₓTₙₓAₙₓTₙₓGₙₓAₙₓAₙₓAₙₓGₙₓTₙₓGₙₛAₙ | 282 | 263 |
| ^{m}CₙₛAₙₓGₙₓ^{m}CₙₓAₙₓTₙₓTₙₓAₙₓTₙₓGₙₓAₙₓAₙₓAₙₓGₙₓTₙₛGₙ | 277 | 264 |
| ^{m}CₙₛAₙₓGₙₓ^{m}CₙₓAₙₓTₙₓTₙₓAₙₓTₙₓGₙₓAₙₓAₙₓAₙₓGₙₛTₙ | 267 | 265 |

Subscripts in the table above: "x" represents an internucleoside linkage selected from among a phosphorothioate internucleoside linkage or a phosphate internucleoside linkage, "n" represents a 2'-O-(N-methylacetamide) modified nucleoside. Superscripts: "m" before a C represents a 5-methylcysteine.

In certain embodiments, the present disclosure provides modified oligonucleotides comprising a sequence selected from among any of SEQ ID Nos: 1, 2, 3, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 241, 242, 243, 244, 245, 246, 247, 248, 249, 250, 251, 252, 253, 254, 255, 256, 257, 258, 259, 260, 261, 262, 263, 264, or 265.

In certain embodiments, the present disclosure provides modified oligonucleotides consisting of a sequence selected from among any of SEQ ID Nos: 1, 2, 3, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 241, 242, 243, 244, 245, 246, 247, 248, 249, 250, 251, 252, 253, 254, 255, 256, 257, 258, 259, 260, 261, 262, 263, 264, or 265.

In certain embodiments, the present disclosure provides modified oligonucleotides comprising a sequence selected from among any of SEQ ID Nos: 1, 2, 3, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 241, 242, 243, 244, 245, 246, 247, 248, 249, 250, 251, 252, 253, 254, 255, 256, 257, 258, 259, 260, 261, 262, 263, 264, or 265; and wherein at least one, at least two, at least three, at least four, at least five, or at least six nucleosides of the modified oligonucleotide comprises a 2'-O-(N-methyl acetamide) modified sugar moiety.

In certain embodiments, the present disclosure provides modified oligonucleotides consisting of a sequence selected from among any of SEQ ID Nos: 1, 2, 3, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 241, 242, 243, 244, 245, 246, 247, 248, 249, 250, 251, 252, 253, 254, 255, 256, 257, 258, 259, 260, 261, 262, 263, 264, or 265; and wherein at least one, at least two, at least three, at least four, at least five, or at least six nucleosides of the modified oligonucleotide comprises a 2'-O-(N-methyl acetamide) modified sugar moiety.

### Certain Combination Administrations

In certain embodiments, a first agent comprising the compound described herein is co-administered with one or more secondary agents. In certain embodiments, such second agents are designed to treat the same disease, disorder, or condition as the first agent described herein. In certain embodiments, second agents are co-administered with the first agent to produce a combinational effect. In certain embodiments, second agents are co-administered with the first agent to produce a synergistic effect. In certain embodiments, the co-administration of the first and second agents permits use of lower dosages than would be required to achieve a therapeutic or prophylactic effect if the agents were administered as independent therapy.

In certain embodiments, the present disclosure provides administration of a first antisense compound into the CSF, in combination with systemic delivery of a second antisense compound. Systemic administration and CSF administration can occur simultaneously, separately or sequentially. In certain embodiments, a subject receives a first dose of an antisense compound in the CSF and subsequently receives a second dose of an antisense compound systemically. In certain embodiments, the antisense compound administered into the CSF is Isis 396443. In certain embodiments, the antisense compound administered into the CSF is a modified oligonucleotide having one or more 2'-O-(N-alkyl acetamide) modified sugar moieties. In certain embodiments, the antisense compound administered into the CSF is an unconjugated modified oligonucleotide having one or more 2'-O-(N-alkyl acetamide) modified sugar moieties.

In certain embodiments, the antisense compound administered systemically is a modified oligonucleotide having one or more 2'-O-(N-alkyl acetamide) modified sugar moieties. In certain embodiments, the antisense compound administered systemically is a modified oligonucleotide having one or more 2'-O-(N-methyl acetamide) modified sugar moieties. In certain embodiments, the antisense compound administered systemically is a modified oligonucleotide having one or more 2'-O-(N-alkyl acetamide) modified sugar moieties and a conjugate group. In certain embodiments, the antisense compound administered systemically is a modified oligonucleotide having one or more 2'-O-(N-methyl acetamide) modified sugar moieties and a conjugate group.

Administration can occur in any order and with varying frequency. For example, a subject may receive a systemic dose of a modified oligonucleotide having one or more 2'-O-(N-alkyl acetamide) or 2'-O-(N-methyl acetamide) modified sugar moieties, and then subsequently receives a dose of an antisense compound (e.g. ISIS 396443) into the CSF. Alternatively, a subject may receive a systemic dose of a modified oligonucleotide having one or more 2'-O-(N-alkyl acetamide) or 2'-O-(N-methyl acetamide) modified sugar moieties subsequent to receiving a dose of an antisense compound (e.g. ISIS 396443) into the CSF. Alternatively, a subject may simultaneously receive a CSF dose of an antisense compound (e.g. ISIS 396443) and a systemic dose of a modified oligonucleotide having one or more 2'-O-(N-alkyl acetamide) or 2'-O-(N-methyl acetamide) modified sugar moieties. Administration into the CSF and systemic administration can occur at different time points and with different frequencies. For example, a subject may receive systemic administration of a modified oligonucleotide having one or more 2'-O-(N-alkyl acetamide) or 2'-O-(N-methyl acetamide) modified sugar moieties on a weekly or monthly basis, and receives CSF administration of an antisense compound (e.g. ISIS 396443) into the CSF once every four months or once every six months. In certain embodiments in which a CSF dose of an antisense compound (e.g. ISIS 396443) and a systemic dose of a modified oligonucleotide having one or more 2'-O-(N-alkyl acetamide) or 2'-O-(N-methyl acetamide) modified sugar moieties are administered separately, serially, or sequentially, administration of such doses can be spaced apart by various durations such as daily, weekly, or monthly.

### I. Certain Oligonucleotides

In certain embodiments, the invention provides oligonucleotides, which consist of linked nucleosides. Oligonucleotides may be unmodified oligonucleotides (unmodified RNA or DNA) or may be modified oligonucleotides. Modified oligonucleotides comprise at least one modification relative to unmodified RNA or DNA (i.e., comprise at least one modified nucleoside (comprising a modified sugar moiety and/or a modified nucleobase) and/or at least one modified internucleoside linkage).

### A. Certain Modified Nucleosides

Modified nucleosides comprise a modified sugar moiety or a modified nucleobase or both a modifed sugar moiety and a modified nucleobase.

### 1. Certain Sugar Moieties

In certain embodiments, modified sugar moieties are non-bicyclic modified sugar moieties. In certain embodiments, modified sugar moieties are bicyclic or tricyclic sugar moieties. In certain embodiments, modified sugar moieties are sugar surrogates. Such sugar surrogates may comprise one or more substitutions corresponding to those of other types of modified sugar moieties.

In certain embodiments, modified sugar moieties are non-bicyclic modified sugar moieties comprising a furanosyl ring with one or more acyclic substituent, including but not limited to substituents at the 2', 4', and/or 5' positions. In certain embodiments one or more acyclic substituent of non-bicyclic modified sugar moieties is branched. Examples of 2'-substituent groups suitable for non-bicyclic modified sugar moieties include but are not limited to: 2'-O-(N-alkyl acetamide), *e.g*., 2'-O-(N-methyl acetamide). For example, see U.S. 6,147,200 and Prakash et al., Org. Lett., 5, 403-6 (2003). A "2'-O-(N-methyl acetamide)" or "2'-NMA" modified nucleoside is shown below:

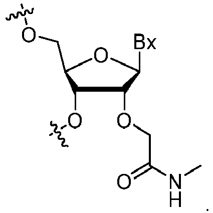

In certain embodiments, 2'-substituent groups are selected from among: 2'-F, 2'-OCH₃ ("OMe" or "O-methyl"), 2'-O(CH₂)₂OCH₃ ("MOE"), halo, allyl, amino, azido, SH, CN, OCN, CF₃, OCF₃, O-C₁-C₁₀ alkoxy, O-C₁-C₁₀ substituted alkoxy, O-C₁-C₁₀ alkyl, O-C₁-C₁₀ substituted alkyl, S-alkyl, N(Rₘ)-alkyl, O-alkenyl, S-alkenyl, N(Rₘ)-alkenyl, O-alkynyl, S-alkynyl, N(Rₘ)-alkynyl, O-alkylenyl-O-alkyl, alkynyl, alkaryl, aralkyl, O-alkaryl, O-aralkyl, O(CH₂)₂SCH₃, O(CH₂)₂ON(Rₘ)(Rₙ) or OCH₂C(=O)-N(Rₘ)(Rₙ), where each Rₘ and Rₙ is, independently, H, an amino protecting group, or substituted or unsubstituted C₁-C₁₀ alkyl, and the 2'-substituent groups described in Cook et al., U.S. 6,531,584; Cook et al., U.S. 5,859,221; and Cook et al., U.S. 6,005,087. Certain embodiments of these 2'-substituent groups can be further substituted with one or more substituent groups independently selected from among: hydroxyl, amino, alkoxy, carboxy, benzyl, phenyl, nitro (NO₂), thiol, thioalkoxy, thioalkyl, halogen, alkyl, aryl, alkenyl and alkynyl. Examples of 4'-substituent groups suitable for non-bicyclic modified sugar moieties include but are not limited to alkoxy (*e.g*., methoxy), alkyl, and those described in Manoharan et al., WO 2015/106128. Examples of 5'-substituent groups suitable for non-bicyclic modified sugar moieties include but are not limited to: 5'-methyl (R or S), 5'-vinyl, and 5'-methoxy. In certain embodiments, non-bicyclic modified sugars comprise more than one non-bridging sugar substituent, for example, 2'-F-5'-methyl sugar moieties and the modified sugar moieties and modified nucleosides described in Migawa et al., WO 2008/101157 and Rajeev et al., US2013/0203836.).

In certain embodiments, a 2'-substituted nucleoside or 2'- non-bicyclic modified nucleoside comprises a sugar moiety comprising a non-bridging 2'-substituent group selected from: F, NH₂, N₃, OCF₃, OCH₃, O(CH₂)₃NH₂, CH₂CH=CH₂, OCH₂CH=CH₂, OCH₂CH₂OCH₃, O(CH₂)₂SCH₃, O(CH₂)₂ON(Rₘ)(Rₙ), O(CH₂)₂O(CH₂)₂N(CH₃)₂, and N-substituted acetamide (OCH₂C(=O)-N(Rₘ)(Rₙ)), where each Rₘ and Rₙ is, independently, H, an amino protecting group, or substituted or unsubstituted C₁-C₁₀ alkyl. In certain embodiments, each Rₘ and Rₙ is, independently, H or C₁-C₃ alkyl. In certain embodiments, each Rₘ and Rₙ is, independently, H or methyl.

In certain embodiments, a 2'-substituted nucleoside or 2'- non-bicyclic modified nucleoside comprises a sugar moiety comprising a non-bridging 2'-substituent group selected from: F, OCF₃, OCH₃, OCH₂CH₂OCH₃, O(CH₂)₂SCH₃, O(CH₂)₂ON(CH₃)₂, O(CH₂)₂O(CH₂)₂N(CH₃)₂, and OCH₂C(=O)-N(H)CH₃.

In certain embodiments, a 2'-substituted nucleoside or 2'- non-bicyclic modified nucleoside comprises a sugar moiety comprising a non-bridging 2'-substituent group selected from: F, OCH₃, OCH₂CH₂OCH₃, and OCH₂C(=O)-N(H)CH₃.

Nucleosides comprising modified sugar moieties, such as non-bicyclic modified sugar moieties, may be referred to by the position(s) of the substitution(s) on the sugar moiety of the nucleoside. For example, nucleosides comprising 2'-substituted or 2-modified sugar moieties are referred to as 2'-substituted nucleosides or 2-modified nucleosides.

Certain modifed sugar moieties comprise a bridging sugar substituent that forms a second ring resulting in a bicyclic sugar moiety. In certain such embodiments, the bicyclic sugar moiety comprises a bridge between the 4' and the 2' furanose ring atoms. Examples of such 4' to 2' bridging sugar substituents include but are not limited to: 4'-CH₂-2', 4'-(CH₂)₂-2', 4'-(CH₂)₃-2', 4'-CH₂-O-2' ("LNA"), 4'-CH₂-S-2', 4'-(CH₂)₂-O-2' ("ENA"), 4'-CH(CH₃)-O-2' (referred to as "constrained ethyl" or "cEt" when in the S configuration), 4'-CH₂-O-CH₂-2', 4'-CH₂-N(R)-2', 4'-CH(CH₂OCH₃)-O-2' ("constrained MOE" or "cMOE") and analogs thereof (*see, e.g.,* Seth et al., U.S. 7,399,845, Bhat et al., U.S. 7,569,686, Swayze et al., U.S. 7,741,457, and Swayze et al., U.S. 8,022,193), 4'-C(CH₃)(CH₃)-O-2' and analogs thereof (*see, e.g.,* Seth et al., U.S. 8,278,283), 4'-CH₂-N(OCH₃)-2' and analogs thereof (*see, e.g.,* Prakash et al., U.S. 8,278,425), 4'-CH₂-O-N(CH₃)-2' (*see, e.g.,* Allerson et al., U.S. 7,696,345 and Allerson et al., U.S. 8,124,745), 4'-CH₂-C(H)(CH₃)-2' (*see, e.g.,* Zhou, et al., J. Org. Chem., 2009, 74, 118-134), 4'-CH₂-C(=CH₂)-2' and analogs thereof (*see e.g.,* Seth et al., U.S. 8,278,426), 4'-C(RₐR_{b})-N(R)-O-2', 4'-C(RₐR_{b})-O-N(R)-2', 4'-CH₂-O-N(R)-2', and 4'-CH₂-N(R)-O-2', wherein each R, Rₐ, and R_{b} is, independently, H, a protecting group, or C₁-C₁₂ alkyl (*see, e.g.* Imanishi et al., U.S. 7,427,672).

In certain embodiments, such 4' to 2' bridges independently comprise from 1 to 4 linked groups independently selected from: -[C(Rₐ)(R_{b})]ₙ-, -[C(Rₐ)(R_{b})]ₙ-O-, -C(Rₐ)=C(R_{b})-, -C(Ra)=N-, -C(=NRa)-, - C(=O)-, -C(=S)-, -O-, -Si(Rₐ)₂-, -S(=O)ₓ-, and -N(Rₐ)-;
wherein:
x is 0, 1, or 2;
n is 1, 2, 3, or 4;
each Rₐ and R_{b} is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-Ji); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl, or a protecting group.

Additional bicyclic sugar moieties are known in the art, see, for example: Freier et al., Nucleic Acids Research, 1997, 25(22), 4429-4443, Albaek et al., J. Org. Chem., 2006, 71, 7731-7740, Singh et al., Chem. Commun., 1998, 4, 455-456; Koshkin et al., Tetrahedron, 1998, 54, 3607-3630; Kumar et al., Bioorg. Med. Chem. Lett., 1998, 8, 2219-2222; Singh et al., J. Org. Chem., 1998, 63, 10035-10039; Srivastava et al., J. Am. Chem. Soc., 20017, 129, 8362-8379; Wengel et a., U.S. 7,053,207; Imanishi et al., U.S. 6,268,490; Imanishi et al. U.S. 6,770,748; Imanishi et al., U.S. RE44,779; Wengel et al., U.S. 6,794,499; Wengel et al., U.S. 6,670,461; Wengel et al., U.S. 7,034,133; Wengel et al., U.S. 8,080,644; Wengel et al., U.S. 8,034,909; Wengel et al., U.S. 8,153,365; Wengel et al., U.S. 7,572,582; and Ramasamy et al., U.S. 6,525,191;; Torsten et al., WO 2004/106356;Wengel et al., WO 1999/014226; Seth et al., WO 2007/134181; Seth et al., U.S. 7,547,684; Seth et al., U.S. 7,666,854; Seth et al., U.S. 8,088,746; Seth et al., U.S. 7,750,131; Seth et al., U.S. 8,030,467; Seth et al., U.S. 8,268,980; Seth et al., U.S. 8,546,556; Seth et al., U.S. 8,530,640; Migawa et al., U.S. 9,012,421; Seth et al., U.S. 8,501,805; and U.S. Patent Publication Nos. Allerson et al., US2008/0039618 and Migawa et al., US2015/0191727..

In certain embodiments, bicyclic sugar moieties and nucleosides incorporating such bicyclic sugar moieties are further defined by isomeric configuration. For example, an LNA nucleoside (described herein) may be in the α-L configuration or in the β-D configuration. 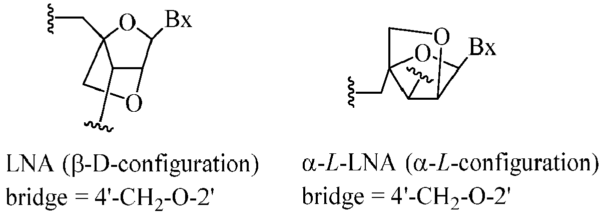 α-L-methyleneoxy (4'-CH₂-O-2') or α-L-LNA bicyclic nucleosides have been incorporated into antisense oligonucleotides that showed antisense activity (Frieden et al., Nucleic Acids Research, 2003, 21, 6365-6372). Herein, general descriptions of bicyclic nucleosides include both isomeric configurations. When the positions of specific bicyclic nucleosides (*e.g*., LNA or cEt) are identified in exemplified embodiments herein, they are in the β-D configuration, unless otherwise specified.

In certain embodiments, modified sugar moieties comprise one or more non-bridging sugar substituent and one or more bridging sugar substituent (e.g., 5'-substituted and 4'-2' bridged sugars).

In certain embodiments, modified sugar moieties are sugar surrogates. In certain such embodiments, the oxygen atom of the sugar moiety is replaced, *e.g*., with a sulfur, carbon or nitrogen atom. In certain such embodiments, such modified sugar moieties also comprise bridging and/or non-bridging substituents as described herein. For example, certain sugar surrogates comprise a 4'-sulfur atom and a substitution at the 2'-position (*see, e.g.,* Bhat et al., U.S. 7,875,733 and Bhat et al., U.S. 7,939,677) and/or the 5' position.

In certain embodiments, sugar surrogates comprise rings having other than 5 atoms. For example, in certain embodiments, a sugar surrogate comprises a six-membered tetrahydropyran ("THP"). Such tetrahydropyrans may be further modified or substituted. Nucleosides comprising such modified tetrahydropyrans include but are not limited to hexitol nucleic acid ("HNA"), anitol nucleic acid ("ANA"), manitol nucleic acid ("MNA") (*see, e.g.,* Leumann, CJ. Bioorg. & Med. Chem. 2002, 10, 841-854), fluoro HNA: 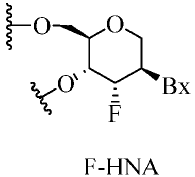 ("F-HNA", *see e.g*. Swayze et al., U.S. 8,088,904; Swayze et al., U.S. 8,440,803; Swayze et al., U.S. 8,796,437; and Swayze et al., U.S. 9,005,906; F-HNA can also be referred to as a F-THP or 3'-fluoro tetrahydropyran), and nucleosides comprising additional modified THP compounds having the formula: 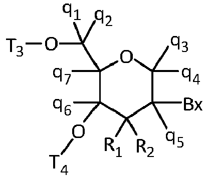 wherein, independently, for each of said modified THP nucleoside:
Bx is a nucleobase moiety;
T₃ and T₄ are each, independently, an internucleoside linking group linking the modified THP nucleoside to the remainder of an oligonucleotide or one of T₃ and T₄ is an internucleoside linking group linking the modified THP nucleoside to the remainder of an oligonucleotide and the other of T₃ and T₄ is H, a hydroxyl protecting group, a linked conjugate group, or a 5' or 3'-terminal group;
   q₁, q₂, q₃, q₄, q₅, q₆ and q₇ are each, independently, H, C₁-C₆ alkyl, substituted C₁-C₆ alkyl, C₂-C₆ alkenyl, substituted C₂-C₆ alkenyl, C₂-C₆ alkynyl, or substituted C₂-C₆ alkynyl; and
each of R₁ and R₂ is independently selected from among: hydrogen, halogen, substituted or unsubstituted alkoxy, NJ₁J₂, SJ₁, N₃, OC(=X)J₁, OC(=X)NJ₁J₂, NJ₃C(=X)NJ₁J₂, and CN, wherein X is O, S or NJ₁, and each J₁, J₂, and J₃ is, independently, H or C₁-C₆ alkyl.

In certain embodiments, modified THP nucleosides are provided wherein q₁, q₂, q₃, q₄, q₅, q₆ and q₇ are each H. In certain embodiments, at least one of q₁, q₂, q₃, q₄, q₅, q₆ and q₇ is other than H. In certain embodiments, at least one of q₁, q₂, q₃, q₄, q₅, q₆ and q₇ is methyl. In certain embodiments, modified THP nucleosides are provided wherein one of R₁ and R₂ is F. In certain embodiments, R₁ is F and R₂ is H, in certain embodiments, R₁ is methoxy and R₂ is H, and in certain embodiments, R₁ is methoxyethoxy and R₂ is H.

In certain embodiments, sugar surrogates comprise rings having more than 5 atoms and more than one heteroatom. For example, nucleosides comprising morpholino sugar moieties and their use in oligonucleotides have been reported (*see, e.g.,* Braasch et al., Biochemistry, 2002, 41, 4503-4510 and Summerton et al., U.S. 5,698,685; Summerton et al., U.S. 5,166,315; Summerton et al., U.S. 5,185,444; and Summerton et al., U.S. 5,034,506). As used here, the term "morpholino" means a sugar surrogate having the following structure:

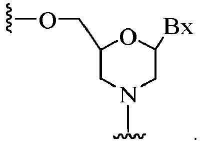

In certain embodiments, morpholinos may be modified, for example by adding or altering various substituent groups from the above morpholino structure. Such sugar surrogates are refered to herein as "modifed morpholines."

In certain embodiments, sugar surrogates comprise acyclic moieites. Examples of nucleosides and oligonucleotides comprising such acyclic sugar surrogates include but are not limited to: peptide nucleic acid ("PNA"), acyclic butyl nucleic acid (*see, e.g.,* Kumar et al., Org. Biomol. Chem., 2013, 11, 5853-5865), and nucleosides and oligonucleotides described in Manoharan et al., WO2011/133876.

Many other bicyclic and tricyclic sugar and sugar surrogate ring systems are known in the art that can be used in modified nucleosides).

### 2. Certain Modified Nucleobases

In certain embodiments, modified oligonucleotides comprise one or more nucleoside comprising an unmodified nucleobase. In certain embodiments, modified oligonucleotides comprise one or more nucleoside comprising a modified nucleobase.

In certain embodiments, modified nucleobases are selected from: 5-substituted pyrimidines, 6-azapyrimidines, alkyl or alkynyl substituted pyrimidines, alkyl substituted purines, and N-2, N-6 and O-6 substituted purines. In certain embodiments, modified nucleobases are selected from: 2-aminopropyladenine, 5-hydroxymethyl cytosine, xanthine, hypoxanthine, 2-aminoadenine, 6-N-methylguanine, 6-N-methyladenine, 2-propyladenine , 2-thiouracil, 2-thiothymine and 2-thiocytosine, 5-propynyl (-C≡C-CH₃) uracil, 5-propynylcytosine, 6-azouracil, 6-azocytosine, 6-azothymine, 5-ribosyluracil (pseudouracil), 4-thiouracil, 8-halo, 8-amino, 8-thiol, 8-thioalkyl, 8-hydroxyl, 8-aza and other 8-substituted purines, 5-halo, particularly 5-bromo, 5-trifluoromethyl, 5-halouracil, and 5-halocytosine, 7-methylguanine, 7-methyladenine, 2-F-adenine, 2-aminoadenine, 7-deazaguanine, 7-deazaadenine, 3-deazaguanine, 3-deazaadenine, 6-N-benzoyladenine, 2-N-isobutyrylguanine, 4-N-benzoylcytosine, 4-N-benzoyluracil, 5-methyl 4-N-benzoylcytosine, 5-methyl 4-N-benzoyluracil, universal bases, hydrophobic bases, promiscuous bases, size-expanded bases, and fluorinated bases. Further modified nucleobases include tricyclic pyrimidines, such as 1,3-diazaphenoxazine-2-one, 1,3-diazaphenothiazine-2-one and 9-(2-aminoethoxy)-1,3-diazaphenoxazine-2-one (G-clamp). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in Merigan et al., U.S. 3,687,808, those disclosed in The Concise Encyclopedia Of Polymer Science And Engineering, Kroschwitz, J.I., Ed., John Wiley & Sons, 1990, 858-859; Englisch et al., Angewandte Chemie, International Edition, 1991, 30, 613; Sanghvi, Y.S., Chapter 15, Antisense Research and Applications, Crooke, S.T. and Lebleu, B., Eds., CRC Press, 1993, 273-288; and those disclosed in Chapters 6 and 15, Antisense Drug Technology, Crooke S.T., Ed., CRC Press, 2008, 163-166 and 442-443.

Publications that teach the preparation of certain of the above noted modified nucleobases as well as other modified nucleobases include without limitation, Manoharan et al., US2003/0158403; Manoharan et al., US2003/0175906; Dinh et al., U.S. 4,845,205; Spielvogel et al., U.S. 5,130,302; Rogers et al., U.S. 5,134,066; Bischofberger et al., U.S. 5,175,273; Urdea et al., U.S. 5,367,066; Benner et al., U.S. 5,432,272; Matteucci et al., U.S. 5,434,257; Gmeiner et al., U.S. 5,457,187; Cook et al., U.S. 5,459,255; Froehler et al., U.S. 5,484,908; Matteucci et al., U.S. 5,502,177; Hawkins et al., U.S. 5,525,711; Haralambidis et al., U.S. 5,552,540; Cook et al., U.S. 5,587,469; Froehler et al., U.S. 5,594,121; Switzer et al., U.S. 5,596,091; Cook et al., U.S. 5,614,617; Froehler et al., U.S. 5,645,985; Cook et al., U.S. 5,681,941; Cook et al., U.S. 5,811,534; Cook et al., U.S. 5,750,692; Cook et al., U.S. 5,948,903; Cook et al., U.S. 5,587,470; Cook et al., U.S. 5,457,191; Matteucci et al., U.S. 5,763,588; Froehler et al., U.S. 5,830,653; Cook et al., U.S. 5,808,027; Cook et al., 6,166,199; and Matteucci et al., U.S. 6,005,096.

### B. Certain Modified Internucleoside Linkages

In certain embodiments, nucleosides of modified oligonucleotides may be linked together using any internucleoside linkage. The two main classes of internucleoside linking groups are defined by the presence or absence of a phosphorus atom. Representative phosphorus-containing internucleoside linkages include but are not limited to phosphates, which contain a phosphodiester bond ("P=O") (also referred to as unmodified or naturally occurring linkages), phosphotriesters, methylphosphonates, phosphoramidates, and phosphorothioates ("P=S"), and phosphorodithioates ("HS-P=S"). Representative non-phosphorus containing internucleoside linking groups include but are not limited to methylenemethylimino (-CH₂-N(CH₃)-O-CH₂-), thiodiester, thionocarbamate (-O-C(=O)(NH)-S-); siloxane (-O-SiH₂-O-); and N,N'-dimethylhydrazine (-CH₂-N(CH₃)-N(CH₃)-). Modified internucleoside linkages, compared to naturally occurring phosphate linkages, can be used to alter, typically increase, nuclease resistance of the oligonucleotide. In certain embodiments, internucleoside linkages having a chiral atom can be prepared as a racemic mixture, or as separate enantiomers. Representative chiral internucleoside linkages include but are not limited to alkylphosphonates and phosphorothioates. Methods of preparation of phosphorous-containing and non-phosphorous-containing internucleoside linkages are well known to those skilled in the art.

Neutral internucleoside linkages include, without limitation, phosphotriesters, methylphosphonates, MMI (3'-CH₂-N(CH₃)-O-5'), amide-3 (3'-CH₂-C(=O)-N(H)-5'), amide-4 (3'-CH₂-N(H)-C(=O)-5'), formacetal (3'-O-CH₂-O-5'), methoxypropyl, and thioformacetal (3'-S-CH₂-O-5'). Further neutral internucleoside linkages include nonionic linkages comprising siloxane (dialkylsiloxane), carboxylate ester, carboxamide, sulfide, sulfonate ester and amides (See for example: Carbohydrate Modifications in Antisense Research; Y.S. Sanghvi and P.D. Cook, Eds., ACS Symposium Series 580; Chapters 3 and 4, 40-65). Further neutral internucleoside linkages include nonionic linkages comprising mixed N, O, S and CH₂ component parts.

### C. Certain Motifs

In certain embodiments, modified oligonucleotides comprise one or more modified nucleoside comprising a modified sugar. In certain embodiments, modified oligonucleotides comprise one or more modified nucleosides comprising a modified nucleobase. In certain embodiments, modified oligonucleotides comprise one or more modified internucleoside linkage. In such embodiments, the modified, unmodified, and differently modified sugar moieties, nucleobases, and/or internucleoside linkages of a modified oligonucleotide define a pattern or motif. In certain embodiments, the patterns of sugar moieties, nucleobases, and internucleoside linkages are each independent of one another. Thus, a modified oligonucleotide may be described by its sugar motif, nucleobase motif and/or internucleoside linkage motif (as used herein, nucleobase motif describes the modifications to the nucleobases independent of the sequence of nucleobases).

### 1. Certain Sugar Motifs

In certain embodiments, oligonucleotides comprise one or more type of modified sugar and/or unmodified sugar moiety arranged along the oligonucleotide or region thereof in a defined pattern or sugar motif. In certain instances, such sugar motifs include but are not limited to any of the sugar modifications discussed herein.

In certain embodiments, modified oligonucleotides comprise or consist of a region having a gapmer motif, which comprises two external regions or "wings" and a central or internal region or "gap." The three regions of a gapmer motif (the 5'-wing, the gap, and the 3'-wing) form a contiguous sequence of nucleosides wherein at least some of the sugar moieties of the nucleosides of each of the wings differ from at least some of the sugar moieties of the nucleosides of the gap. Specifically, at least the sugar moieties of the nucleosides of each wing that are closest to the gap (the 3'-most nucleoside of the 5'-wing and the 5'-most nucleoside of the 3'-wing) are modified sugar moieties and differ from the sugar moieties of the neighboring gap nucleosides, which are unmodified sugar moieties, thus defining the boundary between the wings and the gap (i.e., the wing/gap junction). In certain embodiments, the sugar moieties within the gap are the same as one another. In certain embodiments, the gap includes one or more nucleoside having a sugar moiety that differs from the sugar moiety of one or more other nucleosides of the gap. In certain embodiments, the sugar motifs of the two wings are the same as one another (symmetric gapmer). In certain embodiments, the sugar motif of the 5'-wing differs from the sugar motif of the 3'-wing (asymmetric gapmer).

In certain embodiments, the wings of a gapmer comprise 1-5 nucleosides. In certain embodiments, the wings of a gapmer comprise 2-5 nucleosides. In certain embodiments, the wings of a gapmer comprise 3-5 nucleosides. In certain embodiments, the nucleosides of a gapmer are all modified nucleosides.

In certain embodiments, the gap of a gapmer comprises 7-12 nucleosides. In certain embodiments, the gap of a gapmer comprises 7-10 nucleosides. In certain embodiments, the gap of a gapmer comprises 8-10 nucleosides. In certain embodiments, the gap of a gapmer comprises 10 nucleosides. In certain embodiment, each nucleoside of the gap of a gapmer is an unmodified 2'-deoxy nucleoside.

In certain embodiments, the gapmer is a deoxy gapmer. In such embodiments, the nucleosides on the gap side of each wing/gap junction are unmodified 2'-deoxy nucleosides and the nucleosides on the wing sides of each wing/gap junction are modified nucleosides. In certain such embodiments, each nucleoside of the gap is an unmodified 2'-deoxy nucleoside. In certain such embodiments, each nucleoside of each wing is a modified nucleoside.

In certain embodiments, modified oligonucleotides comprise or consist of a region having a fully modified sugar motif. In such embodiments, each nucleoside of the fully modified region of the modified oligonucleotide comprises a modified sugar moiety. In certain such embodiments, each nucleoside in the entire modified oligonucleotide comprises a modified sugar moiety. In certain embodiments, modified oligonucleotides comprise or consist of a region having a fully modified sugar motif, wherein each nucleoside within the fully modified region comprises the same modified sugar moiety, referred to herein as a uniformly modified sugar motif. In certain embodiments, a fully modified oligonucleotide is a uniformly modified oligonucleotide. In certain embodiments, each nucleoside of a uniformly modified oligonucleotide comprises the same 2'-modification. In certain embodiments, each nucleoside of a uniformly modified oligonucleotide comprises a 2'-O-(N-alkyl acetamide) group. In certain embodiments, each nucleoside of a uniformly modified oligonucleotide comprises a 2'-O-(N-methyl acetamide) group.

### 2. Certain Nucleobase Motifs

In certain embodiments, oligonucleotides comprise modified and/or unmodified nucleobases arranged along the oligonucleotide or region thereof in a defined pattern or motif. In certain embodiments, each nucleobase is modified. In certain embodiments, none of the nucleobases are modified. In certain embodiments, each purine or each pyrimidine is modified. In certain embodiments, each adenine is modified. In certain embodiments, each guanine is modified. In certain embodiments, each thymine is modified. In certain embodiments, each uracil is modified. In certain embodiments, each cytosine is modified. In certain embodiments, some or all of the cytosine nucleobases in a modified oligonucleotide are 5-methylcytosines.

In certain embodiments, modified oligonucleotides comprise a block of modified nucleobases. In certain such embodiments, the block is at the 3'-end of the oligonucleotide. In certain embodiments the block is within 3 nucleosides of the 3'-end of the oligonucleotide. In certain embodiments, the block is at the 5'-end of the oligonucleotide. In certain embodiments the block is within 3 nucleosides of the 5'-end of the oligonucleotide.

In certain embodiments, oligonucleotides having a gapmer motif comprise a nucleoside comprising a modified nucleobase. In certain such embodiments, one nucleoside comprising a modified nucleobase is in the central gap of an oligonucleotide having a gapmer motif. In certain such embodiments, the sugar moiety of said nucleoside is a 2'-deoxyribosyl moiety. In certain embodiments, the modified nucleobase is selected from: a 2-thiopyrimidine and a 5-propynepyrimidine.

### 3. Certain Internucleoside Linkage Motifs

In certain embodiments, oligonucleotides comprise modified and/or unmodified internucleoside linkages arranged along the oligonucleotide or region thereof in a defined pattern or motif. In certain embodiments, essentially each internucleoside linking group is a phosphate internucleoside linkage (P=O). In certain embodiments, each internucleoside linking group of a modified oligonucleotide is a phosphorothioate (P=S). In certain embodiments, each internucleoside linking group of a modified oligonucleotide is independently selected from a phosphorothioate and phosphate internucleoside linkage. In certain embodiments, the sugar motif of a modified oligonucleotide is a gapmer and the internucleoside linkages within the gap are all modified. In certain such embodiments, some or all of the internucleoside linkages in the wings are unmodified phosphate linkages. In certain embodiments, the terminal internucleoside linkages are modified.

### D. Certain Lengths

In certain embodiments, oligonucleotides (including modified oligonucleotides) can have any of a variety of ranges of lengths. In certain embodiments, oligonucleotides consist of X to Y linked nucleosides, where X represents the fewest number of nucleosides in the range and Y represents the largest number nucleosides in the range. In certain such embodiments, X and Y are each independently selected from 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50; provided that X≤Y. For example, in certain embodiments, oligonucleotides consist of 12 to 13, 12 to 14, 12 to 15, 12 to 16, 12 to 17, 12 to 18, 12 to 19, 12 to 20, 12 to 21, 12 to 22, 12 to 23, 12 to 24, 12 to 25, 12 to 26, 12 to 27, 12 to 28, 12 to 29, 12 to 30, 13 to 14, 13 to 15, 13 to 16, 13 to 17, 13 to 18, 13 to 19, 13 to 20, 13 to 21, 13 to 22, 13 to 23, 13 to 24, 13 to 25, 13 to 26, 13 to 27, 13 to 28, 13 to 29, 13 to 30, 14 to 15, 14 to 16, 14 to 17, 14 to 18, 14 to 19, 14 to 20, 14 to 21, 14 to 22, 14 to 23, 14 to 24, 14 to 25, 14 to 26, 14 to 27, 14 to 28, 14 to 29, 14 to 30, 15 to 16, 15 to 17, 15 to 18, 15 to 19, 15 to 20, 15 to 21, 15 to 22, 15 to 23, 15 to 24, 15 to 25, 15 to 26, 15 to 27, 15 to 28, 15 to 29, 15 to 30, 16 to 17, 16 to 18, 16 to 19, 16 to 20, 16 to 21, 16 to 22, 16 to 23, 16 to 24, 16 to 25, 16 to 26, 16 to 27, 16 to 28, 16 to 29, 16 to 30, 17 to 18, 17 to 19, 17 to 20, 17 to 21, 17 to 22, 17 to 23, 17 to 24, 17 to 25, 17 to 26, 17 to 27, 17 to 28, 17 to 29, 17 to 30, 18 to 19, 18 to 20, 18 to 21, 18 to 22, 18 to 23, 18 to 24, 18 to 25, 18 to 26, 18 to 27, 18 to 28, 18 to 29, 18 to 30, 19 to 20, 19 to 21, 19 to 22, 19 to 23, 19 to 24, 19 to 25, 19 to 26, 19 to 29, 19 to 28, 19 to 29, 19 to 30, 20 to 21, 20 to 22, 20 to 23, 20 to 24, 20 to 25, 20 to 26, 20 to 27, 20 to 28, 20 to 29, 20 to 30, 21 to 22, 21 to 23, 21 to 24, 21 to 25, 21 to 26, 21 to 27, 21 to 28, 21 to 29, 21 to 30, 22 to 23, 22 to 24, 22 to 25, 22 to 26, 22 to 27, 22 to 28, 22 to 29, 22 to 30, 23 to 24, 23 to 25, 23 to 26, 23 to 27, 23 to 28, 23 to 29, 23 to 30, 24 to 25, 24 to 26, 24 to 27, 24 to 28, 24 to 29, 24 to 30, 25 to 26, 25 to 27, 25 to 28, 25 to 29, 25 to 30, 26 to 27, 26 to 28, 26 to 29, 26 to 30, 27 to 28, 27 to 29, 27 to 30, 28 to 29, 28 to 30, or 29 to 30 linked nucleosides

### E. Certain Modified Oligonucleotides

In certain embodiments, the above modifications (sugar, nucleobase, internucleoside linkage) are incorporated into a modified oligonucleotide. In certain embodiments, modified oligonucleotides are characterized by their modification motifs and overall lengths. In certain embodiments, such parameters are each independent of one another. Thus, unless otherwise indicated, each internucleoside linkage of an oligonucleotide having a gapmer sugar motif may be modified or unmodified and may or may not follow the gapmer modification pattern of the sugar modifications. For example, the internucleoside linkages within the wing regions of a sugar gapmer may be the same or different from one another and may be the same or different from the internucleoside linkages of the gap region of the sugar motif. Likewise, such sugar gapmer oligonucleotides may comprise one or more modified nucleobase independent of the gapmer pattern of the sugar modifications. Furthermore, in certain instances, an oligonucleotide is described by an overall length or range and by lengths or length ranges of two or more regions (e.g., a regions of nucleosides having specified sugar modifications), in such circumstances it may be possible to select numbers for each range that result in an oligonucleotide having an overall length falling outside the specified range. In such circumstances, both elements must be satisfied. For example, in certain embodiments, a modified oligonucleotide consists if of 15-20 linked nucleosides and has a sugar motif consisting of three regions, A, B, and C, wherein region A consists of 2-6 linked nucleosides having a specified sugar motif, region B consists of 6-10 linked nucleosides having a specified sugar motif, and region C consists of 2-6 linked nucleosides having a specified sugar motif. Such embodiments do not include modified oligonucleotides where A and C each consist of 6 linked nucleosides and B consists of 10 linked nucleosides (even though those numbers of nucleosides are permitted within the requirements for A, B, and C) because the overall length of such oligonucleotide is 22, which exceeds the upper limit of the overall length of the modified oligonucleotide (20). Herein, if a description of an oligonucleotide is silent with respect to one or more parameter, such parameter is not limited. Thus, a modified oligonucleotide described only as having a gapmer sugar motif without further description may have any length, internucleoside linkage motif, and nucleobase motif. Unless otherwise indicated, all modifications are independent of nucleobase sequence.

### F. Nucleobase Sequence

In certain embodiments, oligonucleotides (unmodified or modified oligonucleotides) are further described by their nucleobase sequence. In certain embodiments oligonucleotides have a nucleobase sequence that is complementary to a second oligonucleotide or an identified reference nucleic acid, such as a target precursor transcript. In certain such embodiments, a region of an oligonucleotide has a nucleobase sequence that is complementary to a second oligonucleotide or an identified reference nucleic acid, such as a target precursor transcript. In certain embodiments, the nucleobase sequence of a region or entire length of an oligonucleotide is at least 50%, at least 60%, at least 70%, at least 80%, at least 90%, at least 95%, or 100% complementary to the second oligonucleotide or nucleic acid, such as a target precursor transcript.

### II. Certain Oligomeric Compounds

In certain embodiments, the invention provides oligomeric compounds, which consist of an oligonucleotide (modified or unmodified) and optionally one or more conjugate groups and/or terminal groups. Conjugate groups consist of one or more conjugate moiety and a conjugate linker which links the conjugate moiety to the oligonucleotide. Conjugate groups may be attached to either or both ends of an oligonucleotide and/or at any internal position. In certain embodiments, conjugate groups are attached to the 2'-position of a nucleoside of a modified oligonucleotide. In certain embodiments, conjugate groups that are attached to either or both ends of an oligonucleotide are terminal groups. In certain such embodiments, conjugate groups or terminal groups are attached at the 3' and/or 5'-end of oligonucleotides. In certain such embodiments, conjugate groups (or terminal groups) are attached at the 3'-end of oligonucleotides. In certain embodiments, conjugate groups are attached near the 3'-end of oligonucleotides. In certain embodiments, conjugate groups (or terminal groups) are attached at the 5'-end of oligonucleotides. In certain embodiments, conjugate groups are attached near the 5'-end of oligonucleotides.

Examples of terminal groups include but are not limited to conjugate groups, capping groups, phosphate moieties, protecting groups, abasic nucleosides, modified or unmodified nucleosides, and two or more nucleosides that are independently modified or unmodified.

### A. Certain Conjugate Groups

In certain embodiments, oligonucleotides are covalently attached to one or more conjugate groups. In certain embodiments, conjugate groups modify one or more properties of the attached oligonucleotide, including but not limited to pharmacodynamics, pharmacokinetics, stability, binding, absorption, tissue distribution, cellular distribution, cellular uptake, charge and clearance. In certain embodiments, conjugate groups impart a new property on the attached oligonucleotide, *e.g*., fluorophores or reporter groups that enable detection of the oligonucleotide. Certain conjugate groups and conjugate moieties have been described previously, for example: cholesterol moiety (Letsinger et al., Proc. Natl. Acad. Sci. USA, 1989, 86, 6553-6556), cholic acid (Manoharan et al., Bioorg. Med. Chem. Lett., 1994, 4, 1053-1060), a thioether, *e.g.,* hexyl-S-tritylthiol (Manoharan et al., Ann. N. Y. Acad. Sci., 1992, 660, 306-309; Manoharan et al., Bioorg. Med. Chem. Lett., 1993, 3, 2765-2770), a thiocholesterol (Oberhauser et al., Nucl. Acids Res., 1992, 20, 533-538), an aliphatic chain, *e.g.,* do-decan-diol or undecyl residues (Saison-Behmoaras et al., EMBO J., 1991, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethyl-ammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid, a palmityl moiety (Mishra et al., Biochim. Biophys. Acta, 1995, 1264, 229-237), an octadecylamine or hexylamino-carbonyl-oxycholesterol moiety (Crooke et al., J. Pharmacol. Exp. Ther., 1996, 277, 923-937), a tocopherol group (Nishina et al., Molecular Therapy Nucleic Acids, 2015, 4, e220; and Nishina et al., Molecular Therapy, 2008, 16, 734-740), or a GalNAc cluster (*e.g*., WO2014/179620).

In certain embodiments, conjugate groups may be selected from any of a C22 alkyl, C20 alkyl, C16 alkyl, C10 alkyl, C21 alkyl, C19 alkyl, C18 alkyl, C15 alkyl, C14 alkyl, C13 alkyl, C12 alkyl, C11 alkyl, C9 alkyl, C8 alkyl, C7 alkyl, C6 alkyl, C5 alkyl, C22 alkenyl, C20 alkenyl, C16 alkenyl, C10 alkenyl, C21 alkenyl, C19 alkenyl, C18 alkenyl, C15 alkenyl, C14 alkenyl, C13 alkenyl, C12 alkenyl, C11 alkenyl, C9 alkenyl, C8 alkenyl, C7 alkenyl, C6 alkenyl, or C5 alkenyl.

In certain embodiments, conjugate groups may be selected from any of C22 alkyl, C20 alkyl, C16 alkyl, C10 alkyl, C21 alkyl, C19 alkyl, C18 alkyl, C15 alkyl, C14 alkyl, C13 alkyl, C12 alkyl, C11 alkyl, C9 alkyl, C8 alkyl, C7 alkyl, C6 alkyl, and C5 alkyl, where the alkyl chain has one or more unsaturated bonds.

### 1. Conjugate Moieties

Conjugate moieties include, without limitation, intercalators, reporter molecules, polyamines, polyamides, peptides, carbohydrates (e.g., GalNAc), vitamin moieties, polyethylene glycols, thioethers, polyethers, cholesterols, thiocholesterols, cholic acid moieties, folate, lipids, lipophilic groups, phospholipids, biotin, phenazine, phenanthridine, anthraquinone, adamantane, acridine, fluoresceins, rhodamines, coumarins, fluorophores, and dyes.

In certain embodiments, a conjugate moiety comprises an active drug substance, for example, aspirin, warfarin, phenylbutazone, ibuprofen, suprofen, fen-bufen, ketoprofen, (S)-(+)-pranoprofen, carprofen, dansylsarcosine, 2,3,5-triiodobenzoic acid, fingolimod, flufenamic acid, folinic acid, a benzothiadiazide, chlorothiazide, a diazepine, indo-methicin, a barbiturate, a cephalosporin, a sulfa drug, an antidiabetic, an antibacterial or an antibiotic.

### 2. Conjugate linkers

Conjugate moieties are attached to oligonucleotides through conjugate linkers. In certain oligomeric compounds, the conjugate linker is a single chemical bond (i.e., the conjugate moiety is attached directly to an oligonucleotide through a single bond). In certain oligomeric compounds, a conjugate moiety is attached to an oligonucleotide via a more complex conjugate linker comprising one or more conjugate linker moieities, which are sub-units making up a conjugate linker. In certain embodiments, the conjugate linker comprises a chain structure, such as a hydrocarbyl chain, or an oligomer of repeating units such as ethylene glycol, nucleosides, or amino acid units.

In certain embodiments, a conjugate linker comprises one or more groups selected from alkyl, amino, oxo, amide, disulfide, polyethylene glycol, ether, thioether, and hydroxylamino. In certain such embodiments, the conjugate linker comprises groups selected from alkyl, amino, oxo, amide and ether groups. In certain embodiments, the conjugate linker comprises groups selected from alkyl and amide groups. In certain embodiments, the conjugate linker comprises groups selected from alkyl and ether groups. In certain embodiments, the conjugate linker comprises at least one phosphorus moiety. In certain embodiments, the conjugate linker comprises at least one phosphate group. In certain embodiments, the conjugate linker includes at least one neutral linking group.

In certain embodiments, conjugate linkers, including the conjugate linkers described above, are bifunctional linking moieties, *e.g*., those known in the art to be useful for attaching conjugate groups to parent compounds, such as the oligonucleotides provided herein. In general, a bifunctional linking moiety comprises at least two functional groups. One of the functional groups is selected to bind to a particular site on a parent compound and the other is selected to bind to a conjugate group. Examples of functional groups used in a bifunctional linking moiety include but are not limited to electrophiles for reacting with nucleophilic groups and nucleophiles for reacting with electrophilic groups. In certain embodiments, bifunctional linking moieties comprise one or more groups selected from amino, hydroxyl, carboxylic acid, thiol, alkyl, alkenyl, and alkynyl.

Examples of conjugate linkers include but are not limited to pyrrolidine, 8-amino-3,6-dioxaoctanoic acid (ADO), succinimidyl 4-(N-maleimidomethyl) cyclohexane-1-carboxylate (SMCC) and 6-aminohexanoic acid (AHEX or AHA). Other conjugate linkers include but are not limited to substituted or unsubstituted C₁-C₁₀ alkyl, substituted or unsubstituted C₂-C₁₀ alkenyl or substituted or unsubstituted C₂-C₁₀ alkynyl, wherein a nonlimiting list of preferred substituent groups includes hydroxyl, amino, alkoxy, carboxy, benzyl, phenyl, nitro, thiol, thioalkoxy, halogen, alkyl, aryl, alkenyl and alkynyl.

In certain embodiments, conjugate linkers comprise 1-10 linker-nucleosides. In certain embodiments, such linker-nucleosides are modified nucleosides. In certain embodiments such linker-nucleosides comprise a modified sugar moiety. In certain embodiments, linker-nucleosides are unmodified. In certain embodiments, linker-nucleosides comprise an optionally protected heterocyclic base selected from a purine, substituted purine, pyrimidine or substituted pyrimidine. In certain embodiments, a cleavable moiety is a nucleoside selected from uracil, thymine, cytosine, 4-N-benzoylcytosine, 5-methylcytosine, 4-N-benzoyl-5-methylcytosine, adenine, 6-N-benzoyladenine, guanine and 2-N-isobutyrylguanine. It is typically desirable for linker-nucleosides to be cleaved from the oligomeric compound after it reaches a target tissue. Accordingly, linker-nucleosides are typically linked to one another and to the remainder of the oligomeric compound through cleavable bonds. In certain embodimements, such cleavable bonds are phosphodiester bonds.

Herein, linker-nucleosides are not considered to be part of the oligonucleotide. Accordingly, in embodiments in which an oligomeric compound comprises an oligonucleotide consisting of a specified number or range of linked nucleosides and/or a specified percent complementarity to a reference nucleic acid and the oligomeric compound also comprises a conjugate group comprising a conjugate linker comprising linker-nucleosides, those linker-nucleosides are not counted toward the length of the oligonucleotide and are not used in determining the percent complementarity of the oligonucleotide for the reference nucleic acid. For example, an oligomeric compound may comprise (1) a modified oligonucleotide consisting of 8-30 nucleosides and (2) a conjugate group comprising 1-10 linker-nucleosides that are contiguous with the nucleosides of the modified oligonucleotide. The total number of contiguous linked nucleosides in such an oligomeric compound is more than 30. Alternatively, an oligomeric compound may comprise a modified oligonucleotide consisting of 8-30 nucleosides and no conjugate group. The total number of contiguous linked nucleosides in such an oligomeric compound is no more than 30. Unless otherwise indicated conjugate linkers comprise no more than 10 linker-nucleosides. In certain embodiments, conjugate linkers comprise no more than 5 linker-nucleosides. In certain embodiments, conjugate linkers comprise no more than 3 linker-nucleosides. In certain embodiments, conjugate linkers comprise no more than 2 linker-nucleosides. In certain embodiments, conjugate linkers comprise no more than 1 linker-nucleoside.

In certain embodiments, it is desirable for a conjugate group to be cleaved from the oligonucleotide. For example, in certain circumstances oligomeric compounds comprising a particular conjugate moiety are better taken up by a particular cell type, but once the oligomeric compound has been taken up, it is desirable that the conjugate group be cleaved to release the unconjugated or parent oligonucleotide. Thus, certain conjugate linkers may comprise one or more cleavable moieties. In certain embodiments, a cleavable moiety is a cleavable bond. In certain embodiments, a cleavable moiety is a group of atoms comprising at least one cleavable bond. In certain embodiments, a cleavable moiety comprises a group of atoms having one, two, three, four, or more than four cleavable bonds. In certain embodiments, a cleavable moiety is selectively cleaved inside a cell or subcellular compartment, such as a lysosome. In certain embodiments, a cleavable moiety is selectively cleaved by endogenous enzymes, such as nucleases.

In certain embodiments, a cleavable bond is selected from among: an amide, an ester, an ether, one or both esters of a phosphodiester, a phosphate ester, a carbamate, or a disulfide. In certain embodiments, a cleavable bond is one or both of the esters of a phosphodiester. In certain embodiments, a cleavable moiety comprises a phosphate or phosphodiester. In certain embodiments, the cleavable moiety is a phosphate linkage between an oligonucleotide and a conjugate moiety or conjugate group.

In certain embodiments, a cleavable moiety comprises or consists of one or more linker-nucleosides. In certain such embodiments, the one or more linker-nucleosides are linked to one another and/or to the remainder of the oligomeric compound through cleavable bonds. In certain embodiments, such cleavable bonds are unmodified phosphodiester bonds. In certain embodiments, a cleavable moiety is 2'-deoxy nucleoside that is attached to either the 3' or 5'-terminal nucleoside of an oligonucleotide by a phosphate internucleoside linkage and covalently attached to the remainder of the conjugate linker or conjugate moiety by a phosphate or phosphorothioate linkage. In certain such embodiments, the cleavable moiety is 2'-deoxyadenosine.

### 3. Certain Cell-Targeting Conjugate Moietiess

In certain embodiments, a conjugate group comprises a cell-targeting conjugate moiety. In certain embodiments, a conjugate group has the general formula: 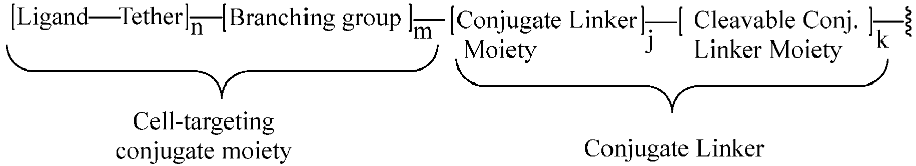 wherein n is from 1 to about 3, m is 0 when n is 1, m is 1 when n is 2 or greater, j is 1 or 0, and k is 1 or 0.

In certain embodiments, n is 1, j is 1 and k is 0. In certain embodiments, n is 1, j is 0 and k is 1. In certain embodiments, n is 1, j is 1 and k is 1. In certain embodiments, n is 2, j is 1 and k is 0. In certain embodiments, n is 2, j is 0 and k is 1. In certain embodiments, n is 2, j is 1 and k is 1. In certain embodiments, n is 3, j is 1 and k is 0. In certain embodiments, n is 3, j is 0 and k is 1. In certain embodiments, n is 3, j is 1 and k is 1.

In certain embodiments, conjugate groups comprise cell-targeting moieties that have at least one tethered ligand. In certain embodiments, cell-targeting moieties comprise two tethered ligands covalently attached to a branching group. In certain embodiments, cell-targeting moieties comprise three tethered ligands covalently attached to a branching group.

In certain embodiments, conjugate groups comprise a cell-targeting moiety having the formula:

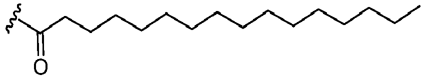

In certain embodiments, conjugate groups comprise a cell-targeting moiety having the formula: 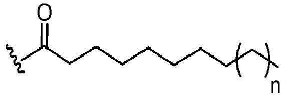 wherein n is an integer selected from 1, 2, 3, 4, 5, 6, or 7. In certain embodiments, n is 1. In certain embodiments, n is 2. In certain embodiments, n is 3. In certain embodiments, n is 4. In certain embodiments, n is 5.

In certain embodiments, conjugate groups comprise a cell-targeting moiety having the formula:

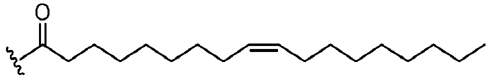

In certain embodiments, conjugate groups comprise a cell-targeting moiety having the formula:

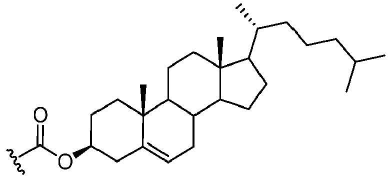

In certain embodiments, conjugate groups comprise a cell-targeting moiety having the formula:

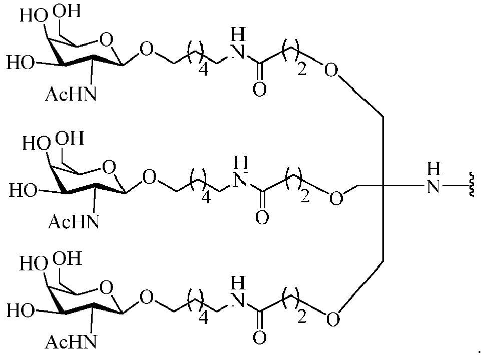

In certain embodiments, conjugate groups comprise a cell-targeting moiety having the formula:

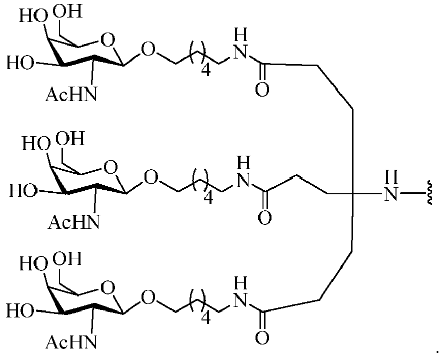

In certain embodiments, conjugate groups comprise a cell-targeting moiety having the formula:

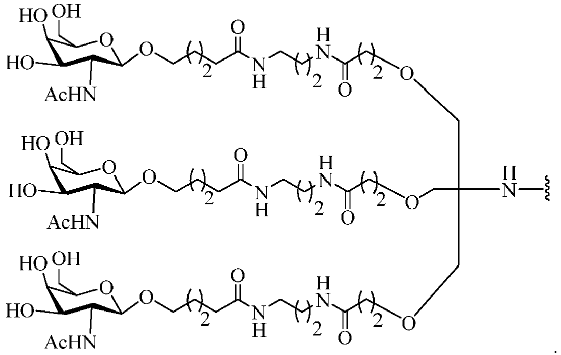

In certain embodiments, oligomeric compounds comprise a conjugate group described herein as "LICA-1". LICA-1 has the formula:

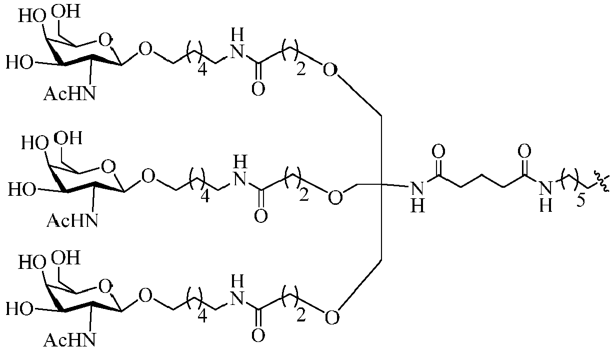

In certain embodiments, oligomeric compounds comprising LICA-1 have the formula: 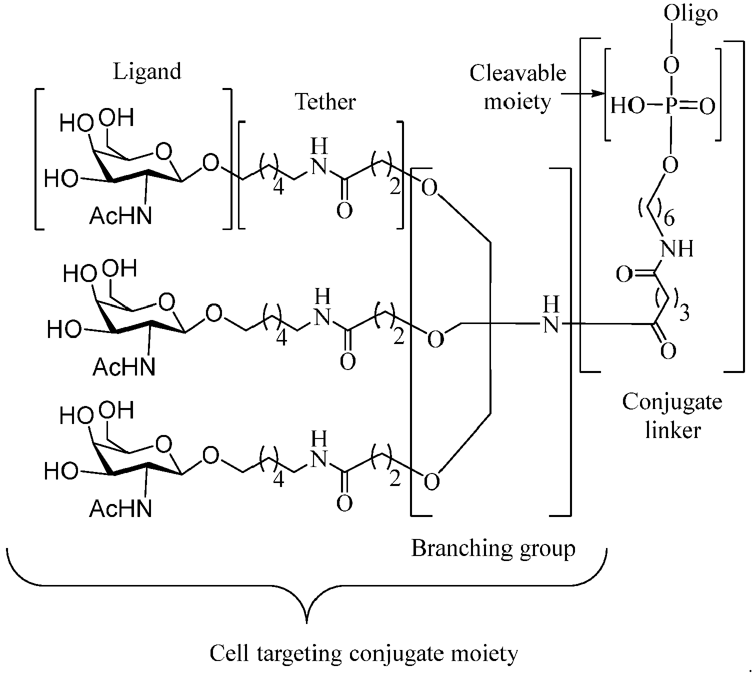 wherein oligo is an oligonucleotide.

Representative United States patents, United States patent application publications, international patent application publications, and other publications that teach the preparation of certain of the above noted conjugate groups, oligomeric compounds comprising conjugate groups, tethers, conjugate linkers, branching groups, ligands, cleavable moieties as well as other modifications include without limitation, US 5,994,517, US 6,300,319, US 6,660,720, US 6,906,182, US 7,262,177, US 7,491,805, US 8,106,022, US 7,723,509, US 2006/0148740, US 2011/0123520, WO 2013/033230 and WO 2012/037254, Biessen et al., J. Med. Chem. 1995, 38, 1846-1852, Lee et al., Bioorganic & Medicinal Chemistry 2011,19, 2494-2500, Rensen et al., J. Biol. Chem. 2001, 276, 37577-37584, Rensen et al., J. Med. Chem. 2004, 47, 5798-5808, Sliedregt et al., J. Med. Chem. 1999, 42, 609-618, and Valentijn et al., Tetrahedron, 1997, 53, 759-770.

In certain embodiments, oligomeric compounds comprise modified oligonucleotides comprising a fully modified sugar motif and a conjugate group comprising at least one, two, or three GalNAc ligands. In certain embodiments antisense compounds and oligomeric compounds comprise a conjugate group found in any of the following references: Lee, Carbohydr Res, 1978, 67, 509-514; Connolly et al., J Biol Chem, 1982, 257, 939-945; Pavia et al., Int J Pep Protein Res, 1983, 22, 539-548; Lee et al., Biochem, 1984, 23, 4255-4261; Lee et al., Glycoconjugate J, 1987, 4, 317-328; Toyokuni et al., Tetrahedron Lett, 1990, 31, 2673-2676; Biessen et al., J Med Chem, 1995, 38, 1538-1546; Valentijn et al., Tetrahedron, 1997, 53, 759-770; Kim et al., Tetrahedron Lett, 1997, 38, 3487-3490; Lee et al., Bioconjug Chem, 1997, 8, 762-765; Kato et al., Glycobiol, 2001, 11, 821-829; Rensen et al., J Biol Chem, 2001, 276, 37577-37584; Lee et al., Methods Enzymol, 2003, 362, 38-43; Westerlind et al., Glycoconj J, 2004, 21, 227-241; Lee et al., BioorgMed Chem Lett, 2006, 16(19), 5132-5135; Maierhofer et al., BioorgMed Chem, 2007, 15, 7661-7676; Khorev et al., BioorgMed Chem, 2008, 16, 5216-5231; Lee et al., BioorgMed Chem, 2011, 19, 2494-2500; Kornilova et al., Analyt Biochem, 2012, 425, 43-46; Pujol et al., Angew Chemie Int Ed Engl, 2012, 51, 7445-7448; Biessen et al., J Med Chem, 1995, 38, 1846-1852; Sliedregt et al., J Med Chem, 1999, 42, 609-618; Rensen et al., J Med Chem, 2004, 47, 5798-5808; Rensen et al., Arterioscler Thromb Vasc Biol, 2006, 26, 169-175; van Rossenberg et al., Gene Ther, 2004, 11, 457-464; Sato et al., J Am Chem Soc, 2004, 126, 14013-14022; Lee et al., J Org Chem, 2012, 77, 7564-7571; Biessen et al., FASEB J, 2000, 14, 1784-1792; Rajur et al., Bioconjug Chem, 1997, 8, 935-940; Duff et al., Methods Enzymol, 2000, 313, 297-321; Maier et al., Bioconjug Chem, 2003, 14, 18-29; Jayaprakash et al., Org Lett, 2010, 12, 5410-5413; Manoharan, Antisense Nucleic Acid Drug Dev, 2002, 12, 103-128; Merwin et al., Bioconjug Chem, 1994, 5, 612-620; Tomiya et al., Bioorg Med Chem, 2013, 21, 5275-5281; International applications WO1998/013381; WO2011/038356; WO1997/046098; WO2008/098788; WO2004/101619; WO2012/037254; WO2011/120053; WO2011/100131; WO2011/163121; WO2012/177947; WO2013/033230; WO2013/075035; WO2012/083185; WO2012/083046; WO2009/082607; WO2009/134487; WO2010/144740; WO2010/148013; WO1997/020563; WO2010/088537; WO2002/043771; WO2010/129709; WO2012/068187; WO2009/126933; WO2004/024757; WO2010/054406; WO2012/089352; WO2012/089602; WO2013/166121; WO2013/165816; U.S. Patents 4,751,219; 8,552,163; 6,908,903; 7,262,177; 5,994,517; 6,300,319; 8,106,022; 7,491,805; 7,491,805; 7,582,744; 8,137,695; 6,383,812; 6,525,031; 6,660,720; 7,723,509; 8,541,548; 8,344,125; 8,313,772; 8,349,308; 8,450,467; 8,501,930; 8,158,601; 7,262,177; 6,906,182; 6,620,916; 8,435,491; 8,404,862; 7,851,615; Published U.S. Patent Application Publications US2011/0097264; US2011/0097265; US2013/0004427; US2005/0164235; US2006/0148740; US2008/0281044; US2010/0240730; US2003/0119724; US2006/0183886; US2008/0206869; US2011/0269814; US2009/0286973; US2011/0207799; US2012/0136042; US2012/0165393; US2008/0281041; US2009/0203135; US2012/0035115; US2012/0095075; US2012/0101148; US2012/0128760; US2012/0157509; US2012/0230938; US2013/0109817; US2013/0121954; US2013/0178512; US2013/0236968; US2011/0123520; US2003/0077829; US2008/0108801; and US2009/0203132.

In certain embodiments, compounds of the invention are single-stranded. In certain embodiments, oligomeric compounds are paired with a second oligonucleotide or oligomeric compound to form a duplex, which is double-stranded.

### III. Certain Antisense Compounds

In certain embodiments, the present invention provides antisense compounds, which comprise or consist of an oligomeric compound comprising an antisense oliognucleotide, having a nucleobase sequences complementary to that of a target nucleic acid. In certain embodiments, antisense compounds are single-stranded. Such single-stranded antisense compounds typically comprise or consist of an oligomeric compound that comprises or consists of a modified oligonucleotide and optionally a conjugate group. In certain embodiments, antisense compounds are double-stranded. Such double-stranded antisense compounds comprise a first oligomeric compound having a region complementary to a target nucleic acid and a second oligomeric compound having a region complementary to the first oligomeric compound. The first oligomeric compound of such double stranded antisense compounds typically comprises or consists of a modified oligonucleotide and optionally a conjugate group. The oligonucleotide of the second oligomeric compound of such double-stranded antisense compound may be modified or unmodified. Either or both oligomeric compounds of a double-stranded antisense compound may comprise a conjugate group. The oligomeric compounds of double-stranded antisense compounds may include non-complementary overhanging nucleosides.

In certain embodiments, oligomeric compounds of antisense compounds are capable of hybridizing to a target nucleic acid, resulting in at least one antisense activity. In certain embodiments, antisense compounds selectively affect one or more target nucleic acid. Such selective antisense compounds comprises a nucleobase sequence that hybridizes to one or more target nucleic acid, resulting in one or more desired antisense activity and does not hybridize to one or more non-target nucleic acid or does not hybridize to one or more non-target nucleic acid in such a way that results in significant undesired antisense activity.

In certain embodiments, hybridization of an antisense compound to a target nucleic acid results in alteration of processing, e.g., splicing, of the target precursor transcript. In certain embodiments, hybridization of an antisense compound to a target precursor transcript results in inhibition of a binding interaction between the target nucleic acid and a protein or other nucleic acid. In certain such embodiments, hybridization of an antisense compound to a target precursor transcript results in alteration of translation of the target nucleic acid.

Antisense activities may be observed directly or indirectly. In certain embodiments, observation or detection of an antisense activity involves observation or detection of a change in an amount of a target nucleic acid or protein encoded by such target nucleic acid, a change in the ratio of splice variants of a nucleic acid or protein, and/or a phenotypic change in a cell or animal.

### IV. Certain Target Nucleic Acids

In certain embodiments, antisense compounds comprise or consist of an oligonucleotide comprising a region that is complementary to a target nucleic acid. In certain embodiments, the target nucleic acid is DNA encoding SMN2, including the human SMN2 genomic sequence provided in GENBANK Accession No. NT_006713.14 truncated from nucleotides 19939708_to 19967777, incorporated by reference herein as SEQ ID NO: 240. In certain embodiments, the target nucleic acid comprises SEQ ID NO: 10. Nucleotides 1-60 of SEQ ID NO: 10 represent a portion of intron 6 of SMN2 pre-mRNA, nucleotides 61-114 of SEQ ID NO: 10 represent exon 7 of SM2 pre-mRNA, and nucleotides 115-174 of SEQ ID NO: 10 represent a portion of intron 7 of SMN2 pre-mRNA. In certain embodiments, the target nucleic acid comprises ISS-N1 comprising the nucleobase sequence of SEQ ID NO: 11.

### A. Complementarity/Mismatches to the Target Nucleic Acid

In certain embodiments, antisense compounds and/or oligomeric compounds comprise antisense oligonucleotides that are complementary to the target nucleic acid over the entire length of the oligonucleotide. In certain embodiments, such oligonucleotides are 99% complementary to the target nucleic acid. In certain embodiments, such oligonucleotides are 95% complementary to the target nucleic acid. In certain embodiments, such oligonucleotides are 90% complementary to the target nucleic acid. In certain embodiments, such oligonucleotides are 85% complementary to the target nucleic acid. In certain embodiments, such oligonucleotides are 80% complementary to the target nucleic acid. In certain embodiments, antisense oligonucleotides are at least 80% complementary to the target nucleic acid over the entire length of the oligonucleotide and comprise a region that is 100% or fully complementary to a target nucleic acid. In certain such embodiments, the region of full complementarity is from 6 to 20 nucleobases in length. In certain such embodiments, the region of full complementarity is from 10 to 18 nucleobases in length. In certain such embodiments, the region of full complementarity is from 18 to 20 nucleobases in length.

In certain embodiments, oligomeric compounds and/or antisense compounds comprise one or more mismatched nucleobases relative to the target nucleic acid. In certain such embodiments, antisense activity against the target is reduced by such mismatch, but activity against a non-target is reduced by a greater amount. Thus, in certain such embodiments selectivity of the antisense compound is improved. In certain embodiments, the mismatch is specifically positioned within an oligonucleotide having a gapmer motif. In certain such embodiments, the mismatch is at position 1, 2, 3, 4, 5, 6, 7, or 8 from the 5'-end of the gap region. In certain such embodiments, the mismatch is at position 9, 8, 7, 6, 5, 4, 3, 2, 1 from the 3'-end of the gap region. In certain such embodiments, the mismatch is at position 1, 2, 3, or 4 from the 5'-end of the wing region. In certain such embodiments, the mismatch is at position 4, 3, 2, or 1 from the 3'-end of the wing region.

### B. Modulation of processing of certain target nucleic acids

In certain embodiments, oligomeric compounds comprise or consist of a modified oligonucleotide that is complementary to a target precursor transcript. In certain such embodiments, the target precursor transcript is a target pre-mRNA. In certain embodiments, contacting a cell with a compound complementary to a target precursor transcript modulates processing of the target precursor transcript. In certain such embodiments, the resulting target processed transcript has a different nucleobase sequence than the target processed transcript that is produced in the absence of the compound. In certain embodiments, the target precursor transcript is a target pre-mRNA and contacting a cell with a compound complementary to the target pre-mRNA modulates splicing of the target pre-mRNA. In certain such embodiments, the resulting target mRNA has a different nucleobase sequence than the target mRNA that is produced in the absence of the compound. In certain such embodiments, an exon is excluded from the target mRNA. In certain embodiments, an exon is included in the target mRNA. In certain embodiments, the exclusion or inclusion of an exon induces or prevents nonsense mediated decay of the target mRNA, removes or adds a premature termination codon from the target mRNA, and/or changes the reading frame of the target mRNA.

### C. Certain diseases and conditions associated with certain target nucleic acids

In certain embodiments, a target precursor transcript is associated with a disease or condition. In certain such embodiments, an oligomeric compound comprising or consisting of a modified oligonucleotide that is complementary to the target precursor transcript is used to treat the disease or condition. In certain such embodiments, the compound modulates processing of the target precursor transcript to produce a beneficial target processed transcript. In certain such embodiments, the disease or condition is associated with aberrant processing of a precursor transcript. In certain such embodiments, the disease or condition is associated with aberrant splicing of a pre-mRNA.

### V. Certain Pharmaceutical Compositions

In certain embodiments, the present invention provides pharmaceutical compositions comprising one or more antisense compound or a salt thereof. In certain such embodiments, the pharmaceutical composition comprises a suitable pharmaceutically acceptable diluent or carrier. In certain embodiments, a pharmaceutical composition comprises a sterile saline solution and one or more antisense compound. In certain embodiments, such pharmaceutical composition consists of a sterile saline solution and one or more antisense compound. In certain embodiments, the sterile saline is pharmaceutical grade saline. In certain embodiments, a pharmaceutical composition comprises one or more antisense compound and sterile water. In certain embodiments, a pharmaceutical composition consists of one antisense compound and sterile water. In certain embodiments, the sterile water is pharmaceutical grade water. In certain embodiments, a pharmaceutical composition comprises one or more antisense compound and phosphate-buffered saline (PBS). In certain embodiments, a pharmaceutical composition consists of one or more antisense compound and sterile PBS. In certain embodiments, the sterile PBS is pharmaceutical grade PBS.

In certain embodiments, pharmaceutical compositions comprise one or more or antisense compound and one or more excipients. In certain such embodiments, excipients are selected from water, salt solutions, alcohol, polyethylene glycols, gelatin, lactose, amylase, magnesium stearate, talc, silicic acid, viscous paraffin, hydroxymethylcellulose and polyvinylpyrrolidone.

In certain embodiments, antisense compounds may be admixed with pharmaceutically acceptable active and/or inert substances for the preparation of pharmaceutical compositions or formulations. Compositions and methods for the formulation of pharmaceutical compositions depend on a number of criteria, including, but not limited to, route of administration, extent of disease, or dose to be administered.

In certain embodiments, pharmaceutical compositions comprising an oligomeric compound and/or antisense compound encompass any pharmaceutically acceptable salts of the antisense compound, esters of the antisense compound, or salts of such esters. In certain embodiments, pharmaceutical compositions comprising antisense compounds and/or oligomeric compounds comprising one or more antisense oligonucleotide, upon administration to an animal, including a human, are capable of providing (directly or indirectly) the biologically active metabolite or residue thereof. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts. In certain embodiments, prodrugs comprise one or more conjugate group attached to an oligonucleotide, wherein the conjugate group is cleaved by endogenous nucleases within the body.

Lipid moieties have been used in nucleic acid therapies in a variety of methods. In certain such methods, the nucleic acid, such as an antisense compound, is introduced into preformed liposomes or lipoplexes made of mixtures of cationic lipids and neutral lipids. In certain methods, DNA complexes with mono- or poly-cationic lipids are formed without the presence of a neutral lipid. In certain embodiments, a lipid moiety is selected to increase distribution of a pharmaceutical agent to a particular cell or tissue. In certain embodiments, a lipid moiety is selected to increase distribution of a pharmaceutical agent to fat tissue. In certain embodiments, a lipid moiety is selected to increase distribution of a pharmaceutical agent to muscle tissue.

In certain embodiments, pharmaceutical compositions comprise a delivery system. Examples of delivery systems include, but are not limited to, liposomes and emulsions. Certain delivery systems are useful for preparing certain pharmaceutical compositions including those comprising hydrophobic compounds. In certain embodiments, certain organic solvents such as dimethylsulfoxide are used.

In certain embodiments, pharmaceutical compositions comprise one or more tissue-specific delivery molecules designed to deliver the one or more pharmaceutical agents of the present invention to specific tissues or cell types. For example, in certain embodiments, pharmaceutical compositions include liposomes coated with a tissue-specific antibody.

In certain embodiments, pharmaceutical compositions comprise a co-solvent system. Certain of such co-solvent systems comprise, for example, benzyl alcohol, a nonpolar surfactant, a water-miscible organic polymer, and an aqueous phase. In certain embodiments, such co-solvent systems are used for hydrophobic compounds. A non-limiting example of such a co-solvent system is the VPD co-solvent system, which is a solution of absolute ethanol comprising 3% w/v benzyl alcohol, 8% w/v of the nonpolar surfactant Polysorbate 80^{™} and 65% w/v polyethylene glycol 300. The proportions of such co-solvent systems may be varied considerably without significantly altering their solubility and toxicity characteristics. Furthermore, the identity of co-solvent components may be varied: for example, other surfactants may be used instead of Polysorbate 80^{™}; the fraction size of polyethylene glycol may be varied; other biocompatible polymers may replace polyethylene glycol, e.g., polyvinyl pyrrolidone; and other sugars or polysaccharides may substitute for dextrose.

In certain embodiments, pharmaceutical compositions are prepared for oral administration. In certain embodiments, pharmaceutical compositions are prepared for buccal administration. In certain embodiments, a pharmaceutical composition is prepared for administration by injection (e.g., intravenous, subcutaneous, intramuscular, etc.). In certain of such embodiments, a pharmaceutical composition comprises a carrier and is formulated in aqueous solution, such as water or physiologically compatible buffers such as Hanks's solution, Ringer's solution, or physiological saline buffer. In certain embodiments, other ingredients are included (e.g., ingredients that aid in solubility or serve as preservatives). In certain embodiments, injectable suspensions are prepared using appropriate liquid carriers, suspending agents and the like. Certain pharmaceutical compositions for injection are presented in unit dosage form, e.g., in ampoules or in multi-dose containers. Certain pharmaceutical compositions for injection are suspensions, solutions or emulsions in oily or aqueous vehicles, and may contain formulatory agents such as suspending, stabilizing and/or dispersing agents. Certain solvents suitable for use in pharmaceutical compositions for injection include, but are not limited to, lipophilic solvents and fatty oils, such as sesame oil, synthetic fatty acid esters, such as ethyl oleate or triglycerides, and liposomes. Aqueous injection suspensions may contain.

### Nonlimiting disclosure and incorporation by reference

All documents, or portions of documents, cited in this application, including, but not limited to, patents, patent applications, articles, books, treatises, and GenBank and NCBI reference sequence records are hereby expressly incorporated by reference in their entirety.

While certain compounds, compositions and methods described herein have been described with specificity in accordance with certain embodiments, the following examples serve only to illustrate the compounds described herein and are not intended to limit the same.

Although the sequence listing accompanying this filing identifies each sequence as either "RNA" or "DNA" as required, in reality, those sequences may be modified with any combination of chemical modifications. One of skill in the art will readily appreciate that such designation as "RNA" or "DNA" to describe modified oligonucleotides is, in certain instances, arbitrary. For example, an oligonucleotide comprising a nucleoside comprising a 2'-OH sugar moiety and a thymine base could be described as a DNA having a modified sugar (2'-OH in place of one 2'-H of DNA) or as an RNA having a modified base (thymine (methylated uracil) in place of a uracil of RNA). Accordingly, nucleic acid sequences provided herein, including, but not limited to those in the sequence listing, are intended to encompass nucleic acids containing any combination of natural or modified RNA and/or DNA, including, but not limited to such nucleic acids having modified nucleobases. By way of further example and without limitation, an oligomeric compound having the nucleobase sequence "ATCGATCG" encompasses any oligomeric compounds having such nucleobase sequence, whether modified or unmodified, including, but not limited to, such compounds comprising RNA bases, such as those having sequence "AUCGAUCG" and those having some DNA bases and some RNA bases such as "AUCGATCG" and oligomeric compounds having other modified nucleobases, such as "AT^{m}CGAUCG," wherein ^{m}C indicates a cytosine base comprising a methyl group at the 5-position.

Certain compounds described herein (e.g., modified oligonucleotides) have one or more asymmetric center and thus give rise to enantiomers, diastereomers, and other stereoisomeric configurations that may be defined, in terms of absolute stereochemistry, as (R) or (S), as α or β, such as for sugar anomers, or as (D) or (L), such as for amino acids, etc. Included in the compounds provided herein are all such possible isomers, including their racemic and optically pure forms, unless specified otherwise. Likewise, all cis- and trans- isomers and tautomeric forms are also included unless otherwise indicated. Oligomeric compounds described herein include chirally pure or enriched mixtures as well as racemic mixtures. For example, oligomeric compounds having a plurality of phosphorothioate internucleoside linkages include such compounds in which chirality of the phosphorothioate internucleoside linkages is controlled or is random.

Unless otherwise indicated, any compound, including oligomeric compounds, described herein includes a pharmaceutically acceptable salt thereof.

The compounds described herein include variations in which one or more atoms are replaced with a non-radioactive isotope or radioactive isotope of the indicated element. For example, compounds herein that comprise hydrogen atoms encompass all possible deuterium substitutions for each of the ¹H hydrogen atoms. Isotopic substitutions encompassed by the compounds herein include but are not limited to: ²H or ³H in place of ¹H, ¹³C or ¹⁴C in place of ¹²C, ¹⁵N in place of ¹⁴N, ¹⁷O or ¹⁸O in place of ¹⁶O, and ³³S, ³⁴S, ³⁵S, or ³⁶S in place of ³²S. In certain embodiments, non-radioactive isotopic substitutions may impart new properties on the oligomeric compound that are beneficial for use as a therapeutic or research tool. In certain embodiments, radioactive isotopic substitutions may make the compound suitable for research or diagnostic purposes such as imaging.

Herein, tables with a "SEQ ID NO." column first list a SEQ ID NO. including modifications, followed by the SEQ ID NO. of the corresponding nucleobase sequence without listing modifications. For example, in Table 1, the "SEQ ID NO." column shows "283" in the left-hand portion of the column for "Tₑₛ ^{m}Cₑₛ Aₑₛ ^{m}Cₑₛ Tₑₛ Tₑₛ Tₑₛ ^{m}Cₑₛ Aₑₛ Tₑₛ Aₑₛ Aₑₛ Tₑₛ Gₑₛ ^{m}Cₑₛ Tₑₛ Gₑₛ Gₑ", followed by "1" in the right-hand portion of the column for the corresponding nucleobase sequence of TCACTTTCATAATGCTGG.

### EXAMPLES

### Non-limiting disclosure and incorporation by reference

While certain compounds, compositions and methods described herein have been described with specificity in accordance with certain embodiments, the following examples serve only to illustrate the compounds described herein and are not intended to limit the same. Each of the references recited in the present application is incorporated herein by reference in its entirety.

### Example 1: Effect of modified oligonucleotides targeting SMN2 in vitro

Modified oligonucleotides comprising 2'-MOE or 2'-NMA modifications, shown in the table below, were tested *in vitro* for their effects on splicing of exon 7 in SMN2.

A spinal muscular atrophy (SMA) patient fibroblast cell line (GM03813: Cornell Institute) was plated at a density of 25,000 cells per well and transfected using electroporation at 120V with a concentration of modified oligonucleotide listed in the table below. After a treatment period of approximately 24 hours, cells were washed with DPBS buffer and lysed. RNA was extracted using Qiagen RNeasy purification and mRNA levels were measured by qRT-PCR. The level of SMN2 with exon 7 was measured using primer/probe set hSMN2vd#4_LTS00216_MGB; the level of SMN2 without exon 7 was measured using hSMN2va#4_LTS00215_MGB; and the level of total SMN2 was measured using HTS4210. The amounts of SMN2 with and without exon 7 were normalized to total SMN2. The results are presented in the table below as the levels of SMN2 with exon 7 (+ exon 7) relative to total SMN2 and the levels of SMN2 without exon 7 (- exon 7) relative to total SMN2. As illustrated in the table below, treatment with the modified oligonucleotide comprising 2'-NMA modifications exhibited greater exon 7 inclusion (and reduced exon 7 exclusion) compared to the modified oligonucleotide comprising 2'-MOE modifications in SMA patient fibroblast cells.

**Table 1: Modified oligonucleotides targeting human SMN2**

| Compound No. | Sequence (5' to 3') | SEQ ID NO. (modified / unmodified) | |
|---|---|---|---|
| 396443 | Tₑₛ ^{m}Cₑₛ Aₑₛ ^{m}Cₑₛ Tₑₛ Tₑₛ Tₑₛ ^{m}Cₑₛ Aₑₛ Tₑₛ Aₑₛ Aₑₛ Tₑₛ Gₑₛ ^{m}Cₑₛ Tₑₛ Gₑₛ Gₑ | 283 | 1 |
| 443305 | Tₙₛ ^{m}Cₙₛ Aₙₛ ^{m}Cₙₛ Tₙₛ Tₙₛ Tₙₛ ^{m}Cₙₛ Aₙₛ Tₙₛ Aₙₛ Aₙₛ Tₙₛ Gₙₛ ^{m}Cₙₛ Tₙₛ Gₙₛ Gₙ | 284 | 1 |

Subscripts in the table above: "s" represents a phosphorothioate internucleoside linkage, "e" represents a 2'-MOE modified nucleoside, "n" represents a 2'-O-(N-methylacetamide) modified nucleoside. Superscripts: "m" before a C represents a 5-methylcytosine.

**Table 2: Exon 7 inclusion and exclusion**

| Compound No. | Concentration (nM) | + exon7/total SMN | - exon7/total SMN |
|---|---|---|---|
| 396443 | 51 | 1.12 | 0.73 |
| | 128 | 1.16 | 0.59 |
| | 320 | 1.40 | 0.49 |
| | 800 | 1.34 | 0.41 |
| | 2000 | 1.48 | 0.37 |
| | 5000 | 1.57 | 0.37 |
| 443305 | 51 | 1.44 | 0.61 |
| | 128 | 1.42 | 0.45 |
| | 320 | 1.60 | 0.42 |
| | 800 | 1.60 | 0.38 |
| | 2000 | 1.63 | 0.36 |
| | 5000 | 1.63 | 0.42 |

### Example 2: Effect of modified oligonucleotides targeting SMN2 in transgenic mice

Taiwan strain of SMA Type III human transgenic mice (Jackson Laboratory, Bar Harbor, Maine) lack mouse SMN and are homozygous for human SMN2. These mice have been described in Hsieh-Li et al., Nature Genet. 24, 66-70 (2000). Each mouse received an intracerebroventricular (ICV) bolus of saline (PBS) or Compound 396443 or Compound 443305 (see Example 1) once on Day 1. Each treatment group consisted of 3-4 mice. The mice were sacrificed 7 days later, on Day 7. Total RNA from the spinal cord and brain was extracted and analyzed by RT-qPCR, as described in Example 1. The ratios of SMN2 with exon 7 to total SMN2 and SMN2 without exon 7 to total SMN2 were set to 1.0 for the PBS treated control group. The normalized results for all treatment groups are presented in the table below. As illustrated in the table below, the modified oligonucleotide comprising 2'-NMA modifications exhibited greater exon 7 inclusion and less exon 7 exclusion than the modified oligonucleotide comprising 2'-MOE modifications *in vivo.*

**Table 3: Exon 7 inclusion and exclusion**

| Compound No. | Dose (ug) | Spinal Cord | | | Brain | |
|---|---|---|---|---|---|---|
| | | + exon 7/total SMN | - exon 7/total SMN | ED₅₀ (ug) | + exon 7/total SMN | - exon 7/total SMN |
| PBS | 0 | 1.0 | 1.0 | n/a | 1.0 | 1.0 |
| 396443 | 10 | 2.1 | 0.8 | 15 | 1.6 | 0.9 |
| | 30 | 2.9 | 0.5 | | 2.5 | 0.7 |
| | 100 | 3.5 | 0.4 | | 3.3 | 0.5 |
| 443305 | 10 | 2.7 | 0.5 | 8 | 2.4 | 0.6 |
| | 30 | 3.6 | 0.3 | | 3.3 | 0.5 |
| | 100 | 3.8 | 0.3 | | 3.9 | 0.3 |

### Example 3: Effect of modified oligonucleotides targeting SMN2 in transgenic mice following systemic administration

Taiwan Type III human transgenic mice received an intraperitoneal (IP) injection of saline (PBS), Compound No. 396443, or Compound No. 443305 (see Example 1) once every 48 hours for a total of four injections. Each treatment group consisted of 3-4 mice. The mice were sacrificed 72 hours following the last dose. Various tissues including liver, diaphragm, quadriceps and heart were collected, and total RNA was isolated. SMN2 with and without exon 7 and total SMN2 levels were measured by RT-qPCR as described in Examples 1 and 2, except that the primer/probe sets for this experiment were those described in Tiziano, et al., Eur J Humn Genet, 2010. The results are presented in the tables below. The results show that systemic administration of the modified oligonucleotide comprising 2'-NMA modifications resulted in greater exon 7 inclusion and less exon 7 exclusion than the modified oligonucleotide comprising 2'-MOE modifications.

**Table 4: Exon 7 inclusion and exclusion**

| Comp. No. | Dose (mg/kg) | Liver | | Diaphragm | | Quadriceps | | Heart | |
|---|---|---|---|---|---|---|---|---|---|
| | | + exon 7/total SMN | - exon 7/total SMN | + exon 7/total SMN | - exon 7/total SMN | + exon 7/total SMN | - exon 7/total SMN | + exon 7/total SMN | - exon 7/total SMN |
| 396443 | 8.3 | 1.7 | 0.7 | 1.5 | 0.7 | 1.0 | 0.8 | 1.3 | 0.9 |
| | 25 | 2.6 | 0.4 | 2.3 | 0.6 | 1.2 | 0.8 | 1.4 | 0.9 |
| | 75 | 3.2 | 0.3 | 2.5 | 0.4 | 1.4 | 0.7 | 1.8 | 0.8 |
| 443305 | 8.3 | 2.1 | 0.4 | 2.2 | 0.5 | 1.3 | 0.8 | 1.3 | 0.8 |
| | 25 | 2.7 | 0.3 | 2.8 | 0.3 | 1.6 | 0.7 | 1.7 | 0.8 |
| | 75 | 3.3 | 0.2 | 3.3 | 0.3 | 2.3 | 0.4 | 2.1 | 0.5 |

**Table 5: ED₅₀ values (mg/kg) calculated from Table 4 results**

| Compound No. | Liver | Diaphragm | Quadriceps | Heart |
|---|---|---|---|---|
| 396443 | 13 | 27 | >75 | 32 |
| 443305 | 9 | 8 | 21 | 15 |

### Example 4: Effect of modified oligonucleotides targeting SMN2 in transgenic mice

Taiwan Type III human transgenic mice received an ICV bolus of saline (PBS) or a modified oligonucleotide listed in the table below. Each treatment group consisted of 3-4 mice. The mice were sacrificed two weeks following the dose. The brain and spinal cord of each mouse was collected, and total RNA was isolated from each tissue. SMN2 with and without exon 7 and total SMN2 levels were measured by RT-qPCR as described in Examples 1 and 2, and the results are presented in the tables below. The results show that the modified oligonucleotides comprising 2'-NMA modifications resulted greater exon 7 inclusion and less exon 7 exclusion than the modified oligonucleotide comprising 2'-MOE modifications.

**Table 6: Modified oligonucleotides targeting human SMN2**

| Comp. No. | Sequence | SEQ ID NO. (modified / unmodified) | |
|---|---|---|---|
| 387954 | Aₑₛ Tₑₛ Tₑₛ ^{m}Cₑₛ Aₑₛ ^{m}Cₑₛ Tₑₛ Tₑₛ Tₑₛ ^{m}Cₑₛ Aₑₛ Tₑₛ Aₑₛ Aₑₛ Tₑₛ Gₑₛ ^{m}Cₑₛ Tₑₛ Gₑₛ Gₑ | 304 | 2 |
| 443305 | Tₙₛ ^{m}Cₙₛ Aₙₛ ^{m}Cₙₛ Tₙₛ Tₙₛ Tₙₛ ^{m}Cₙₛ Aₙₛ Tₙₛ Aₙₛ Aₙₛ Tₙₛ Gₙₛ ^{m}Cₙₛ Tₙₛ Gₙₛ Gₙ | 284 | 1 |
| 819735 | ^{m}Cₙₛ Aₙₛ ^{m}Cₙₛ Tₙₛ Tₙₛ Tₙₛ ^{m}Cₙₛ Aₙₛ Tₙₛ Aₙₛ Aₙₛ Tₙₛ Gₙₛ ^{m}Cₙₛ Tₙₛ Gₙₛ Gₙₛ ^{m}Cₙ | 285 | 3 |
| 819736 | Tₙₛ ^{m}Cₙₛ Aₙₛ ^{m}Cₙₒ Tₙₛ Tₙₒ Tₙₛ ^{m}Cₙₒ Aₙₛ Tₙₒ Aₙₛ Aₙₒ Tₙₛ Gₙₒ ^{m}Cₙₛ Tₙₛ Gₙₛ Gₙ | 286 | 1 |

Subscripts in the table above: "s" represents a phosphorothioate internucleoside linkage, "e" represents a 2'-MOE modified nucleoside, "n" represents a 2'-O-(N-methylacetamide) modified nucleoside. Superscripts: "m" before a C represents a 5-methylcytosine.

**Table 7: Exon 7 inclusion and exclusion**

| Comp. No. | Dose (ug) | Spinal Cord | | Brain | | |
|---|---|---|---|---|---|---|
| | | + exon 7/total SMN | - exon 7/total SMN | + exon 7/total SMN | - exon 7/total SMN | ED₅₀ (µg) |
| PBS | 0 | 1.0 | 1.0 | 1.0 | 1.0 | n/a |
| 387954 | 10 | 3.2 | 0.6 | 1.5 | 0.8 | 40 |
| | 30 | 3.9 | 0.4 | 2.6 | 0.6 | |
| | 100 | 3.8 | 0.3 | 5.4 | 0.2 | |
| 443305 | 10 | 3.8 | 0.3 | 3.0 | 0.6 | 15 |
| | 30 | 4.1 | 0.2 | 4.3 | 0.4 | |
| | 100 | 4.2 | 0.1 | 5.4 | 0.2 | |
| 819735 | 10 | 3.5 | 0.4 | 3.3 | 0.6 | 13 |
| | 30 | 4.4 | 0.2 | 4.3 | 0.4 | |
| | 100 | 4.2 | 0.2 | 5.6 | 0.1 | |
| 819736 | 10 | 2.3 | 0.6 | 2.4 | 0.8 | 26 |
| | 30 | 3.3 | 0.4 | 3.7 | 0.6 | |
| | 100 | 4.3 | 0.2 | 4.9 | 0.3 | |

### Example 5: Effect of modified oligonucleotides targeting SMN2 in transgenic mice following systemic administration

Taiwan Type III human transgenic mice received a subcutaneous injection of saline (PBS) or a modified oligonucleotide listed in Example 4 once every 48-72 hours for a total of 10-150 mg/kg/week for three weeks. Each treatment group consisted of 4 mice. The mice were sacrificed 72 hours following the last dose. Various tissues were collected, and total RNA was isolated from each tissue. SMN2 with and without exon 7 and total SMN2 levels were measured by RT-qPCR as described in Examples 1 and 2, and the results are presented in the tables below. The results show that systemic administration of the modified oligonucleotides comprising 2'-NMA modifications resulted greater exon 7 inclusion and less exon 7 exclusion than the modified oligonucleotide comprising 2'-MOE modifications.

**Table 8: Exon 7 inclusion and exclusion**

| Comp. No. | Dose (mg/ kg/ wk) | Tissue | | | | | | | | | |
|---|---|---|---|---|---|---|---|---|---|---|---|
| | | Quadriceps | | TA Muscle | | Diaphragm | | Liver | | Lung | |
| | | + exon 7/ total SMN | exon 7/ total SMN | + exon 7/ total SMN | - exon 7/total SMN | + exon 7/total SMN | - exon 7/total SMN | + exon 7/total SMN | - exon 7/total SMN | + exon 7/total SMN | - exon 7/ total SMN |
| PBS | - | 1.0 | 1.0 | 1.0 | 1.0 | 1.0 | 1.0 | 1.0 | 1.0 | 1.0 | 1.0 |
| 387954 | 10 | 1.0 | 0.9 | 1.2 | 1.0 | 1.1 | 0.9 | 1.3 | 0.9 | 1.4 | 0.8 |
| | 30 | 1.2 | 0.8 | 1.5 | 0.9 | 1.4 | 0.8 | 1.8 | 0.6 | 1.4 | 0.6 |
| | 100 | 1.5 | 0.5 | 1.8 | 0.6 | 2.1 | 0.5 | 2.4 | 0.3 | 1.6 | 0.4 |
| | 150 | 1.6 | 0.4 | 2.3 | 0.5 | 2.3 | 0.4 | 2.7 | 0.2 | 1.8 | 0.4 |
| 443305 | 10 | 1.1 | 0.7 | 1.4 | 0.9 | 1.6 | 0.8 | 1.9 | 0.5 | 1.2 | 0.6 |
| | 30 | 1.4 | 0.5 | 1.7 | 0.7 | 2.1 | 0.5 | 2.6 | 0.3 | 1.6 | 0.5 |
| | 100 | 2 | 0.2 | 2.4 | 0.3 | 2.7 | 0.2 | 2.7 | 0.1 | 1.7 | 0.3 |
| | 150 | 2.1 | 0.2 | 2.8 | 0.2 | 2.9 | 0.2 | 2.9 | 0.1 | 1.7 | 0.3 |
| 819735 | 30 | 1.4 | 0.4 | 2 | 0.7 | 2.1 | 0.5 | 3.2 | 0.2 | 1.5 | 0.5 |
| | 100 | 2 | 0.2 | 2.8 | 0.3 | 3 | 0.2 | 3 | 0.1 | 1.8 | 0.4 |
| 819736 | 8.3 | 1.5 | 0.4 | 2 | 0.6 | 2 | 0.5 | 2.5 | 0.4 | 1.3 | 0.6 |

**Table 9: ED₅₀ values (mg/kg) calculated from Table 9 results**

| Comp. No. | Tissue | | | | |
|---|---|---|---|---|---|
| | Quadriceps | TA muscle | Diaphragm | Liver | Lung |
| 387954 | >150 | 142 | 105 | 57 | 31 |
| 443305 | 68 | 56 | 30 | 16 | 24 |
| 819735 | 58 | 37 | 31 | <30 | 25 |

| | | | | | |
|---|---|---|---|---|---|
| "n.d." indicates no data, the ED₅₀ was not calculated. | | | | | |

### Example 6: Effect of compounds comprising a conjugate group and a modified oligonucleotide targeting SMN2 in transgenic mice following systemic administration

Taiwan type III human transgenic mice were treated by subcutaneous administration with 10-300 mg/kg/week of a modified oligonucleotide listed in the table below or saline (PBS) alone for three weeks and sacrificed 48-72 hours after the last dose. There were 3-4 mice per group. Total RNA from various tissues was extracted and RT-qPCR was performed as described in Examples 1 and 2. The results presented in the table below show that the oligomeric compound comprising a C16 conjugate and 2'-NMA modifications exhibited greater exon 7 inclusion and less exon 7 exclusion than the other compounds tested.

**Table 10: Modified oligonucleotides targeting human SMN2**

| Comp. No. | Sequence (5' to 3') | SEQ ID NO. (modified / unmodified) | |
|---|---|---|---|
| 387954 | Aₑₛ Tₑₛ Tₑₛ ^{m}Cₑₛ Aₑₛ ^{m}Cₑₛ Tₑₛ Tₑₛ Tₑₛ ^{m}Cₑₛ Aₑₛ Tₑₛ Aₑₛ Aₑₛ Tₑₛ Gₑₛ ^{m}Cₑₛ Tₑₛ Gₑₛ Gₑ | 304 | 2 |
| 881068 | C16-HA-Aₑₛ Tₑₛ Tₑₛ ^{m}Cₑₛ Aₑₛ ^{m}Cₑₛ Tₑₛ Tₑₛ Tₑₛ ^{m}Cₑₛ Aₑₛ Tₑₛ Aₑₛ Aₑₛ Tₑₛ Gₑₛ ^{m}Cₑₛ Tₑₛ Gₑₛ Gₑ | 305 | 2 |
| 881069 | C16-HA -Tₑₛ ^{m}Cₑₛ Aₑₛ ^{m}Cₑₛ Tₑₛ Tₑₛ Tₑₛ ^{m}Cₑₛ Aₑₛ Tₑₛ Aₑₛ Aₑₛ Tₑₛ Gₑₛ ^{m}Cₑₛ Tₑₛ Gₑₛ Gₑ | 287 | 1 |
| 881070 | C16-HA -Tₑₛ ^{m}Cₑₛ Aₑₛ ^{m}Cₑₒ Tₑₛ Zₑₒ Tₑₛ ^{m}Cₑₒ Aₑₛ Zₑₒ Aₑₛ Aₑₒ Tₑₛ Gₑₒ ^{m}Cₑₛ Tₑₛ Gₑₛ Gₑ | 288 | 1 |
| 881071 | C16-HA -Tₙₛ ^{m}Cₙₛ Aₙₛ ^{m}Cₙₛ Tₙₛ Tₙₛ Tₙₛ ^{m}Cₙₛ Aₙₛ Tₙₛ Aₙₛ Aₙₛ Tₙₛ Gₙₛ ^{m}Cₙₛ Tₙₛ Gₙₛ Gₙ | 289 | 1 |

Subscripts in the table above: "s" represents a phosphorothioate internucleoside linkage, "o" represents a phosphate internucleoside linkage, "d" represents a 2'-deoxynucleoside, "e" represents a 2'-MOE modified nucleoside, "n" represents a 2'-O-(N-methylacetamide) modified nucleoside. Superscripts: "m" before a C represents a 5-methylcysteine.
The structure of C16-HA is:

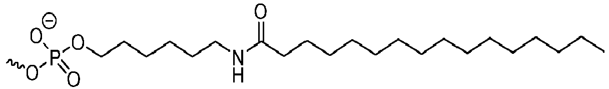

**Table 11: Exon 7 inclusion and exclusion**

| Comp. No. | Dose (mg/ kg/w k) | TA Muscle | | | Gastrocnemius | | | Diaphragm | | |
|---|---|---|---|---|---|---|---|---|---|---|
| | | + exon 7/ total SMN | - exon 7/ total SMN | ED₅₀ (mg/ kg) | + exon 7/ total SMN | - exon 7/ total SMN | ED₅₀ (mg/ kg) | + exon 7/ total SMN | - exon 7/ total SMN | ED₅₀ (mg/ kg) |
| PBS | - | 1.0 | 1 | n/a | 1.0 | 1.0 | n/a | 1.0 | 1.0 | n/a |
| 387954 | 30 | 1.0 | 0.9 | 242 | 1.0 | 1.0 | 204 | 1.5 | 0.8 | 122 |
| | 100 | 1.4 | 0.6 | | 1.7 | 0.7 | | 1.9 | 0.6 | |
| | 300 | 2.1 | 0.4 | | 2.3 | 0.3 | | 2.6 | 0.4 | |
| 881068 | 10 | 1.0 | 1.0 | 74 | 0.9 | 1.0 | 69 | 1.1 | 0.9 | 46 |
| | 30 | 1.3 | 0.8 | | 1.3 | 0.8 | | 1.7 | 0.7 | |
| | 100 | 2.2 | 0.2 | | 2.5 | 0.2 | | 2.8 | 0.2 | |
| 881069 | 10 | 1.0 | 1.0 | 56 | 1.0 | 1.0 | 53 | 1.3 | 0.8 | 33 |
| | 30 | 1.4 | 0.7 | | 1.6 | 0.8 | | 2.0 | 0.6 | |
| | 100 | 2.5 | 0.2 | | 2.6 | 0.2 | | 2.9 | 0.1 | |
| 881070 | 10 | 1.1 | 0.9 | 59 | 0.9 | 0.9 | 60 | 1.3 | 1.0 | 26 |
| | 30 | 1.5 | 0.7 | | 1.5 | 0.6 | | 2.3 | 0.6 | |
| | 100 | 2.3 | 0.2 | | 2.6 | 0.2 | | 3.0 | 0.2 | |
| 881071 | 10 | 1.4 | 0.7 | 23 | 1.5 | 0.7 | 19 | 2.0 | 0.6 | 12 |
| | 30 | 2.2 | 0.2 | | 2.5 | 0.2 | | 2.7 | 0.2 | |
| | 100 | 2.6 | 0.1 | | 2.8 | 0.1 | | 3.0 | 0.2 | |

### Example 7: Dose response effects of oligomeric compounds comprising a lipophilic conjugate group in vivo

The oligomeric compounds described in the table below are complementary to both human and mouse MALAT-1 transcripts. Their effects on MALAT-1 expression were tested *in vivo.* Male diet-induced obesity (DIO) mice each received an intravenous injection, via the tail vein, of an oligomeric compound listed in the table below or saline vehicle alone once per week for two weeks. Each treatment group consisted of three or four mice. Three days after the final injection, the animals were sacrificed. MALAT-1 RNA expression in the heart analyzed by RT-qPCR and normalized to total RNA using RiboGreen (Thermo Fisher Scientific, Carlsbad, CA) is shown below. The average results for each group are shown as the percent normalized MALAT-1 RNA levels relative to average results for the vehicle treated animals. The data below show that the oligomeric compounds comprising a lipophilic conjugate group were more potent in the heart compared to the parent compound that does not comprise a lipophilic conjugate group.

**Table 12: MALAT-1 expression in vivo**

| Isis No. | Sequence (5' to 3') | Dosage (µmol/kg/week) | MALAT-1 RNA level in heart (% Vehicle) | SEQ ID NO. (modified / unmodified) | |
|---|---|---|---|---|---|
| 556089 | Gₖₛ ^{m}Cₖₛ Aₖₛ T_{ds} T_{ds} ^{m}C_{ds} T_{ds} A_{ds} A_{ds} T_{ds} A_{ds} G_{ds} ^{m}C_{ds} Aₖₛ Gₖₛ ^{m}Cₖ | 0.2 | 105 | 269 | 4 |
| | | 0.6 | 104 | | |
| | | 1.8 | 74 | | |
| 812133 | Ole-HA-T_{do} ^{m}C_{do} A_{do} Gₖₛ ^{m}Cₖₛ Aₖₛ T_{ds} T_{ds} ^{m}C_{ds} T_{ds} A_{ds} A_{ds} T_{ds} A_{ds} G_{ds} ^{m}C_{ds} Aₖₛ Gₖₛ ^{m}Cₖ | 0.2 | 71 | 294 | 5 |
| | | 0.6 | 61 | | |
| | | 1.8 | 42 | | |
| 812134 | C16-HA-T_{do} ^{m}C_{do} A_{do} Gₖₛ ^{m}Cₖₛ Aₖₛ T_{ds} T_{ds} ^{m}C_{ds} T_{ds} A_{ds} A_{ds} T_{ds} A_{ds} G_{ds} ^{m}C_{ds} Aₖₛ Gₖₛ ^{m}Cₖ | 0.2 | 86 | 295 | 5 |
| | | 0.6 | 65 | | |
| | | 1.8 | 31 | | |

Subscript "k" represents a cEt modified bicyclic sugar moiety. See above Tables for additional subscripts and superscript. The structure of "C16-HA-", is shown in Example 2. The structure of "Ole-HA-" is:

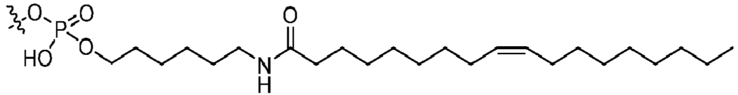

### Example 8: Effects of oligomeric compounds comprising a lipophilic conjugate group in vivo following different routes of administration

The effects of Isis Numbers 556089 and 812134 (see Example 9) on MALAT-1 expression were tested *in vivo.* Male, wild type C57bl/6 mice each received either an intravenous (IV) injection, via the tail vein, or a subcutaneous (SC) injection of Isis No. 556089, Isis No. 812134, or saline vehicle alone. Each treatment group consisted of four mice. Three days after the injection, the animals were sacrificed. MALAT-1 RNA expression analyzed from heart by RT-qPCR and normalized to total RNA using RiboGreen (Thermo Fisher Scientific, Carlsbad, CA) is shown below. The average results for each group are shown as the percent normalized MALAT-1 RNA levels relative to average results for the vehicle treated animals. The data below show that the oligomeric compound comprising a lipophilic conjugate group was more potent in the heart compared to the parent compound that does not comprise a lipophilic conjugate group.

**Table 13: MALAT-1 expression in vivo**

| Isis No. | Dosage (µmol/kg) | Route of administration | MALAT-1 RNA level in heart (% Vehicle) | SEQ ID NO. (modified / unmodified) | |
|---|---|---|---|---|---|
| 556089 | 0.4 | SC | 85 | 269 | 4 |
| | 1.2 | SC | 79 | | |
| | 3.6 | SC | 53 | | |
| | | IV | 56 | | |
| 812134 | 0.4 | SC | 71 | 295 | 5 |
| | 1.2 | SC | 48 | | |
| | 3.6 | SC | 29 | | |
| | | IV | 30 | | |

### Example 9: Effects of oligomeric compounds comprising a lipophilic conjugate group in vivo following different routes of administration

The compounds listed in the table below are complementary to CD36 and were tested *in vivo.* Female, wild type C57bl/6 mice each received either an intravenous injection or an intraperitoneal injection of a compound or saline vehicle alone once per week for three weeks. Each treatment group consisted of four mice. Three days after the final injection, the animals were sacrificed. CD36 mRNA expression analyzed from heart and quadriceps by RT-qPCR and normalized to total RNA using RiboGreen (Thermo Fisher Scientific, Carlsbad, CA) is shown below. The average results for each group are shown as the percent normalized CD36 RNA levels relative to average results for the vehicle treated animals. The data below show that the oligomeric compound comprising a lipophilic conjugate group was more potent in both heart and quadriceps compared to the parent compound that does not comprise a lipophilic conjugate group.

**Table 14: CD36 expression in vivo**

| Isis No. | Sequence (5' to 3') | Dose (µmol/kg/week) | Route of administration | CD36 mRNA level (% Vehicle) | | SEQ ID No. (modified / unmodified) | |
|---|---|---|---|---|---|---|---|
| | | | | Heart | Quad | | |
| 583363 | Aₖₛ Gₖₛ Gₖₛ A_{ds} T_{ds} A_{ds} T_{ds} G_{ds} G_{ds} A_{ds} A_{ds} ^{m}C_{ds} ^{m}C_{ds} Aₖₛ Aₖₛ Aₖ | 1 | IV | 102 | 84 | 270 | 6 |
| | | 3 | IV | 98 | 69 | | |
| | | 9 | IV | 81 | 30 | | |
| | | | IP | 94 | 36 | | |
| 847939 | C16-HA-T_{do} ^{m}C_{do} A_{do} Aₖₛ Gₖₛ Gₖₛ A_{ds} T_{ds} A_{ds} T_{ds} G_{ds} G_{ds} A_{ds} A_{ds} ^{m}C_{ds} ^{m}C_{ds} Aₖₛ Aₖₛ Aₖ | 1 | IV | 94 | 37 | 298 | 7 |
| | | 3 | IV | 69 | 22 | | |
| | | 9 | IV | 28 | 9 | | |
| | | | IP | 52 | 21 | | |

| | | | | | | | |
|---|---|---|---|---|---|---|---|
| See tables above for legend. | | | | | | | |

### Example 10: Effects of oligomeric compounds comprising a lipophilic conjugate group in vivo

The oligomeric compounds described in the table below are complementary to both human and mouse Dystrophia Myotonica-Protein Kinase (DMPK) transcript. Their effects on DMPK expression were tested *in vivo.* Wild type Balb/c mice each received an intravenous injection of an oligomeric compound at a dosage listed in the table below or saline vehicle alone. Each animal received one dose per week for 3 ½ weeks, for a total of 4 doses. Each treatment group consisted of three or four mice. Two days after the last dose, the animals were sacrificed. DMPK mRNA expression analyzed from quadriceps by RT-qPCR and normalized to total RNA using RiboGreen (Thermo Fisher Scientific, Carlsbad, CA) is shown below. The average results for each group are shown as the percent normalized DMPK RNA levels relative to average results for the vehicle treated animals. An entry of "nd" means no data. The data below show that the oligomeric compounds comprising a lipophilic conjugate group were more potent in the quadriceps compared to the parent compound that does not comprise a lipophilic conjugate group.

**Table 15: DMPK expression in vivo**

| Isis No. | Sequence (5' to 3') | Dosage (mg/kg/week) | DMPK mRNA level in quad (% Vehicle) | SEQ ID NO. (modified / unmodified) | |
|---|---|---|---|---|---|
| 486178 | Aₖₛ ^{m}Cₖₛ Aₖₛ A_{ds} T_{ds} A_{ds} A_{ds} A_{ds} T_{ds} A_{ds} ^{m}C_{ds} ^{m}C_{ds} G_{ds} Aₖₛ Gₖₛ Gₖ | 12.5 | 50 | 268 | 8 |
| | | 25 | 33 | | |
| | | 50 | 14 | | |
| 819733 | | 12.5 | 8 | 296 | 9 |
| | | 25 | nd | | |
| | Chol-TEG-T_{ds} mC_{do} A_{do} Aₖₛ ^{m}Cₖₛ Aₖₛ A_{ds} T_{ds} A_{ds} A_{ds} A_{ds} T_{ds} A_{ds} ^{m}C_{ds} ^{m}C_{ds} G_{ds} Aₖₛ Gₖₛ Gₖ | 50 | nd | | |
| 819734 | Toco-TEG-T_{ds} ^{m}C_{do} A_{do} Aₖₛ ^{m}Cₖₛ Aₖₛ A_{ds} T_{ds} A_{ds} A_{ds} A_{ds} T_{ds} A_{ds} ^{m}C_{ds} ^{m}C_{ds} G_{ds} Aₖₛ Gₖₛ Gₖ | 12.5 | 15 | 297 | 9 |
| | | 25 | 10 | | |
| | | 50 | 5 | | |

See tables above for legend. The structures of "Chol-TEG-" and "Toco-TEG-" are shown in Examples 1 and 2, respectively.
"HA-Chol" is a 2'-modification shown below: 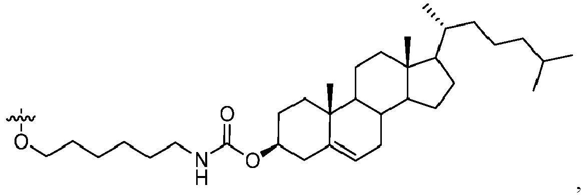
"HA-C10" and "HA-C16" are 2'-modifications shown below: 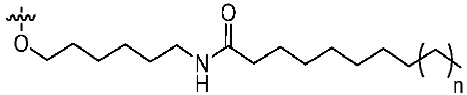 wherein n is 1 in subscript "HA-C10", and n is 7 in subscript "HA-C16".

### Example 11: Effects of oligomeric compounds in vivo

The oligomeric compounds described in the table below are complementary to both human and mouse MALAT-1 transcripts. Their effects on MALAT-1 expression were tested *in vivo.* Wild type male C57bl/6 mice each received a subcutaneous injection of an oligomeric compound at a dose listed in the table below or saline vehicle alone on days 0, 4, and 10 of the treatment period. Each treatment group consisted of three mice. Four days after the last injection, the animals were sacrificed. MALAT-1 RNA expression analyzed from heart by RT-qPCR and normalized to total RNA using RiboGreen (Thermo Fisher Scientific, Carlsbad, CA) is shown below. The average results for each group are shown as the percent normalized MALAT-1 RNA levels relative to average results for the vehicle treated animals. The data below show that the compounds comprising a lipophilic conjugate group were more potent in the heart compared to the parent compound that does not comprise a lipophilic conjugate group.

**Table 16: MALAT-1 expression in vivo**

| Isis No. | Sequence (5' to 3') | Dosage (µmol/kg) | MALAT-1 RNA level in heart (% Vehicle) | SEQ ID NO. (modified / unmodified) | |
|---|---|---|---|---|---|
| 556089 | Gₖₛ ^{m}Cₖₛ Aₖₛ T_{ds} T_{ds} ^{m}C_{ds} T_{ds} A_{ds} A_{ds} T_{ds} A_{ds} G_{ds} ^{m}C_{ds} Aₖₛ Gₖₛ ^{m}Cₖ | 0.4 | 83 | 269 | 4 |
| | | 1.2 | 81 | | |
| | | 3.6 | 57 | | |
| | | 10.8 | 27 | | |
| 812134 | C16-HA-T_{do} ^{m}C_{do} A_{do} Gₖₛ ^{m}Cₖₛ Aₖₛ T_{ds} T_{ds} ^{m}C_{ds} T_{ds} A_{ds} A_{ds} T_{ds} A_{ds} G_{ds} ^{m}C_{ds} Aₖₛ Gₖₛ ^{m}Cₖ | 0.4 | 88 | 295 | 5 |
| | | 1.2 | 69 | | |
| | | 3.6 | 17 | | |
| 859299 | C16-HA-Gₖₛ ^{m}Cₖₛ Aₖₛ T_{ds} T_{ds} ^{m}C_{ds} T_{ds} A_{ds} A_{ds} T_{ds} A_{ds} G_{ds} ^{m}C_{ds} Aₖₛ Gₖₛ ^{m}Cₖ | 0.4 | 80 | 271 | 4 |
| | | 1.2 | 42 | | |
| | | 3.6 | 14 | | |
| 861242 | C16-2x-C6-Gₖₛ ^{m}Cₖₛ Aₖₛ T_{ds} T_{ds} ^{m}C_{ds} T_{ds} A_{ds} A_{ds} T_{ds} A_{ds} G_{ds} ^{m}C_{ds} Aₖₛ Gₖₛ ^{m}Cₖ | 0.4 | 78 | 272 | 4 |
| | | 1.2 | 45 | | |
| | | 3.6 | 13 | | |
| 861244 | C16-C6-Gₖₛ ^{m}Cₖₛ Aₖₛ T_{ds} T_{ds} ^{m}C_{ds} T_{ds} A_{ds} A_{ds} T_{ds} A_{ds} G_{ds} ^{m}C_{ds} Aₖₛ Gₖₛ ^{m}Cₖ | 0.4 | 76 | 273 | 4 |
| | | 1.2 | 67 | | |
| | | 3.6 | 18 | | |
| 863406 | C16-2x-C3-Gₖₛ^{m}CₖₛAₖₛ T_{ds} T_{ds} ^{m}C_{ds} T_{ds} A_{ds} A_{ds} T_{ds} A_{ds} G_{ds} ^{m}C_{ds} Aₖₛ Gₖₛ ^{m}Cₖ | 0.4 | 97 | 274 | 4 |
| | | 1.2 | 63 | | |
| | | 3.6 | 26 | | |
| 863407 | C16-C3-Ab-Gₖₛ ^{m}Cₖₛ Aₖₛ T_{ds} T_{ds} ^{m}C_{ds} T_{ds} A_{ds} A_{ds} T_{ds} A_{ds} G_{ds} ^{m}C_{ds} Aₖₛ Gₖₛ ^{m}Cₖ | 0.4 | 109 | 278 | 4 |
| | | 1.2 | 67 | | |
| | | 3.6 | 32 | | |

See tables above for legend. The structure of "C16-HA-" is shown in Example 2.

The structures of "C16-2x-C6-" and "C16-2x-C3-" are: 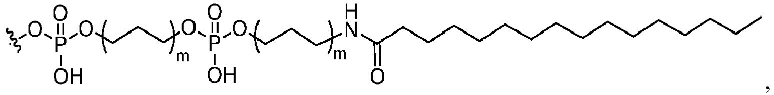
wherein m = 2 in "C16-2x-C6-"; and m = 1 in "C16-2x-C3-";
the structure of "C16-C6-" is: 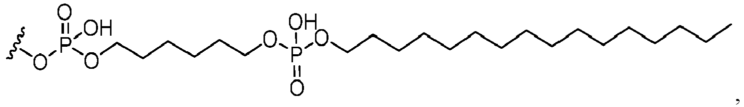
and the structure of "C16-C3-Ab-" is :

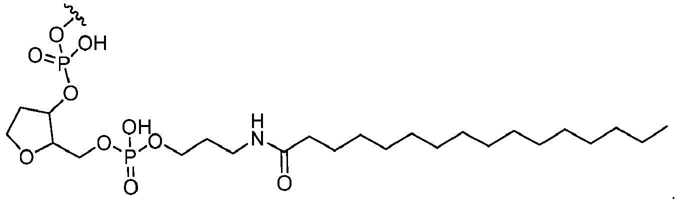

### EMBODIMENTS:

1. An oligomeric compound comprising a modified oligonucleotide consisting of 14-25 linked nucleosides, wherein the modified oligonucleotide is complementary to an SMN2 pre-mRNA; and wherein at least one nucleoside of the modified oligonucleotide has a structure of Formula II: 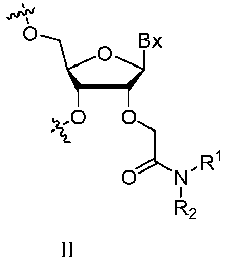 wherein for each nucleoside of Formula II:
   Bx is an independently selected nucleobase; and
   R¹ and R² are each independently selected from among: hydrogen and methyl,
   or R¹ is hydrogen and R² is independently selected from among ethyl, propyl, or isopropyl.
2. The oligomeric compound of embodiment 1, comprising at least one nucleoside of Formula II wherein one of R¹ and R² is not hydrogen.
3. The oligomeric compound of any of embodiments 1-2, wherein R¹ is hydrogen and R² is selected from among methyl, ethyl, propyl, or isopropyl for at least one nucleoside having a structure of Formula II.
4. The oligomeric compound of any of embodiments 1-3, comprising at least one nucleoside of Formula II wherein R¹ is hydrogen and R² is selected from among methyl or ethyl.
5. The oligomeric compound of any of embodiments 1-4, comprising at least one nucleoside of Formula II wherein at least one of R¹ and R² is methyl.
6. The oligomeric compound of any of embodiments 1-5, comprising at least one nucleoside of Formula II wherein one of R¹ and R² is hydrogen and the other of R¹ and R² is methyl.
7. The oligomeric compound of any of embodiments 1-6, wherein the selection for R¹ is different for at least two of the nucleosides having a structure of Formula II.
8. The oligomeric compound of any of embodiments 1-7, wherein the selection for R² is different for at least two of the nucleosides having a structure of Formula II.
9. The oligomeric compound of any of embodiments 1-6, wherein the selection for R¹ is the same for each of the nucleosides having a structure of Formula II, and the selection for R² is the same for each of the nucleosides having a structure of Formula II.
10. The oligomeric compound of any of embodiments 1-9, wherein Bx is selected from among adenine, guanine, cytosine, thymine, uracil, and 5-methyl cytosine.
11. The oligomeric compound of embodiment 1 or 2, wherein R¹ is methyl.
12. The oligomeric compound of any of embodiments 1-11, wherein each of at least 2 nucleosides of the modified oligonucleotide has a structure independently selected from Formula II.
13. The oligomeric compound of any of embodiments 1-11, wherein each of 7 nucleosides of the modified oligonucleotide has a structure independently selected from Formula II.
14. The oligomeric compound of any of embodiments 1-11, wherein each of 8 nucleosides of the modified oligonucleotide has a structure independently selected from Formula II.
15. The oligomeric compound of any of embodiments 1-11, wherein each of 9 nucleosides of the modified oligonucleotide has a structure independently selected from Formula II.
16. The oligomeric compound of any of embodiments 1-11, wherein each of 10 nucleosides of the modified oligonucleotide has a structure independently selected from Formula II.
17. The oligomeric compound of any of embodiments 1-11, wherein each of 11 nucleosides of the modified oligonucleotide has a structure independently selected from Formula II.
18. The oligomeric compound of any of embodiments 1-11, wherein each of 12 nucleosides of the modified oligonucleotide has a structure independently selected from Formula II.
19. The oligomeric compound of any of embodiments 1-11, wherein each of 13 nucleosides of the modified oligonucleotide has a structure independently selected from Formula II.
20. The oligomeric compound of any of embodiments 1-11, wherein each of 14 nucleosides of the modified oligonucleotide has a structure independently selected from Formula II.
21. The oligomeric compound of any of embodiments 1-11, wherein each of 15 nucleosides of the modified oligonucleotide has a structure independently selected from Formula II.
22. The oligomeric compound of any of embodiments 1-11, wherein each of 16 nucleosides of the modified oligonucleotide has a structure independently selected from Formula II.
23. The oligomeric compound of any of embodiments 1-11, wherein each of 17 nucleosides of the modified oligonucleotide has a structure independently selected from Formula II.
24. The oligomeric compound of any of embodiments 1-11, wherein each of 18 nucleosides of the modified oligonucleotide has a structure independently selected from Formula II.
25. The oligomeric compound of any of embodiments 1-11, wherein each of 19 nucleosides of the modified oligonucleotide has a structure independently selected from Formula II.
26. The oligomeric compound of any of embodiments 1-11, wherein each of 20 nucleosides of the modified oligonucleotide has a structure independently selected from Formula II.
27. The oligomeric compound of any of embodiments 1-26, wherein R¹ of at least one nucleoside having a structure of Formula II is methyl.
28. The oligomeric compound of any of embodiments 1-27, wherein R¹ is the same for all of the nucleosides having a structure of Formula II.
29. An oligomeric compound comprising a modified oligonucleotide consisting of 14-25 linked nucleosides, wherein the modified oligonucleotide is complementary to an SMN2 pre-mRNA; and wherein at least one nucleoside of the modified oligonucleotide comprises a 2'-O-(N-alkyl acetamide) modified sugar moiety.
30. The oligomeric compound of embodiment 29, wherein the sugar moiety of each nucleoside comprising a 2'-O-(N-alkyl acetamide) modified sugar moiety is selected from 2'-O-(N-methyl acetamide) and 2'-O-(N-ethyl acetamide).
31. The oligomeric compound of embodiment 29 or 30, wherein each of 7 nucleosides of the modified oligonucleotide comprises an independently selected 2'-O-(N-alkyl acetamide) modified sugar moiety.
32. The oligomeric compound of embodiment 29 or 30, wherein each of 8 nucleosides of the modified oligonucleotide comprises an independently selected 2'-O-(N-alkyl acetamide) modified sugar moiety.
33. The oligomeric compound of embodiment 29 or 30, wherein each of 9 nucleosides of the modified oligonucleotide comprises an independently selected 2'-O-(N-alkyl acetamide) modified sugar moiety.
34. The oligomeric compound of embodiment 29 or 30, wherein each of 10 nucleosides of the modified oligonucleotide comprises an independently selected 2'-O-(N-alkyl acetamide) modified sugar moiety.
35. The oligomeric compound of embodiment 29 or 30, wherein each of 11 nucleosides of the modified oligonucleotide comprises an independently selected 2'-O-(N-alkyl acetamide) modified sugar moiety.
36. The oligomeric compound of embodiment 29 or 30, wherein each of 12 nucleosides of the modified oligonucleotide comprises an independently selected 2'-O-(N-alkyl acetamide) modified sugar moiety.
37. The oligomeric compound of embodiment 29 or 30, wherein each of 13 nucleosides of the modified oligonucleotide comprises an independently selected 2'-O-(N-alkyl acetamide) modified sugar moiety.
38. The oligomeric compound of embodiment 29 or 30, wherein each of 14 nucleosides of the modified oligonucleotide comprises an independently selected 2'-O-(N-alkyl acetamide) modified sugar moiety.
39. The oligomeric compound of embodiment 29 or 30, wherein each of 15 nucleosides of the modified oligonucleotide comprises an independently selected 2'-O-(N-alkyl acetamide) modified sugar moiety.
40. The oligomeric compound of embodiment 29 or 30, wherein each of 16 nucleosides of the modified oligonucleotide comprises an independently selected 2'-O-(N-alkyl acetamide) modified sugar moiety.
41. The oligomeric compound of embodiment 29 or 30, wherein each of 17 nucleosides of the modified oligonucleotide comprises an independently selected 2'-O-(N-alkyl acetamide) modified sugar moiety.
42. The oligomeric compound of embodiment 29 or 30, wherein each of 18 nucleosides of the modified oligonucleotide comprises an independently selected 2'-O-(N-alkyl acetamide) modified sugar moiety.
43. The oligomeric compound of embodiment 29 or 30, wherein each of 19 nucleosides of the modified oligonucleotide comprises an independently selected 2'-O-(N-alkyl acetamide) modified sugar moiety.
44. The oligomeric compound of embodiment 29 or 30, wherein each of 20 nucleosides of the modified oligonucleotide comprises an independently selected 2'-O-(N-alkyl acetamide) modified sugar moiety.
45. The oligomeric compound of any of embodiments 29-44, wherein the sugar moiety of at least one of the nucleosides comprising a 2'-O-(N-alkyl acetamide) modified sugar moiety is a 2'-O-(N-methyl acetamide) modified sugar moiety.
46. The oligomeric compound of any of embodiments 29-45, wherein the N-alkyl group of each of the 2'-O-(N-alkyl acetamide) modified sugar moieties is the same N-alkyl group.
47. The oligomeric compound of any of embodiments 29-46, wherein each of the 2'-O-(N-alkyl acetamide) modified sugar moieties is a 2'-O-(N-methyl acetamide) sugar moiety.
48. The oligomeric compound of any of embodiments 29-47, wherein each sugar moiety of each nucleoside of the modified oligonucleotide is a 2'-O-(N-methyl acetamide) modified sugar moiety.
49. The oligomeric compound of any of embodiments 29-47, wherein at least one sugar moiety is not a 2'-O-(N-alkyl acetamide) modified sugar moiety.
50. The oligomeric compound of any of embodiments 29-47, wherein at least one sugar moiety is not a 2'-O-(N-methyl acetamide) modified sugar moiety.
51. The oligomeric compound of any of embodiments 29-47, wherein at least one sugar moiety is a modified sugar moiety selected from among: a 2'-MOE nucleoside, a 2'-OMe nucleoside, a 2'-F nucleoside, a (4'-CH₂-O-2') bicyclic nucleoside, a (4'-(CH₂)₂-O-2') bicyclic nucleoside, a (4'-C(CH₃)H-O-2') bicyclic nucleoside; and a morpholino.
52. The oligomeric compound of any of embodiments 29-51, wherein at least one sugar moiety is an unmodified sugar moiety.
53. The oligomeric compound of any of embodiments 1-52, wherein the modified oligonucleotide consists of 16-23 linked nucleosides.
54. The oligomeric compound of any of embodiments 1-52, wherein the modified oligonucleotide consists of 18-20 linked nucleosides.
55. The oligomeric compound of any of embodiments 1-53, wherein the modified oligonucleotide consists of 16 nucleosides.
56. The oligomeric compound of any of embodiments 1-53, wherein the modified oligonucleotide consists of 17 nucleosides.
57. The oligomeric compound of any of embodiments 1-53, wherein the modified oligonucleotide consists of 18 nucleosides.
58. The oligomeric compound of any of embodiments 1-53, wherein the modified oligonucleotide consists of 19 nucleosides.
59. The oligomeric compound of any of embodiments 1-53, wherein the modified oligonucleotide consists of 20 nucleosides.
60. The oligomeric compound of any of embodiments 1-59, wherein the modified oligonucleotide comprises at least one modified internucleoside linkage.
61. The oligomeric compound of any of embodiments 1-60, wherein the modified oligonucleotide comprises at least one phosphorothioate internucleoside linkage.
62. The oligomeric compound of embodiment 61, wherein each internucleoside linkage of the modified oligonucleotide is selected from among a phosphorothioate internucleoside linkage and a phosphate internucleoside linkage.
63. The oligomeric compound of embodiment 62, wherein the phosphate internucleoside linkage is a phosphodiester internucleoside linkage.
64. The oligomeric compound of embodiment 62, wherein the modified oligonucleotide has 5 phosphodiester internucleoside linkage.
65. The oligomeric compound of embodiment 62, wherein the modified oligonucleotide has 6 phosphodiester internucleoside linkages.
66. The oligomeric compound of embodiment 62, wherein the modified oligonucleotide has at least 6 phosphodiester internucleoside linkages.
67. The oligomeric compound of any of embodiments 1-66, wherein each internucleoside linkage of the modified oligonucleotide is a phosphorothioate internucleoside linkage.
68. The oligomeric compound of any of embodiments 1-67, wherein the modified oligonucleotide comprises at least one modified nucleobase.
69. The oligomeric compound of any of embodiments 1-68, wherein the modified oligonucleotide comprises at least one 5-methyl cytosine.
70. The oligomeric compound of any of embodiments 1-69, wherein each nucleobase of the modified oligonucleotide is selected from among thymine, 5-methyl cytosine, cytosine, adenine, uracil, and guanine.
71. The oligomeric compound of any of embodiments 1-70, wherein each cytosine of the modified oligonucleotide is a 5-methyl cytosine.
72. The oligomeric compound of any of embodiments 1-65, wherein each nucleobase of the modified oligonucleotide is selected from among thymine, 5-methyl cytosine, adenine, and guanine.
73. The oligomeric compound of any of embodiments 1-72, wherein the modified oligonucleotide is at least 70% complementary to the SMN2 pre-mRNA.
74. The oligomeric compound of any of embodiments 1-72, wherein the modified oligonucleotide is at least 75% complementary to the SMN2 pre-mRNA.
75. The oligomeric compound of any of embodiments 1-72, wherein the modified oligonucleotide is at least 80% complementary to the SMN2 pre-mRNA.
76. The oligomeric compound of any of embodiments 1-72, wherein the modified oligonucleotide is at least 85% complementary to the SMN2 pre-mRNA.
77. The oligomeric compound of any of embodiments 1-72, wherein the modified oligonucleotide is at least 90% complementary to the SMN2 pre-mRNA.
78. The oligomeric compound of any of embodiments 1-72, wherein the modified oligonucleotide is at least 95% complementary to the SMN2 pre-mRNA.
79. The oligomeric compound of any of embodiments 1-72, wherein the modified oligonucleotide is at least 100% complementary to the SMN2 pre-mRNA.
80. The oligomeric compound of any of embodiments 1-72, wherein the modified oligonucleotide is complementary to intron 7 of the SMN2 pre-mRNA.
81. The oligomeric compound of any of embodiments 1-80, wherein the modified oligonucleotide is complementary to ISS-N1 of the SMN2 pre-mRNA.
82. The oligomeric compound of any of embodiments 1-79, wherein the nucleobase sequence of the modified oligonucleotide comprises a sequence selected from among SEQ ID NOs: 1, 2, or 3.
83. The oligomeric compound of any of embodiments 1-79, wherein the nucleobase sequence of the modified oligonucleotide comprises a sequence selected from among any of SEQ ID NOs: 12-239 or 241-265.
84. The oligomeric compound of any of embodiments 1-79, wherein the nucleobase sequence of the modified oligonucleotide comprises a sequence selected from among any of SEQ ID NOs: 241, 242, 243, 244, 245, 246, 247, 248, 249, 250, 251, 252, 253, 254, 255, 256, 257, 258, 259, 260, 261, 262, 263, 264, or 265.
85. The oligomeric compound of any of embodiments 1-79, wherein the nucleobase sequence of the modified oligonucleotide consists of a sequence selected from among any of SEQ ID NOs: 1, 2, 3, or 12-239 or 241-265.
86. The oligomeric compound of any of embodiments 1-79, wherein the nucleobase sequence of the modified oligonucleotide consists of a sequence selected from among any of SEQ ID NOs: 241, 242, 243, 244, 245, 246, 247, 248, 249, 250, 251, 252, 253, 254, 255, 256, 257, 258, 259, 260, 261, 262, 263, 264, or 265.
87. The oligomeric compound of embodiment 1-79, wherein the modified oligonucleotide is complementary to exon 7 of the SMN2 pre-mRNA.
88. The oligomeric compound of embodiment 1-79, wherein the modified oligonucleotide is complementary to intron 6 of the SMN2 pre-mRNA.
89. The oligomeric compound of any of embodiments 1-79, wherein the modified oligonucleotide is complementary to an exonic splicing enhancer in exon 7 of the SMN2 pre-mRNA.
90. The oligomeric compound of any of embodiments 1-79, wherein the modified oligonucleotide is complementary to an aberrant exonic splicing silencer in exon 7 of the SMN2 pre-mRNA.
91. The oligomeric compound of any of embodiments 1-90, wherein the oligomeric compound comprises a conjugate group.
92. The oligomeric compound of embodiment 91, wherein the conjugate group comprises a lipid or lipophilic group.
93. The oligomeric compound of embodiment 92, wherein the lipid or lipophilic group is selected from among: cholesterol, a C₁₀-C₂₆ saturated fatty acid, a C₁₀- C₂₆ unsaturated fatty acid, C₁₀-C₂₆ alkyl, a triglyceride, tocopherol, or cholic acid.
94. The oligomeric compound of embodiment 93, wherein the lipid or lipophilic group is a saturated hydrocarbon chain or an unsaturated hydrocarbon chain.
95. The oligomeric compound of any of embodiments 92-94, wherein the lipid or lipophilic group is a C₁₆ lipid.
96. The oligomeric compound of any of embodiments 92-94, wherein the lipid or lipophilic group is a Cis lipid.
97. The oligomeric compound of any of embodiments 92-94, wherein the lipid or lipophilic group is C₁₆ alkyl.
98. The oligomeric compound of any of embodiments 92-94, wherein the lipid or lipophilic group is C₁₈ alkyl.
99. The oligomeric compound of embodiment 93, wherein the lipid or lipophilic group is cholesterol.
100. The oligomeric compound of embodiment 93, wherein the lipid or lipophilic group is tocopherol.
101. The oligomeric compound of embodiment 93, wherein the lipid or lipophilic group is saturated C₁₆.
102. The oligomeric compound of any of embodiments 91-101, wherein the conjugate group is attached to the modified oligonucleotide at the 5'-end of the modified oligonucleotide.
103. The oligomeric compound of any of embodiments 91-101, wherein the conjugate group is attached to the modified oligonucleotide at the 3'-end of the modified oligonucleotide.
104. The oligomeric compound of any of embodiments 91-103, wherein the conjugate group comprises a cleavable linker.
105. The oligomeric compound of embodiment 105 wherein the cleavable linker comprises one or more linker nucleosides.
106. The oligomeric compound of any of embodiments 1-105 consisting of the modified oligonucleotide.
107. The oligomeric compound of any of embodiments 91-105 consisting of the modified oligonucleotide and the conjugate group.
108. The oligomeric compound of any of embodiments 1-108, wherein the oligomeric compound modulates splicing of the SMN2 pre-mRNA.
109. The oligomeric compound of any of embodiments 1-108, wherein the oligomeric compound is single stranded.
110. The oligomeric compound of any of embodiments 1-108, wherein the oligomeric compound is paired with a complementary oligomeric compound to form a double stranded compound.
111. The oligomeric compound of embodiment 110, wherein the complementary oligomeric compound comprises a conjugate group.
112. A pharmaceutical composition comprising the oligomeric compound of any of embodiments 1-111 and at least one pharmaceutically acceptable carrier or diluent.
113. A method of modulating splicing of an SMN2 pre-mRNA comprising contacting a cell, tissue, or organ with the oligomeric compound or composition of any of embodiments 1-112.
114. A method of promoting inclusion of exon 7 in SMN2 mRNA in a cell, tissue or organ, comprising contacting said cell, tissue or organ with the oligomeric compound of any of embodiments 1 to 111 or the composition of embodiment 112.
115. The method of embodiment 113 or 114, wherein the cell, tissue, or organ is in an animal.
116. The method of embodiments 113 or 114, wherein the cell, tissue, or organ is in a human.
117. A method comprising administering the oligomeric compound of any of embodiments 1 to 111 or the composition of embodiment 112 to a subject.
118. The method of embodiment 117, wherein the subject has SMA.
119. The method of embodiment 117, wherein the subject has Type I SMA.
120. The method of embodiment 117, wherein the subject has Type II SMA.
121. The method of embodiment 117, wherein the subject has Type III SMA.
122. The method of embodiment 117, wherein the subject has Type IV SMA.
123. A method of treating Spinal Muscular Atrophy in a patient, comprising administering the oligomeric compound of any of embodiments 1 to 111 or the composition of embodiment 112 to a patient in need thereof.
124. A method of treating Spinal Muscular Atrophy Type I in a patient, comprising administering the oligomeric compound of any of embodiments 1 to 111 or the composition of embodiment 112 to a patient in need thereof.
125. A method of treating Spinal Muscular Atrophy Type II in a patient, comprising administering the oligomeric compound of any of embodiments 1 to 111 or the composition of embodiment 112 to a patient in need thereof.
126. A method of treating Spinal Muscular Atrophy Type III in a patient, comprising administering the oligomeric compound of any of embodiments 1 to 111 or the composition of embodiment 112 to a patient in need thereof.
127. A method of treating Spinal Muscular Atrophy Type IV in a patient, comprising administering the oligomeric compound of any of embodiments 1 to 111 or the composition of embodiment 112 to a patient in need thereof.
128. The method of any of embodiments 122-127, wherein the oligomeric compound is administered to the central nervous system.
129. The method of embodiment 128, wherein the oligomeric compound is administered intrathecally.
130. The method of any of embodiments 123-127, wherein the oligomeric compound is administered systemically.
131. The method of embodiment 130, wherein the oligomeric compound is administered subcutaneously.
132. The method of embodiment 130, wherein the oligomeric compound is administered intramuscularly.
133. The method of any of embodiments 122-132, wherein the oligomeric compound is administered to the central nervous system and systemically.
134. The method of any of embodiments 122-132, wherein the oligomeric compound is co-administered with a second compound.
135. The method of embodiment 134, wherein the second compound is selected from among a small molecule and an oligomeric compound.
136. The method of embodiment 135, wherein the second compound is an oligomeric compound according to embodiments 1-111.
137. The method of any of embodiments 134-136, wherein the second compound is administered to the CSF.
138. The method of embodiment 137, wherein the second compound is administered intrathecally.
139. The method of any of embodiments 134-138, wherein the second compound is a second oligomeric compound comprising a second modified oligonucleotide having at least one modified nucleoside comprising a modified sugar moiety.
140. The method of embodiment 139, wherein the modified sugar moiety of the modified nucleoside of the second modified oligonucleotide of the second oligomeric compounds is selected from 2'-OMe and 2'-MOE.
141. The method of any of embodiments 139-140, wherein each nucleoside of the second modified oligonucleotide comprises a modified sugar moiety.
142. The method of embodiment 141, wherein the modified sugar moiety of the second oligonucleotide is a 2'-MOE sugar moiety.
143. The method of embodiment 139, wherein the second compound is an oligomeric compound comprising a morpholino oligonucleotide.
144. The method of any of embodiments 134-143, wherein the second oligomeric compound has the nucleobase sequence of SEQ ID NO: 1.
145. The method of embodiment 144, wherein the second oligomeric compound has the nucleobase sequence and chemical modification motif of Isis 396443.
146. The method of any of embodiments 134-145, wherein the second oligomeric compound consists of the modified oligonucleotide.
147. The method of any of embodiments 134-146, wherein the second compound is administered prior to administration of the oligomeric compound of any of embodiments 1 to 111 or the composition of embodiment 112.
148. The method of any of embodiments 134-146, wherein the second compound is administered after administration of the oligomeric compound of any of embodiments 1 to 111 or the composition of embodiment 112.
149. The method of any of embodiments 134-146, wherein the second compound is administered less frequently than administration of the oligomeric compound of any of embodiments 1 to 111 or the composition of embodiment 112.
150. An oligomeric compound of any of embodiments 1 to 111 or the composition of embodiment 112 for use in therapy.
151. Use of an oligomeric compound of any of embodiments 1 to 111 or the composition of embodiment 112 for the preparation of a medicament for the treatment of Spinal Muscular Atrophy Type I.
152. Use of an oligomeric compound of any of embodiments 1 to 111 or the composition of embodiment 112 for the preparation of a medicament for the treatment of Spinal Muscular Atrophy Type II
153. Use of an oligomeric compound of any of embodiments 1 to 111 or the composition of embodiment 112 for the preparation of a medicament for the treatment of Spinal Muscular Atrophy Type III.
154. Use of an oligomeric compound of any of embodiments 1 to 111 or the composition of embodiment 112 for the preparation of a medicament for the treatment of Spinal Muscular Atrophy Type IV.
155. Use of an oligomeric compound of any of embodiments 1 to 112 for the preparation of a medicament for the treatment of Spinal Muscular Atrophy.
156. A method comprising (i) systemically administering a compound comprising the oligomeric compound of any of embodiments 1-112 and (ii) administering to the CNS a compound comprising the oligomeric compound of any of embodiments 1-112.
157. A method comprising (i) systemically administering a compound comprising the oligomeric compound or composition of any of embodiments 1-112 and (ii) administering to the CNS a compound comprising a modified oligonucleotide having at least one modified nucleoside comprising a modified sugar moiety selected from 2'-OMe and 2'-MOE.
158. A method comprising (i) systemically administering a compound comprising the oligomeric compound of any of embodiments 1-112 and (ii) administering to the CNS a compound comprising the oligomeric compound of any of embodiments 1-112.
159. A method comprising (i) systemically administering a compound comprising the oligomeric compound of any of embodiments 85-106 and (ii) administering to the CNS a compound comprising a modified oligonucleotide having at least one modified nucleoside comprising a modified sugar moiety selected from 2'-OMe and 2'-MOE.
160. A method comprising (i) systemically administering a modified oligonucleotide having at least one modified nucleoside comprising a modified sugar moiety selected from 2'-OMe and 2'-MOE and a conjugate, and (ii) administering to the CNS a compound comprising the oligomeric compound of any of embodiments 1-112.
161. A method comprising (i) systemically administering a modified oligonucleotide having at least one modified nucleoside comprising a modified sugar moiety selected from 2'-OMe and 2'-MOE and a conjugate group, and (ii) administering to the CNS a compound comprising a modified oligonucleotide having at least one modified nucleoside comprising a modified sugar moiety selected from 2'-OMe and 2'-MOE.
162. The method of embodiment 160, wherein the conjugate group comprises a lipid or lipophilic group selected from cholesterol, a C10-C26 saturated fatty acid, a C10- C26 unsaturated fatty acid, C10-C26 alkyl, a triglyceride, tocopherol, and cholic acid.
163. The compound or method of any of embodiments 1-162, wherein the SMN2 pre-mRNA is a nucleobase sequence selected from any of SEQ ID NO: 10, 11, or 240.

## Claims

1. An oligomeric compound comprising a modified oligonucleotide consisting of 14-25 linked nucleosides, wherein the modified oligonucleotide is complementary to an SMN2 pre-mRNA; and wherein at least one nucleoside of the modified oligonucleotide comprises a 2'-O-(N-alkyl acetamide) modified sugar moiety.

2. The oligomeric compound of claim 1, wherein:
a. each of 7 nucleosides of the modified oligonucleotide comprises a 2'-O-(N-alkyl acetamide) modified sugar moiety;
b. each of 8 nucleosides of the modified oligonucleotide comprises a 2'-O-(N-alkyl acetamide) modified sugar moiety;
c. each of 9 nucleosides of the modified oligonucleotide comprises a 2'-O-(N-alkyl acetamide) modified sugar moiety;
d. each of 10 nucleosides of the modified oligonucleotide comprises a 2'-O-(N-alkyl acetamide) modified sugar moiety;
e. each of 11 nucleosides of the modified oligonucleotide comprises a 2'-O-(N-alkyl acetamide) modified sugar moiety;
f. each of 12 nucleosides of the modified oligonucleotide comprises a 2'-O-(N-alkyl acetamide) modified sugar moiety;
g. each of 13 nucleosides of the modified oligonucleotide comprises a 2'-O-(N-alkyl acetamide) modified sugar moiety;
h. each of 14 nucleosides of the modified oligonucleotide comprises a 2'-O-(N-alkyl acetamide) modified sugar moiety;
i. each of 15 nucleosides of the modified oligonucleotide comprises a 2'-O-(N-alkyl acetamide) modified sugar moiety;
j. each of 16 nucleosides of the modified oligonucleotide comprises a 2'-O-(N-alkyl acetamide) modified sugar moiety;
k. each of 17 nucleosides of the modified oligonucleotide comprises a 2'-O-(N-alkyl acetamide) modified sugar moiety;
l. each of 18 nucleosides of the modified oligonucleotide comprises a 2'-O-(N-alkyl acetamide) modified sugar moiety;
m. each of 19 nucleosides of the modified oligonucleotide comprises a 2'-O-(N-alkyl acetamide) modified sugar moiety; or
n. each of 20 nucleosides of the modified oligonucleotide comprises a 2'-O-(N-alkyl acetamide) modified sugar moiety.

3. The oligomeric compound of claim 1 or claim 2, wherein:
a. each of the 2'-O-(N-alkyl acetamide) modified sugar moieties is a 2'-O-(N-methyl acetamide) modified sugar moiety; or
b. each sugar moiety of each nucleoside of the modified oligonucleotide is a 2'-O-(N-alkyl acetamide) modified sugar moiety; or
c. each sugar moiety of each nucleoside of the modified oligonucleotide is a 2'-O-(N-methyl acetamide) modified sugar moiety.

4. The oligomeric compound of any of claims 1-3, wherein:
a. the modified oligonucleotide consists of: 16-23 linked nucleosides; or
b. the modified oligonucleotide consists of: 18-20 linked nucleosides.

5. The oligomeric compound of any of claims 1-4, wherein each internucleoside linkage of the modified oligonucleotide is selected from among a phosphorothioate internucleoside linkage and a phosphodiester internucleoside linkage, optionally wherein:
a. the modified oligonucleotide has 5 phosphodiester internucleoside linkages;
b. the modified oligonucleotide has 6 phosphodiester internucleoside linkages; or
c. the modified oligonucleotide has at least 6 phosphodiester internucleoside linkages.

6. The oligomeric compound of any of claims 1-5, wherein the modified oligonucleotide comprises at least one modified nucleobase, optionally wherein the modified oligonucleotide comprises at least one 5-methyl cytosine.

7. The oligomeric compound of any of claims 1-6, wherein the oligomeric compound comprises a conjugate group, optionally wherein the conjugate group comprises a lipid or lipophilic group, optionally wherein:
a. the lipid or lipophilic group is selected from among: cholesterol, a C₁₀-C₂₆ saturated fatty acid, a C₁₀- C₂₆ unsaturated fatty acid, C₁₀-C₂₆ alkyl, a triglyceride, tocopherol, or cholic acid;
b. the lipid or lipophilic group is a saturated hydrocarbon chain or an unsaturated hydrocarbon chain;
c. the lipid or lipophilic group is C₁₆ alkyl; or
d. the lipid or lipophilic group is saturated C₁₆.

8. The oligomeric compound of any of claims 1-7, wherein:
(i) the nucleobase sequence of the modified oligonucleotide comprises a sequence selected from SEQ ID NOs 1, 2, or 3, such as wherein the nucleobase sequence of the modified oligonucleotide consists of SEQ ID NO: 3;
(ii) the oligomeric compound is single stranded; and/or
(iii) the modified oligonucleotide is a sodium salt or a potassium salt.

9. A pharmaceutical composition comprising the oligomeric compound of any of claims 1-8, and at least one pharmaceutically acceptable carrier or diluent, optionally wherein the composition comprises a pharmaceutically acceptable diluent and the pharmaceutically acceptable diluent is phosphate buffered saline (PBS), optionally wherein the pharmaceutical composition consists of the compound and PBS.

10. The oligomeric compound of any of claims 1-8, or the pharmaceutical composition of claim 9, for use in a method of:
(i) modulating splicing of an SMN2 pre-mRNA in a cell, tissue, or organ in a patient; or
(ii) promoting inclusion of exon 7 in SMN2 mRNA in a cell, tissue, or organ in a patient,
wherein the method comprises contacting the cell, tissue, or organ with the oligomeric compound or pharmaceutical composition.

11. The oligomeric compound or pharmaceutical composition for use according to claim 10, wherein the method of modulating splicing of an SMN2 pre-mRNA or promoting inclusion of exon 7 in SMN2 mRNA comprises overcoming the genetic defect of the patient.

12. The oligomeric compound of any of claims 1-8, or the pharmaceutical composition of claim 9, for use in a method of treating Spinal Muscular Atrophy in a patient.

13. The oligomeric compound or pharmaceutical composition for use according to claim 12, wherein:
(i) the oligomeric compound or pharmaceutical composition is administered to the central nervous system, optionally wherein the oligomeric compound or pharmaceutical composition is administered intrathecally; or
(ii) the oligomeric compound or pharmaceutical composition is administered systemically, optionally wherein the oligomeric compound or pharmaceutical composition is administered subcutaneously or intramuscularly.

14. The oligomeric compound or pharmaceutical composition for use according to claim 12 or claim 13, wherein the oligomeric compound or pharmaceutical composition is co-administered with a second compound, optionally wherein the second compound is selected from among a small molecule and an oligomeric compound.
